# EUROPEAN PATENT APPLICATION

(11) **EP 3 616 779 A1**
(43) Date of publication of application: **04.03.2020**
(21) Application number: 19193758.0
(22) Date of filing: 27.08.2019
(51) Int. Cl.: B01F 3/08, B01F 9/00, B01F 9/10, B01F 15/02, B01F 15/00

(54) **SPECIMEN PROCESSING METHOD, SPECIMEN PROCESSING APPARATUS, NON-TRANSITORY COMPUTER-READABLE STORAGE MEDIUM, AND SPECIMEN PROCESSING CARTRIDGE**

(30) Priority: 31.08.2018 JP 2018163064
(71) Applicant: SYSMEX CORPORATION, Kobe-shi Hyogo 651-0073 (JP)
(72) Inventor: Miura, Yoshinobu, Hyogo, 651-0073 (JP); Yamashita, Shuji, Hyogo, 651-0073 (JP); Hasegawa, Yudai, Hyogo, 651-0073 (JP); Hayashi, Tomoya, Hyogo, 651-0073 (JP)
(74) Representative: Hoffmann Eitle

(57) **Abstract**

According to one or more aspects, a specimen processing method may use a cartridge comprising a chamber configured to store a liquid. The method may include: storing a specimen and a dispersion medium in the chamber of the cartridge; and rotating the cartridge about a rotational shaft to agitate the specimen and the dispersion medium in the chamber, to thereby form an emulsion in which a dispersoid containing the specimen is dispersed in the dispersion medium.

## Description

### BACKGROUND

The disclosure relates to a specimen processing method of processing a specimen using a cartridge in which a storage space for storing a liquid is formed (see, for example, US Patent No. 9126160 (Patent Document 1)).

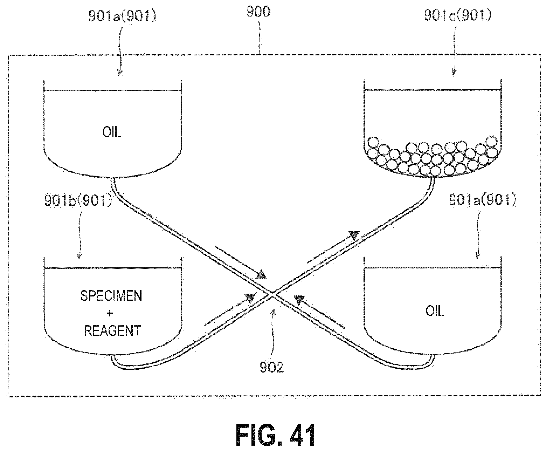

As illustrated in FIG. 41, Patent Document 1 described above discloses a technique of forming an emulsion containing a biological specimen by feeding liquids in a cartridge 900 in which multiple wells 901 for holding liquids are formed. In FIG. 41, the cartridge 900 includes four wells 901, namely two oil wells 901a, one specimen well 901b, and one collection well 901c. The wells 901 are connected via fluid channels 902 formed in the cartridge 900. Each well 901 is opened on the top side. The oil is fed from the two oil wells 901a, and the mixed liquid of the specimen and the reagent is fed from the specimen well 901b. The oil and mixed liquid stored in the wells 901 are injected from the openings at the top of the wells 901, and when air pressure is applied through the openings, the liquids in the wells 901 are fed to the fluid channels 902. The mixed liquid and the oil merge in the fluid channels 902 to form an emulsion in which droplets of the mixed liquid are dispersed in the oil, and the formed emulsion is stored in the collection well 901c.

In a method of forming an emulsion by applying air pressure to wells and feeding a mixed liquid and oil to fluid channels as in Patent Document 1 described above, the flow of the mixed liquid is sheared with the flow of oil at the junction of the fluid channels to form individual droplets in the resultant liquid mixture. For this reason, it is necessary to accurately adjust the air pressure for each single well according to the viscosity of the liquid to be fed through the fluid channel and the affinity between the liquids, and such adjustment is complicated. In addition, when fabricating a specimen processing apparatus which performs such a specimen processing method, it is necessary to provide a mechanism for applying air pressure to each single well and a mechanism for accurately controlling air pressure, which makes the apparatus configuration complicated. Therefore, it is desired to be able to more simply prepare an emulsion when processing a specimen using a cartridge.

One or more aspects aim to make it possible to more easily prepare an emulsion when processing a specimen using a cartridge.

### SUMMARY

According to one or more aspects, a specimen processing method may use a cartridge comprising a chamber configured to store a liquid. The method may include: storing a specimen and a dispersion medium in the chamber of the cartridge; and rotating the cartridge about a rotational shaft to agitate the specimen and the dispersion medium in the chamber, to thereby form an emulsion in which a dispersoid containing the specimen is dispersed in the dispersion medium.
According to one or more aspects, a specimen processing apparatus may include: a setting part in which a cartridge comprising a chamber configured to store a specimen and a dispersion medium is set; a rotation mechanism that rotates the cartridge set in the setting part about a rotational shaft; and a controller that causes the rotation mechanism to rotate the cartridge comprising the chamber storing the specimen and the dispersion medium so as: to agitate the specimen and the dispersion medium in the chamber; and to form an emulsion in which a dispersoid containing the specimen is dispersed in the dispersion medium.
A non-transitory computer-readable storage medium storing a program, which when read and executed, may cause a computer, connected to a specimen processing apparatus that processes a specimen using a cartridge comprising a chamber to store a liquid, to perform operations including causing a rotation mechanism of the specimen processing apparatus to rotate the cartridge that stores the specimen and a dispersion medium about a rotational shaft so as to agitate the specimen and the dispersion medium in the chamber, to thereby form an emulsion in which a dispersoid containing the specimen is dispersed in the dispersion medium.
According to one or more aspects, a specimen processing cartridge including: a specimen introducing unit that introduces a specimen; and a chamber that stores the introduced specimen and a dispersion medium. The specimen processing cartridge may be configured to, by being rotated about a rotational shaft, agitate the specimen and the dispersion medium in the chamber, and to form an emulsion in which a dispersoid containing the specimen is dispersed in the dispersion medium.

### BRIEF DESCRIPTION OF DRAWINGS

FIG. 1A is a diagram illustrating a cartridge before agitation by a specimen processing method, and FIG. 1B is a diagram illustrating a cartridge after agitation by a specimen processing method.
FIG. 2 is a flow diagram illustrating a specimen processing method.
FIG. 3A is a schematic diagram illustrating an inside of a chamber before agitation, and FIG. 3B is a schematic diagram illustrating an inside of a chamber after agitation
FIG. 4A is a schematic diagram illustrating an example of reversing a rotational direction of a cartridge, and FIG. 4B is a schematic diagram illustrating an example of rotating a cartridge in the same direction.
FIG. 5A is a schematic side diagram illustrating a structure of a specimen processing apparatus, and FIG. 5B is a diagram illustrating a cartridge set on a setting part.
FIG. 6 is a schematic diagram illustrating a program.
FIG. 7 is a diagram illustrating an example of providing multiple chambers in a cartridge.
FIG. 8 is a diagram illustrating an example of providing a liquid storing unit in a cartridge.
FIG. 9 is a diagram illustrating a specific configuration example of a cartridge.
FIG. 10 is a flow diagram illustrating an emulsion PCR assay.
FIG. 11 is a diagram illustrating a function of each unit of a cartridge in an emulsion PCR assay.
FIG. 12A to FIG. 12D are diagrams illustrating progress of reaction in an emulsion PCR assay.
FIG. 13 is a schematic plan diagram illustrating a first example of a temperature adjusting unit.
FIG. 14 is a schematic plan diagram illustrating a second example of a temperature adjusting unit.
FIG. 15 is a perspective diagram illustrating a state in which a lid of a specimen processing apparatus is opened.
FIG. 16 is a perspective diagram illustrating a state in which a lid of a specimen processing apparatus is closed.
FIG. 17 is a schematic cross-sectional view illustrating an internal structure of a specimen processing apparatus.
FIG. 18 is a block diagram illustrating a control configuration of a specimen processing apparatus.
FIG. 19 is a schematic diagram illustrating an example of usage of a specimen processing apparatus.
FIG. 20 is a schematic diagram of a scattergram illustrating nucleic acid detection results according to a comparative example.
FIG. 21A to FIG. 21D are diagrams illustrating patterns of changing a rotational speed of a cartridge, such as in Example 1.
FIG. 22A to FIG. 22D are diagrams illustrating microscope images of an emulsion, such as in Example 1.
FIG. 23A to FIG. 23D are schematic diagrams illustrating scattergrams, obtained such as in Example 1.
FIG. 24 is a diagram illustrating results of comparing nucleic acid detection results, such as according to Example 1 with a comparative example.
FIG. 25A to FIG. 25D are schematic diagrams illustrating a chamber at emulsion formation for each volume ratio, such as in Example 2.
FIG. 26 is a diagram illustrating results of comparing nucleic acid detection results, such as according to Example 2 with a comparative example.
FIG. 27 is a diagram illustrating a pattern of changing a rotational speed of a cartridge, such as in Example 3.
FIG. 28 provides schematic diagrams illustrating a chamber at emulsion formation, such as in Example 3.
FIG. 29 provides schematic diagrams illustrating scattergrams, obtained such as in Example 3.
FIG. 30A to FIG. 30D are diagrams illustrating patterns of changing a rotational speed of a cartridge, such as in Example 4.
FIG. 31A to FIG. 31D are schematic diagrams illustrating scattergrams, obtained such as in Example 4.
FIG. 32 is a diagram illustrating results of comparing nucleic acid detection results, such as according to Example 4 with a comparative example.
FIG. 33 is a schematic diagram illustrating a cartridge (modified example) used for an immunoassay.
FIG. 34 is a diagram illustrating progress of reaction in an immunoassay.
FIG. 35 is a diagram illustrating a function of each unit of a cartridge in an immunoassay.
FIG. 36 is a diagram illustrating a cartridge of a second embodiment.
FIG. 37 is a diagram illustrating a process flow using a cartridge of a second embodiment.
FIG. 38 is a diagram illustrating a cartridge of a third embodiment.
FIG. 39 is a diagram illustrating a process flow using a cartridge of a third embodiment.
FIG. 40 is a diagram illustrating a modified example of a specimen processing apparatus.
FIG. 41 is a diagram illustrating a conventional art.

### DETAILED DESCRIPTION

A specimen processing method according to a first aspect is a specimen processing method that uses a cartridge (100) provided with a chamber (11) to store a liquid, the method including: storing a specimen (80) and a dispersion medium (82) in the chamber (11) of the cartridge (100); rotating the cartridge (100) about a rotational shaft (221) to agitate the specimen (80) and the dispersion medium (82) in the chamber (11), to thereby form an emulsion (83) in which a dispersoid containing the specimen (80) is dispersed in the dispersion medium (82).

Here, the emulsion is a disperse system solution in which a dispersoid is dispersed in a dispersion medium. The disperse system refers to the state in which a dispersoid is floating or suspended in a dispersion medium. The dispersion medium and the dispersoid are both liquids. However, the dispersion medium and the dispersoid may be a solution containing matter other than liquid. The dispersoid does not mix with the dispersion medium. Specifically, the dispersion medium and the dispersoid do not form a homogeneous phase by mixing. The dispersoid is separated from one another by the dispersion medium and surrounded by the dispersion medium. Thus, in an emulsion, droplets of dispersoid are formed in the dispersion medium. The formation of an emulsion is called "emulsification." Breaking an emulsion to separate the dispersoid and the dispersion medium individually is called "demulsification."

In the specimen processing method according to a first aspect, rotation of the cartridge (100) about the rotational shaft (221) applies an inertial force associated with the rotation of the cartridge (100) to the chamber (11), which agitates the specimen (80) and the dispersion medium (82) in the chamber (11). With agitation, the dispersoid is finely sheared by the dispersion medium (82). As a result, the emulsion (83) is formed in which droplets (84) of the dispersoid containing a detection matter (MD, or a detection target) contained in the specimen (80) are dispersed in the dispersion medium (82). As described above, mere rotation of the cartridge (100) about the rotational shaft (221) without using liquid feeding by air pressure control makes it possible to form the emulsion (83) in the chamber (11). Therefore, the emulsion (83) can be more simply prepared when processing a specimen using a cartridge.

In the specimen processing method according to a first aspect, preferably, the cartridge (100) includes a plurality of the chambers (11) arranged in an arc form whose radial distances from the rotational shaft (221) are substantially equal. With such configuration, mere rotation of the cartridge (100) about the rotational shaft (221) makes it possible to prepare emulsions (83) containing the same or different specimens in the multiple chamber (11). Here, when the distance of each chamber (11) from the rotational shaft (221) is different, the magnitudes of the inertial forces acting on the chambers (11) are different. Thus, the quality of the emulsions (83) prepared may vary from one chamber (11) to another. On the other hand, in the above configuration in which the multiple chamber (11) are arranged in an arc form with substantially equal radial distances from the rotational shaft (221), the magnitudes of the inertial forces acting on the respective chambers (11) during rotation are substantially equal. Therefore, the quality of the emulsions (83) prepared can be made more uniform. Note that the quality of the emulsion (83) is, for example, the number or size (or diameter) of the droplets of dispersoid contained in the emulsion (83).

In the specimen processing method according to a first aspect, the emulsion (83) is formed in the chamber (11) preferably by repeating an operation of changing a rotational speed of the cartridge (100) being rotated about the rotational shaft (221). With such configuration, when the operation of changing the rotational speed of the cartridge (100) is repeated, the inertial force associated with the speed change acts to effectively agitate the specimen (80) and the dispersion medium (82) in the chamber (11), making it possible to finely shear the dispersoid with the dispersion medium (82) rapidly. As a result, the emulsion (83) can be efficiently prepared.

In the configuration which repeats the operation of changing the rotational speed of the cartridge (100), preferably, the operation of changing the rotational speed of the cartridge (100) includes an operation of reversing a rotational direction of the cartridge (100) or an operation of acceleration and deceleration in the same direction. Note that the operation of reversing the rotational direction of the cartridge includes rotation of the cartridge in one direction, stop of rotation, and rotation of the cartridge in another direction. The operation of acceleration and deceleration in the same direction may include stopping the rotation by deceleration. With such configuration, in the case where the rotational direction of the cartridge (100) is reversed, a large inertial force can be exerted when reversing the rotational direction, making it possible to efficiently prepare the emulsion (83). When the cartridge (100) is accelerated and decelerated in the same direction, it is unnecessary to reversely rotate the rotation mechanism for rotating the cartridge (100), which makes it possible to easily prepare the emulsion (83).

In that case, preferably, the operation of changing the rotational speed of the cartridge (100) includes an operation of reversing the rotational direction of the cartridge (100) in a cycle of 165 milliseconds or more and 330 milliseconds or less. Here, as a result of the experiments to be described later, the inventors of the present application have found thin a cycle of reversing the rotational direction of the cartridge (100) is preferably 165 milliseconds or more and 330 milliseconds or less in terms of preparing the emulsion (83) for processing and detecting the detection matter (MD). Therefore, only by repeating the operation of reversing the rotational direction in a cycle of 165 milliseconds or more and 330 milliseconds or less, the emulsion (83) suitable for processing and detecting the detection matter (MD) can be prepared.

In the configuration which repeats the operation of changing the rotational speed of the cartridge (100), preferably, a ratio of a total volume of the dispersoid and the dispersion medium (82) to the chamber (11) is 30% or more and 70% or less. Here, as a result of the experiments to be described later, the inventors of the present application have found that the liquid is stored at a ratio of preferably 30% or more and 70% or less of the chamber (11) in terms of preparing the emulsion (83) for processing and detecting the detection matter (MD). Therefore, the above configuration makes it possible to prepare the emulsion (83) suitable for processing and detecting the detection matter (MD).

In the configuration which repeats the operation of changing the rotational speed of the cartridge (100), preferably, the operation of changing the rotational speed of the cartridge (100) includes an operation of reversing a rotational direction of the cartridge (100) in a cycle of 330 milliseconds, and a ratio of a total volume of the dispersoid and the dispersion medium (82) to the chamber (11) is 30%. With such configuration, it is possible to prepare the emulsion (83) even more suitable for processing and detecting the detection matter (MD) based on the experimental results to be described later.

In the configuration which repeats the operation of changing the rotational speed of the cartridge (100), preferably, the operation of changing the rotational speed of the cartridge (100) includes an operation of reversing a rotational direction of the cartridge (100) in a cycle of 165 milliseconds, and a ratio of a total volume of the dispersoid and the dispersion medium (82) to the chamber (11) is 50% or more and 70% or less. With such configuration, it is possible to prepare the emulsion (83) even more suitable for processing and detecting the detection matter (MD) based on the experimental results to be described later.

In the specimen processing method according to a first aspect, preferably, a detection matter (MD) contained in the specimen (80) is a nucleic acid or a protein. With such configuration, it is possible to form the emulsion (83) in which fine droplets (84) of dispersoid containing nucleic acid or protein as the detection matter (MD) are dispersed in the dispersion medium (82). As a result, individual molecules of the detection matter (MD) are compartmentalized into droplets (84), making it possible to reliably process or detect individual proteins or nucleic acids.

The specimen processing method according to a first aspect may further include: processing a detection matter (MD) contained in the specimen (80) with a reagent (88) containing a labeling substance (LS) to label the detection matter (MD); and detecting a signal based on the labeling substance (LS).

The specimen processing method according to a first aspect preferably further includes: storing, in the chamber (11), the specimen (80), the dispersion medium (82), and a reagent (81) to process a detection matter (MD) in the specimen (80); and forming, by rotating the cartridge (100), the emulsion (83) in which the dispersoid containing the specimen (80) and the reagent (81) is dispersed in the dispersion medium (82). With such configuration, it is possible to process the detection matter (MD) with the reagent (81) in the droplets (84) of the dispersoid contained in the emulsion (83).

In this case, preferably, the reagent (81) includes a nucleic acid amplification reagent that amplifies the nucleic acid in the specimen (80), or a substrate that reacts with a labeling substance specifically bound to a protein in the specimen (80). With such configuration, the amplification of the nucleic acid as the detection matter (MD) and the detection based on the labeling substance for the protein as the detection matter (MD) can be reliably performed on individual molecules of the detection matter (MD) in the droplets (84) contained in the emulsion (83).

In the specimen processing method according to a first aspect, the dispersion medium (82) may be an oil that is immiscible with the specimen (80) and the reagent (81).

In the configuration which forms the emulsion (83) of the dispersoid containing the specimen (80) and the reagent (81), preferably, rotation of the cartridge (100) forms in the chamber (11) the emulsion (83) containing droplets (84) of the dispersoid each storing one molecule or one particle of the detection matter (MD). With such configuration, individual molecules of the detection matter (MD) contained in the specimen can be subjected to compartmentalization into the respective droplets (84) contained in the emulsion (83), and can be reliably processed. As a result, even the detection matter (MD) contained in an extremely small amount in the specimen can be processed with high accuracy. Therefore, it is possible to improve the detection accuracy also in the detection of the processed detection matter (MD).

In the configuration which forms the emulsion (83) of the dispersoid containing the specimen (80) and the reagent (81), preferably, the cartridge (100) includes a liquid storing unit (30) that is connected to the chamber (11) and stores a liquid to be transferred to the chamber (11), the liquid storing unit (30) includes a first liquid storing unit (31) to store the dispersion medium (82), and rotation of the cartridge (100) about the rotational shaft (221) transfers the dispersion medium (82) from the first liquid storing unit (31) to the chamber (11). With such configuration, mere rotation of the cartridge (100) enables not only producing the emulsion (83) but also an operation of storing the dispersion medium (82) in the chamber (11). Therefore, since it is not necessary to apply air pressure to, for example, the first liquid storing unit (31), the specimen can be more easily processed.

The configuration which forms the emulsion (83) of the dispersoid containing the specimen (80) and the reagent (81) preferably includes, after the emulsion (83) is formed, causing a reaction between the detection matter (MD) and the reagent (81) contained in the dispersoid by changing a temperature of the cartridge (100). With such configuration, it is possible to reliably cause a reaction between the detection matter (MD) and the reagent (81) by temperature change in the state in which the detection matter (MD) is stored for compartmentalization in the droplets (84) of the dispersoid together with the reagent (81) to process the detection matter (MD). In addition, as compared with the case of causing the reaction between the detection matter (MD) and the reagent (81) to naturally proceed under the temperature of the setting environment of the cartridge (100), it is possible to efficiently cause a reaction between the detection matter (MD) and the reagent (81) using the temperature change even in the droplets (84) contained in the emulsion (83).

In the configuration which forms the emulsion (83) of the dispersoid containing the specimen (80) and the reagent (81), preferably, the detection matter (MD) is a nucleic acid, the reagent (81) is a nucleic acid amplification reagent that amplifies the nucleic acid in the specimen (80), and, after the emulsion (83) is formed, a temperature of the cartridge (100) is cyclically changed to multiple temperature ranges to amplify the nucleic acid contained in the dispersoid. With such configuration, it is possible to perform so-called thermal cycling to efficiently amplify nucleic acids in the droplets (84) contained in the emulsion (83).

In this case, preferably, the cartridge (100) includes a liquid storing unit (30) that is connected to the chamber (11) and stores a liquid to be transferred to the chamber (11), the liquid storing unit (30) includes a second liquid storing unit (32) that is connected to the chamber (11) and stores a reagent (85) to demulsify the emulsion when mixed, and, after the nucleic acid contained in the dispersoid is amplified, the cartridge (100) is rotated about the rotational shaft (221) to transfer the reagent (85) to demulsify the emulsion from the second liquid storing unit (32) to the chamber (11) in which the emulsion (83) is formed. With such configuration, it is possible to take out nucleic acids amplified in the droplets (84) contained in the emulsion (83) with the reagent (85) to demulsify the emulsion. Here, unlike in the case of liquid feeding by air pressure or the like, mere rotation of the cartridge (100) about the rotational shaft (221) causes the reagent (85) to demulsify the emulsion to be fed to the chamber (11), making it possible to simply demulsify the emulsion (83).

In the configuration in which the cartridge (100) includes the liquid storing unit (30), preferably, the liquid storing unit (30) includes a sealing body (30a) that seals the liquid storing unit (30), and, after amplification of the nucleic acid contained in the dispersoid, the sealing body (30a) of the second liquid storing unit (32) is unsealed. With such configuration, the sealing body (30a) makes it possible to prevent unintended feeding of the reagent (85) to demulsify the emulsion from the second liquid storing unit (32). Therefore, the emulsion (83) can be demulsified after reliably forming the emulsion (83) and amplifying nucleic acids.

In the configuration in which the cartridge (100) includes the liquid storing unit (30), preferably, the cartridge (100) includes the chamber (11) in which the emulsion (83) is formed, a second chamber (12) that is connected to the chamber (11), and a third liquid storing unit (33) that is connected to the second chamber (12) and stores a reagent (86) to wash the detection matter (MD), and after the nucleic acid at least contained in the dispersoid is amplified, the cartridge (100) is rotated about the rotational shaft (221) to transfer the reagent (86) to wash the detection matter (MD) from the third liquid storing unit (33) to the second chamber (12). With such configuration, nucleic acids can be washed in the second chamber (12) after amplifying the nucleic acids being the detection matter (MD) in the chamber (11) and taking out the nucleic acids from the droplets (84) by demulsification. In addition, the process of amplifying nucleic acid in the chamber (11) involves a temperature change due to thermal cycling. For this reason, if the reagent (86) to wash the detection matter (MD) is already stored in the second chamber (12), the reagent (86) to wash the detection matter (MD) may be affected by the temperature change. In view of the above, when the reagent (86) to wash the detection matter (MD) is fed to the second chamber (12) after amplification, it is possible to suppress the influence of the temperature change on the reagent (86) to wash the detection matter (MD).

In this case, preferably, the reagent (86) to wash the detection matter (MD) contains an alcohol, and, in amplifying the nucleic acid contained in the dispersoid, the temperature of the cartridge (100) is changed in a range of 30°C or more and 90°C or less. When the temperature change in the amplification of nucleic acids as described above is 30°C or more and 90°C or less, the reagent (86) to wash the detection matter (MD) may be affected by the temperature change to vaporize the alcohol. Therefore, a configuration which amplifies nucleic acids and then feeds the reagent (86) to wash the detection matter (MD) to the second chamber (12) is particularly effective in that the vaporization of alcohol can be suppressed.

In the configuration in which the cartridge (100) includes the chamber (11) to form the emulsion (83) and the second chamber (12) connected to the chamber (11), preferably, the reagent (81) contains magnetic particles (PM) that bind to the nucleic acid in the specimen (80), and, when the reagent (85) to demulsify the emulsion is transferred to the chamber (11) and then a magnetic force is applied to the cartridge (100), the nucleic acid bound to the magnetic particles (PM) is transferred from the demulsified liquid in the chamber (11) to the second chamber (12). With such configuration, once the nucleic acids being the detection matter (MD) are bound to the magnetic particles (PM), it is possible to simply transfer the detection matter (MD) to the second chamber (12) by merely applying an external magnetic force to the cartridge (100) without feeding by air pressure or the like. In addition, in the case of liquid feeding by air pressure or the like, the detection matter (MD) in the chamber (11) is transferred together with the liquid. On the other hand, in the magnetic transfer, only the detection matter (MD) bound to the magnetic particles (PM) can be transferred to the second chamber (12) while leaving the liquid. As a result, it is possible to efficiently wash the nucleic acids in the second chamber (12) by suppressing the transfer of unnecessary components to the second chamber (12).

In the configuration in which the cartridge (100) includes the chamber (11) to form the emulsion (83) and the second chamber (12) connected to the chamber (11), preferably, the cartridge (100) includes a third chamber (13) that is connected to the second chamber (12) and a fourth liquid storing unit (34) that is connected to the third chamber (13) and stores a reagent (87) to denature the nucleic acid, and after the nucleic acid at least contained in the dispersoid is amplified, the cartridge (100) is rotated about the rotational shaft (221) to transfer the reagent (87) to denature the nucleic acid from the fourth liquid storing unit (34) to the third chamber (13). With such configuration, the nucleic acids after being subjected to the processing of washing the nucleic acids in the second chamber (12) can be further subjected to processing of denaturing the nucleic acids in the cartridge (100). Additionally, also in the case of performing the processing of denaturing the nucleic acids, mere rotation of the cartridge (100) about the rotational shaft (221) makes it possible to feed liquid to the third chamber (13) instead of liquid feeding using air pressure or the like. Therefore, it is possible to simply perform the processing of denaturing the nucleic acids.

In this case, preferably, the cartridge (100) includes a fourth chamber (14) that is connected to the third chamber (13) and a fifth liquid storing unit (35) that is connected to the fourth chamber (14) and stores a reagent (88) containing a labeling substance (LS) that reacts with an amplification product of the nucleic acid, and rotation of the cartridge (100) about the rotational shaft (221) transfers the reagent (88) containing the labeling substance (LS) from the fifth liquid storing unit (35) to the fourth chamber (14), and change of the temperature of the cartridge (100) causes a reaction between the nucleic acid and the labeling substance (LS) transferred to the fourth chamber (14). With such configuration, it is possible to perform, in the cartridge (100), not only the processing of denaturing the nucleic acids but also the processing of further labeling the nucleic acids being the detection matter (MD) for the purpose of detection. Additionally, also in the case of performing the processing of labeling the nucleic acids, mere rotation of the cartridge (100) about the rotational shaft (221) makes it possible to feed liquid to the fourth chamber (14) instead of liquid feeding using air pressure or the like. Therefore, it is possible to simply perform the processing of labeling the nucleic acids.

In the configuration in which the temperature of the cartridge (100) is changed to cause a reaction between the nucleic acid and the labeling substance (LS) transferred to the fourth chamber (14), preferably, the temperature of the cartridge (100) is cyclically changed to multiple temperature ranges by at least heating to amplify the nucleic acid contained in the dispersoid in the chamber (11), and the temperature of the cartridge (100) is raised by at least heating to cause a reaction between the nucleic acid and the labeling substance (LS) in the fourth chamber (14). With such configuration, both the processing of amplifying the nucleic acids and the processing of labeling the nucleic acids can be performed in the cartridge (100) simply by at least heating the cartridge (100). Therefore, not only the preparation of the emulsion (83) but also the processing of amplifying the nucleic acids and the processing of labeling the nucleic acids using the cartridge (100) can be more simply performed.

In the configuration in which the temperature of the cartridge (100) is changed to amplify the nucleic acid contained in the dispersoid in the chamber (11), preferably, the temperature of the cartridge (100) is changed by bringing the cartridge (100) into contact with a temperature adjusting unit (260) that changes the temperature of the cartridge (100) at least by heating. With such configuration, as compared with the case of heating the cartridge (100) in a non-contact manner, heat can be efficiently conducted by bringing the temperature adjusting unit (260) into contact with the cartridge (100). Therefore, the temperature of the cartridge (100) can be easily changed.

A specimen processing apparatus according to a second aspect includes: a setting part (210) where to set a cartridge (100) that includes a chamber (11) to store a specimen (80) and a dispersion medium (82); a rotation mechanism (220) that rotates the cartridge (100) set on the setting part (210) about a rotational shaft (221); and a controller (230) that performs control of causing the rotation mechanism (220) to rotate the cartridge (100) including the chamber (11) storing the specimen (80) and the dispersion medium (82), thereby agitating the specimen (80) and the dispersion medium (82) in the chamber (11), and forming an emulsion (83) in which the dispersoid containing the specimen (80) is dispersed in the dispersion medium (82).

In the specimen processing apparatus according to a second aspect, rotation of the cartridge (100) about the rotational shaft (221) applies an inertial force associated with the rotation of the cartridge (100) to the chamber (11), which agitates the specimen (80) and the dispersion medium (82) in the chamber (11). With agitation, the dispersoid is finely sheared by the dispersion medium (82). As a result, the emulsion (83) is formed in which droplets (84) of the dispersoid containing a detection matter (MD) in the specimen (80) are dispersed in the dispersion medium (82). As described above, mere rotation of the cartridge (100) about the rotational shaft (221) without using liquid feeding by air pressure control makes it possible to form the emulsion (83) in the chamber (11). Therefore, the emulsion (83) can be more simply prepared when processing a specimen using a cartridge. Moreover, in the case of forming an emulsion using liquid feeding by air pressure control, it is necessary to provide a mechanism for applying air pressure to the part for storing the dispersion medium (82) and the part for storing the specimen, and a mechanism for accurately controlling the air pressure. In contrast, in the above configuration, the emulsion (83) can be formed only by providing the rotation mechanism (220). Therefore, the apparatus configuration can be simplified to obtain a smaller and simpler specimen processing apparatus.

In the specimen processing apparatus according to a second aspect, preferably, the controller (230) forms the emulsion (83) in the chamber (11) by controlling the rotation mechanism (220) such that the rotation mechanism (220) repeats an operation of changing a rotational speed of the cartridge (100) being rotated about the rotational shaft (221). With such configuration, when the operation of changing the rotational speed of the cartridge (100) is repeated, the inertial force associated with the speed change acts to effectively agitate the specimen (80) and the dispersion medium (82) in the chamber (11), making it possible to finely shear the dispersoid with the dispersion medium (82) rapidly. As a result, the emulsion (83) can be efficiently prepared.

In the configuration which repeats the operation of changing the rotational speed of the cartridge (100), preferably, the controller (230) controls, when forming the emulsion (83), the rotation mechanism (220) such that the rotation mechanism (220) repeats an operation of reversing a rotational direction of the cartridge (100) or an operation of acceleration and deceleration in the same direction. With such configuration, in the case where the rotational direction of the cartridge (100) is reversed, a large inertial force can be exerted when reversing the rotational direction, making it possible to efficiently prepare the emulsion (83). When the cartridge (100) is accelerated and decelerated in the same direction, it is unnecessary to reversely rotate the rotation mechanism (220), which makes it possible by simple control to easily prepare the emulsion (83).

In this case, preferably, the controller (230) controls, when forming the emulsion (83), the rotation mechanism (220) the rotation mechanism (220) repeats an operation of reversing the rotational direction of the cartridge (100) in a cycle of 165 milliseconds or more and 330 milliseconds or less. With such configuration, only by repeating the operation of reversing the rotational direction in a cycle of 165 milliseconds or more and 330 milliseconds or less based on the results of the experiments to be described later, the emulsion (83) suitable for processing and detecting the detection matter (MD) can be prepared.

In the specimen processing apparatus according to a second aspect, preferably, the controller (230) causes the cartridge (100) to rotate to form in the chamber (11) the emulsion (83) containing droplets (84) of the dispersoid each storing one molecule or one particle of the detection matter (MD). With such configuration, individual molecules of the detection matter (MD) contained in the specimen can be subjected to compartmentalization into the respective droplets (84) contained in the emulsion (83), and can be reliably processed. As a result, even the detection matter (MD) contained in an extremely small amount in the specimen can be processed with high accuracy. Therefore, it is possible to improve the detection accuracy also in the detection of the processed detection matter (MD).

In the specimen processing apparatus according to a second aspect, preferably, the controller (230) controls the rotation mechanism (220) such that the dispersion medium (82) stored in the first liquid storing unit (31) included in the cartridge (100) is transferred by rotation to the chamber (11). With such configuration, mere rotation of the cartridge (100) by the rotation mechanism (220) enables not only producing the emulsion (83) but also an operation of storing the dispersion medium (82) in the chamber (11). Therefore, since it is not necessary to apply air pressure to, for example, the first liquid storing unit (31), the specimen can be more easily processed. In addition, it is unnecessary to provide e.g. a mechanism for applying air pressure in order to feed the dispersion medium (82) from the liquid storing unit (30) to the chamber (11). Therefore, the apparatus configuration can be further simplified.

In the specimen processing apparatus according to a second aspect, preferably, the controller (230) causes the rotation mechanism (220) such that the rotation mechanism (220) rotates the cartridge (100) including the chamber (11) storing the specimen (80), a reagent (81) to process a detection matter (MD) in the specimen (80), and the dispersion medium (82), and thereby forms the emulsion (83) in which the dispersoid containing the specimen (80) and the reagent (81) is dispersed in the dispersion medium (82). With such configuration, it is possible to process the detection matter (MD) with the reagent (81) in the droplets (84) of the dispersoid contained in the emulsion (83).

In this case, preferably, the specimen processing apparatus further includes a temperature adjusting unit (260) that changes a temperature of the cartridge (100) to cause a reaction between the detection matter (MD) and the reagent (81) contained in the dispersoid in the emulsion (83). With such configuration, it is possible to reliably cause a reaction between the detection matter (MD) and the reagent (81) by temperature change in the state in which the detection matter (MD) is stored for compartmentalization in the droplets (84) of the dispersoid together with the reagent (81) to process the detection matter (MD). In addition, as compared with the case of causing the reaction between the detection matter (MD) and the reagent (81) to naturally proceed under the temperature of the setting environment of the cartridge (100), it is possible to efficiently cause a reaction between the detection matter (MD) and the reagent (81) using the temperature change even in the droplets (84) contained in the emulsion (83).

In the configuration including the temperature adjusting unit (260), preferably, the detection matter (MD) is a nucleic acid, the reagent (81) is a nucleic acid amplification reagent that amplifies the nucleic acid in the specimen (80), and the temperature adjusting unit (260) cyclically changes a temperature of the cartridge (100) to multiple temperature ranges to amplify the nucleic acid contained in the dispersoid. With such configuration, it is possible to perform so-called thermal cycling to efficiently amplify nucleic acids in the droplets (84) contained in the emulsion (83).

In this case, preferably, the controller (230) controls the rotation mechanism (220) such that a reagent (85) to demulsify the emulsion is transferred by rotation from the second liquid storing unit (32) included in the cartridge (100) to the chamber (11) in which the emulsion (83) is formed. With such configuration, the specimen processing apparatus (200) enables not only the processing of forming the emulsion (83) but also the processing of demulsifying the emulsion (83) in the cartridge (100). Also in this case, it is unnecessary to provide e.g. a mechanism for applying air pressure in order to feed the reagent (85) to demulsify the emulsion to the chamber (11). Therefore, the apparatus configuration can be further simplified.

In the configuration which transfers by rotation the reagent (85) to demulsify the emulsion from the second liquid storing unit (32) included in the cartridge (100), preferably, the specimen processing apparatus further includes an unsealing mechanism (250) that unseals a sealing body (30a) which seals the liquid storing unit (30) of the cartridge (100), wherein the controller (230) controls the unsealing mechanism (250) such that the sealing body (30a) is unsealed after the emulsion (83) is formed in the chamber (11) and the temperature of the cartridge (100) is changed to cause a reaction between the detection matter (MD) and the reagent (81). With such configuration, the sealing body (30a) makes it possible to prevent unintended feeding of the reagent (85) to demulsify the emulsion from the second liquid storing unit (32). Therefore, if the unsealing mechanism (250) which unseals the sealing body (30a) is provided, the emulsion (83) can be demulsified after reliably forming the emulsion (83) and amplifying nucleic acids.

In this case, preferably, the specimen processing apparatus further comprises a transfer mechanism (240) that transfers the detection matter (MD) to a second chamber (12) connected to the chamber (11), wherein the controller (230) controls the rotation mechanism (220) such that a reagent (86) to wash the detection matter (MD) is transferred by rotation from a third liquid storing unit (33) included in the cartridge (100) to the second chamber (12), and, after the emulsion (83) is demulsified, controls the transfer mechanism (240) such that the detection matter (MD) is transferred from the chamber (11) to the second chamber (12). With such configuration, nucleic acids can be washed in the second chamber (12) after amplifying the nucleic acids being the detection matter (MD) in the chamber (11) and taking out the nucleic acids from the droplets (84) by demulsification.

In the configuration which transfers by rotation the reagent (86) to wash the detection matter (MD) from the third liquid storing unit (33) included in the cartridge (100) to the second chamber (12), preferably, the controller (230) controls the unsealing mechanism (250) such that the sealing body (30a) of the liquid storing unit (30) storing the reagent (86) to wash the detection matter (MD) is unsealed at least after the temperature adjusting unit (260) causes a reaction between the detection matter (MD) and the reagent (81). With such configuration, when the reagent (86) to wash the detection matter (MD) is fed to the second chamber (12) after amplification due to the temperature change caused by use of the temperature adjusting unit (260), it is possible to suppress the influence of the temperature change on the reagent (86) to wash the detection matter (MD).

In this case, preferably, the reagent (86) to wash the detection matter (MD) contains an alcohol, and the controller (230) controls the temperature adjusting unit (260) such that the temperature of the cartridge (100) is changed in a range of 30°C or more and 90°C or less. When the temperature change in the amplification of nucleic acids as described above is 30°C or more and 90°C or less, the reagent (86) to wash the detection matter (MD) may be affected by the temperature change to vaporize the alcohol. Therefore, a configuration which amplifies nucleic acids, then unseals the sealing body (30a), and feeds the reagent (86) to wash the detection matter (MD) to the second chamber (12) is particularly effective in that the vaporization of alcohol can be suppressed.

In the configuration which further includes the transfer mechanism (240), preferably, the controller (230) controls the rotation mechanism (220) such that the reagent (87) to denature the nucleic acid is transferred by rotation from a fourth liquid storing unit (34) included in the cartridge (100) to a third chamber (13) connected to the second chamber (12), and controls the transfer mechanism (240) such that the detection matter (MD) stored in the second chamber (12) is transferred to the third chamber (13). With such configuration, the nucleic acids after being subjected to the processing of washing the nucleic acids in the second chamber (12) can be further subjected to processing of denaturing the nucleic acids in the cartridge (100). Additionally, also in the case of performing the processing of denaturing the nucleic acids, mere rotation of the cartridge (100) about the rotational shaft (221) makes it possible to feed liquid to the third chamber (13) instead of liquid feeding using air pressure or the like. Therefore, it is possible to perform the processing of denaturing the nucleic acids with a simple apparatus configuration.

In this case, preferably, the controller (230) controls the unsealing mechanism (250) such that the sealing body (30a) of the liquid storing unit (30) storing the reagent (87) to denature the nucleic acid is unsealed at least after the temperature adjusting unit (260) causes a reaction between the detection matter (MD) and the reagent (81). With such configuration, when the reagent (87) to denature the nucleic acid is fed to the third chamber (13) after amplification due to the temperature change caused by use of the temperature adjusting unit (260), it is possible to suppress the influence of the temperature change on the reagent (87) to denature the nucleic acid.

In the configuration which transfers the detection matter (MD) stored in the second chamber (12) to the third chamber (13), preferably, the controller (230) controls the rotation mechanism (220) such that the reagent (88) containing a labeling substance (LS) that reacts with an amplification product of the nucleic acid is transferred by rotation from a fifth liquid storing unit (35) included in the cartridge (100) to a fourth chamber (14) connected to the third chamber (13), and controls the transfer mechanism (240) such that the detection matter (MD) stored in the third chamber (13) is transferred to the fourth chamber (14). With such configuration, it is possible to perform, in the cartridge (100), not only the processing of denaturing the nucleic acids but also the processing of further labeling the nucleic acids being the detection matter (MD) for the purpose of detection. Additionally, also in the case of performing the processing of labeling the nucleic acids, mere rotation of the cartridge (100) by the rotation mechanism (220) makes it possible to feed liquid to the fourth chamber (14) instead of liquid feeding using air pressure or the like. Therefore, it is possible to perform the processing of labeling the nucleic acids with a simple apparatus configuration.

In this case, preferably, the temperature adjusting unit (260) cyclically changes the temperature of the cartridge (100) to multiple temperature ranges to amplify the nucleic acid contained in the dispersoid in the chamber (11), and raises the temperature of the cartridge (100) to cause a reaction between the nucleic acid and the labeling substance (LS) in the fourth chamber (14). With such configuration, both the processing of amplifying the nucleic acids and the processing of labeling the nucleic acids can be performed in the cartridge (100) by the common temperature adjusting unit (260). Therefore, not only the preparation of the emulsion (83) but also the processing of amplifying the nucleic acids and the processing of labeling the nucleic acids using the cartridge (100) can be more simply performed with a simple apparatus configuration.

In the configuration including the temperature adjusting unit (260), preferably, the temperature adjusting unit (260) is structured to be movable between a contact position and a floating position relative to the cartridge (100) placed on the setting part (210), and changes the temperature of the cartridge (100) while in contact with the cartridge (100). With such configuration, as compared with the case of heating the cartridge (100) in a non-contact manner, heat can be efficiently conducted by bringing the temperature adjusting unit (260) into contact with the cartridge (100). Therefore, the temperature of the cartridge (100) can be easily changed.

In the configuration including the temperature adjusting unit (260), preferably, the detection matter (MD) is a protein, and the reagent (81) contains a substrate that reacts with a labeling substance specifically bound to the protein in the specimen (80). With such configuration, when the detection matter (MD) is a protein, after compartmentalization of the detection matter (MD) into individual droplets (84) contained in the emulsion (83), it is possible to reliably carry out the reaction between the substrate and the labeling substance for detecting the detection matter (MD) in the individual droplets (84).

A program according to a third aspect causes a computer, connected to a specimen processing apparatus that processes a specimen (80) using a cartridge (100) including a chamber (11) to store a liquid, to function as a controller (230) that performs control of: causing a rotation mechanism (220) included in the specimen processing apparatus to rotate the cartridge (100), in which the chamber (11) stores the specimen (80) and a dispersion medium (82), about a rotational shaft (221); rotating the cartridge (100) to agitate the specimen (80) and the dispersion medium (82) in the chamber (11), to thereby form an emulsion (83) in which a dispersoid containing the specimen (80) is dispersed in the dispersion medium (82).

In the program according to a third aspect, rotation of the cartridge (100) about the rotational shaft (221) applies an inertial force associated with the rotation of the cartridge (100) to the chamber (11), which agitates the specimen (80) and the dispersion medium (82) in the chamber (11). With agitation, the dispersoid is finely sheared by the dispersion medium (82). As a result, the emulsion (83) is formed in which droplets (84) of the dispersoid containing a detection matter (MD) in the specimen (80) are dispersed in the dispersion medium (82). As described above, mere rotation of the cartridge (100) about the rotational shaft (221) without using liquid feeding by air pressure control makes it possible to form the emulsion (83) in the chamber (11). Therefore, the emulsion (83) can be more simply prepared when processing a specimen using a cartridge.

A specimen processing cartridge according to a fourth aspect includes: a specimen introducing unit (20) that introduces a specimen (80); and a chamber (11) that stores the introduced specimen (80) and a dispersion medium (82), wherein the specimen processing cartridge is structured to, by being rotated about a rotational shaft (221), agitate the specimen (80) and the dispersion medium (82) in the chamber (11), and form an emulsion (83) in which a dispersoid containing the specimen (80) is dispersed in the dispersion medium (82).

In the specimen processing cartridge according to a fourth aspect, rotation of the cartridge (100) about the rotational shaft (221) applies an inertial force associated with the rotation of the cartridge (100) to the chamber (11), which agitates the specimen (80) and the dispersion medium (82) in the chamber (11). With agitation, the dispersoid is finely sheared by the dispersion medium (82). As a result, the emulsion (83) is formed in which droplets (84) of the dispersoid containing a detection matter (MD) in the specimen (80) are dispersed in the dispersion medium (82). As described above, mere rotation of the cartridge (100) about the rotational shaft (221) without using liquid feeding by air pressure control makes it possible to form the emulsion (83) in the chamber (11). Therefore, the emulsion (83) can be more simply prepared when processing a specimen using a cartridge.

The specimen processing cartridge according to a fourth aspect preferably further includes a liquid storing unit (30) that is fluidly connected to the chamber (11) and stores the dispersion medium (82) in advance, wherein the specimen processing cartridge is structured such to, by being rotated about the rotational shaft (221), transfer the dispersion medium (82) from the liquid storing unit (30) to the chamber (11). With such configuration, since the dispersion medium (82) is stored in advance, the operation of injecting the dispersion medium (82) into the cartridge (100) is unnecessary. Additionally, mere rotation of the cartridge (100) about the rotational shaft (221) without using liquid feeding by air pressure control makes it possible to transfer the dispersion medium (82) into the chamber (11). Thanks to the above, the emulsion (83) can be more simply prepared when processing a specimen using the cartridge (100).

According to one or more aspects, it is possible to more simply prepare an emulsion when processing a specimen using a cartridge.

### [First Embodiment]

Hereinafter, embodiments are described based on the drawings.

### (Overview of Specimen Processing Method)

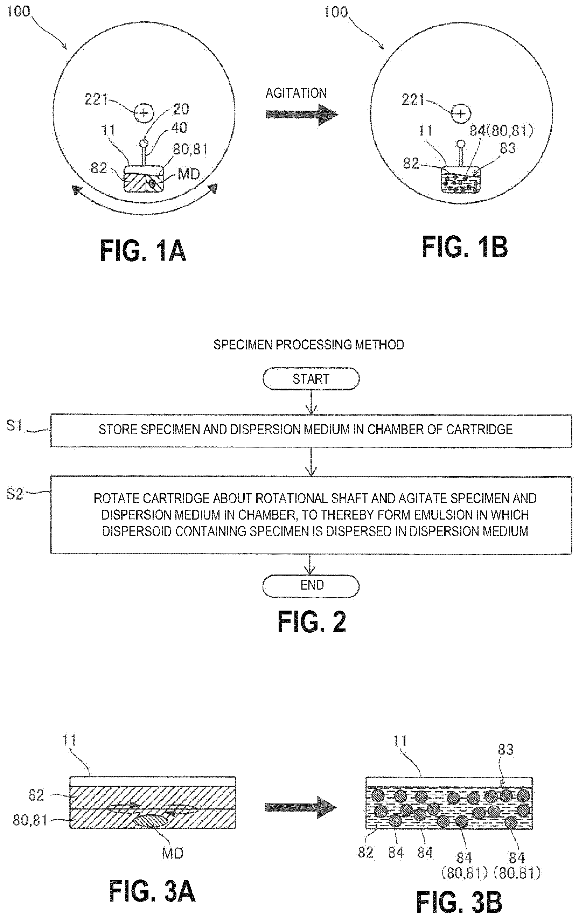

A specimen processing method according to a first embodiment is described with reference to FIG. 1A to FIG. 3B. The specimen processing method of a first embodiment is a specimen processing method that uses a cartridge 100 provided with a chamber 11 to store a liquid. The specimen processing method is a method of performing processing to detect a detection matter MD using the cartridge 100 injected with a specimen 80 containing the detection matter MD.

The specimen is, for example, a biological specimen collected from human being a subject. The specimen is, for example, a liquid such as body fluid or blood (whole blood, serum, or plasma) collected from a patient, a liquid obtained by subjecting the collected body fluid or blood to a predetermined pretreatment, or the like. The specimen contains, as the detection matter MD, nucleic acids such as DNA (deoxyribonucleic acid), cells and intracellular substances, proteins such as antigens or antibodies, peptides and the like, for example. For example, when the detection matter MD is a nucleic acid, an extraction liquid obtained by extracting the nucleic acid from blood or the like by predetermined pretreatment is injected into the cartridge 100 as the specimen 80.

In addition, the cartridge 100 may also store a reagent 81 to process the detection matter MD in the specimen 80. The cartridge 100 stores a dispersion medium 82. The reagent 81 to store the detection matter MD reacts with the detection matter MD or other substance bound to the detection matter MD. The reagent 81 to store the detection matter MD may be of one type or multiple types. The specimen 80, the reagent 81 to store the detection matter MD, and the dispersion medium 82 are liquids. Note that these liquids may contain matter other than liquid. The dispersion medium 82 is immiscible with specimen 80 and the reagent 81. Specifically, the mixed liquid of the specimen 80 and the reagent 81, and the dispersion medium 82 have a property of separating into separate phases. For example, the mixed liquid is water-based, and the dispersion medium 82 is oil-based. The specimen 80 and the reagent 81, and the dispersion medium 82 are agitated in the chamber 11 to form an emulsion 83. Specifically, the mixed liquid of the specimen 80 and the reagent 81, and the dispersion medium 82 is formed into a disperse system solution in which the dispersoid containing the specimen 80 and the reagent 81 is dispersed in the form of droplets 84 in the dispersion medium 82. In a first embodiment, the cartridge 100 does not have to store the reagent 81. It at least suffices to form the emulsion 83 in which the dispersoid containing the specimen 80 is dispersed in the dispersion medium 82.

The cartridge 100 is a replaceable consumable. Specifically, the cartridge 100 is discarded when it is used for measurement a preset number of times. The usable number of times of the cartridge 100 is one or more times. The cartridge is a replaceable component which collectively has the functions required to process the specimen 80.

The cartridge 100 is, for example, a flat plate-shaped member having a space formed therein. The cartridge 100 includes a chamber 11 capable of storing a liquid therein. One or more chambers 11 are provided in the cartridge 100. The chamber 11 is structured as an internal space with a volume capable of storing the specimen 80, the reagent 81, and the dispersion medium 82. The chamber 11 stores the specimen, the reagent 81, and the dispersion medium 82. The cartridge 100 may include a specimen introducing unit 20 that introduces a liquid into the chamber 11, a passage 40, a space functioning as a liquid storing unit, and the like. The cartridge 100 may form an internal space such as the chamber 11 or the passage 40 by, for example, bonding a transparent film on the surface of a member having a hole constituting the chamber 11, the passage, or the like formed therein to thereby close the opening.

The chamber 11 may store the reagent 81 and the dispersion medium 82 in advance, or does not have to store the reagent. The reagent 81 and the dispersion medium 82 may be injected into the chamber 11 not storing the reagent 81 and the dispersion medium 82 from a different portion in the cartridge 100 or from the outside of the cartridge 100.

As illustrated in FIG. 2, the specimen processing method of a first embodiment includes the following steps S1 and S2: (S1) Storing the specimen 80 and the dispersion medium 82 in the chamber 11 of the cartridge 100; and (S2) Rotating the cartridge 100 about a rotational shaft 221 to agitate the specimen 80 and the dispersion medium 82 in the chamber 11, to thereby form the emulsion 83 in which the dispersoid containing the specimen 80 is dispersed in the dispersion medium 82.

In step S1, the method of storing a liquid in the chamber 11 is not particularly limited. An operator may inject, by a procedure using a pipette or the like, the specimen 80 and the dispersion medium 82, and the reagent 81 to process the detection matter MD in the specimen 80 through the opening of the cartridge 100. In addition, the liquid may be injected through the opening of the cartridge 100 with a dispensing apparatus for dispensing liquid. The specimen 80 and the reagent 81 may be injected into the cartridge 100 in the state of being mixed in advance into a mixed liquid.

In step S1, the specimen 80, the reagent 81 to store the detection matter MD, and the dispersion medium 82 may be directly injected into the chamber 11. When the specimen 80, the reagent 81 to store the detection matter MD, and the dispersion medium 82 are to be stored in a section other than the chamber 11 of the cartridge 100, they are transferred via the passage 40 or the like to the chamber 11. As a result of step S1, the specimen 80, the reagent 81 to store the detection matter MD, and the dispersion medium 82 are stored in the same chamber 11.

In step S2, the cartridge 100 is rotated about the rotational shaft 221. The rotational shaft 221 is a shaft along the normal direction to the surface of the flat plate-shaped cartridge 100. In order to suppress vibration associated with the rotation, the rotational shaft 221 is preferably placed approximately at the center of gravity of the cartridge 100. In the case of the disk-shaped cartridge 100 as in FIG. 1A and FIG. 1B, the center of the circular cartridge 100 may be considered as the center of gravity.

Here, an inertial force associated with the rotation of the cartridge 100 acts on the chamber 11 to agitate the specimen 80, the reagent 81, and the dispersion medium 82 in the chamber 11. Specifically, an inertial force causes the dispersoid containing the specimen 80 and the reagent 81, and the dispersion medium 82 to move relative to each other in the chamber 11. Here, it is preferable to generate by rotation a turbulent flow of the dispersoid and the dispersion medium 82 in the chamber 11. This promotes the agitation. With agitation, the dispersoid is finely sheared by the dispersion medium 82.

As a result, the dispersoid is refined in the dispersion medium 82, forming in the chamber 11 multiple droplets 84 of the dispersoid compartmentalized by the interface between the dispersoid and the dispersion medium 82. Specifically, the emulsion 83 in which the droplets 84 of the dispersoid containing the detection matter MD are dispersed in the dispersion medium 82 is formed. The droplets 84 contained in the emulsion 83 contain the detection matter MD. When the reagent 81 to store the detection matter MD is stored in the chamber 11, the droplets 84 contain the detection matter MD and the reagent 81 to store the detection matter MD. In this case, it is possible to reliably process the detection matter MD with the reagent 81 in the finely compartmentalized individual droplets 84.

As described above, in the specimen processing method of a first embodiment, rotation of the cartridge 100 about the rotational shaft 221 forms the emulsion 83 in which the droplets 84 of the dispersoid are dispersed in the dispersion medium 82. As described above, mere rotation of the cartridge 100 about the rotational shaft 221 without using liquid feeding by air pressure control makes it possible to form the emulsion 83 in the chamber 11. Therefore, the emulsion 83 can be more simply prepared when processing a specimen using a cartridge.

### <Reaction Processing with Reagent>

After the emulsion 83 is formed, the detection matter MD and the reagent 81 contained in the dispersoid can be reacted with each other. In the emulsion 83, the detection matter MD and the reagent 81 are stored in the finely compartmentalized individual droplets 84, as illustrated in FIG. 3A and FIG. 3B. Thus, the compartmentalized individual molecules of the detection matter MD can be reacted with the reagent 81.

For example, after the emulsion 83 is formed, the detection matter MD and the reagent 81 contained in the dispersoid are reacted with each other by changing a temperature of the cartridge 100. This makes it possible to reliably cause a reaction between the detection matter MD and the reagent 81 by temperature change in the state in which the detection matter MD is stored for compartmentalization in the droplets 84 of the dispersoid together with the reagent 81 to process the detection matter MD. In addition, as compared with the case of causing the reaction between the detection matter MD and the reagent 81 to naturally proceed under the temperature of the setting environment of the cartridge 100, it is possible to efficiently cause a reaction between the detection matter MD and the reagent 81 using the temperature change even in the droplets 84 contained in the emulsion 83.

### (Operation of Changing Rotational Speed of Cartridge)

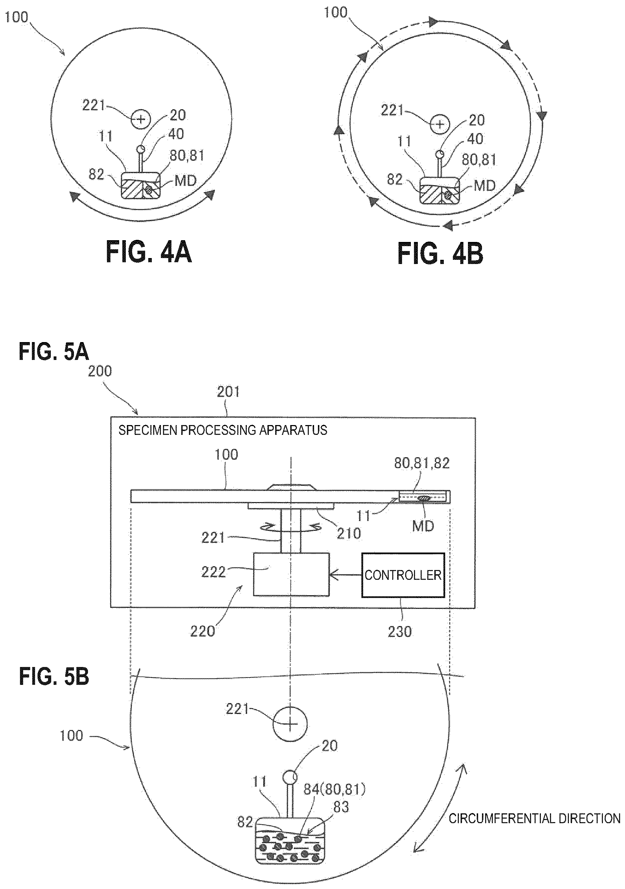

In the example illustrated in FIG. 4A and FIG. 4B, the emulsion 83 is formed in the chamber 11 by repeating an operation of changing a rotational speed of the cartridge 100 being rotated about the rotational shaft 221. Changing the rotational speed includes acceleration or deceleration of the cartridge 100 rotating in a certain rotational direction, and stopping by deceleration and acceleration in the opposite direction for the purpose of reversing the rotational direction of the cartridge 100 for reverse rotation.

In this example, the rotational speed of the cartridge 100 being rotated about the rotational shaft 221 is changed. As the rotational speed changes, the inertial force acting on the liquid in the chamber 11 varies. The variation of the inertial force agitates the dispersoid and the dispersion medium 82 in the chamber 11. Specifically, the change in inertial force attributed to the change in rotational speed causes the dispersoid and the dispersion medium 82 to move relative to each other in the chamber 11. Thereby, agitation is performed rapidly. Here, it is preferable to generate by changing the rotational speed a turbulent flow of the dispersoid and the dispersion medium 82 in the chamber 11. This promotes the agitation.

Then, the operation of changing the rotational speed of the cartridge 100 being rotated about the rotational shaft 221 is repeated. The operation of changing the rotational speed of the cartridge 100 is repeated, for example, a predetermined number of times. The operation of changing the rotational speed of the cartridge 100 may be repeated, for example, in a constant cycle, and may be repeated for a predetermined period of time after the operation of changing the rotational speed is started. As illustrated in FIG. 3A and FIG. 3B, in the chamber 11, the agitation efficiently proceeds as a result of repeated changes in rotational speed. By agitation, the dispersoid is finely sheared by the dispersion medium 82.

As described above, when the operation of changing the rotational speed of the cartridge 100 is repeated, the inertial force associated with the speed change acts to effectively agitate the specimen 80 and the dispersion medium 82 in the chamber 11, making it possible to finely shear the dispersoid with the dispersion medium 82 rapidly. As a result, the emulsion 83 can be efficiently prepared.

The operation of changing the rotational speed of the cartridge 100 includes an operation of reversing a rotational direction of the cartridge 100 or an operation of acceleration and deceleration in the same direction.

FIG. 4A illustrates an example operation of reversing the rotational direction of the cartridge 100. For example, the cartridge 100 is alternately rotated in one direction and the other direction about the rotational shaft 221 with the rotational shaft 221 at the center. In this case, when the one direction about the rotational shaft 221 is set positive (+) and the other direction is set negative (-), the rotational speed is such that positive, zero, negative, zero, positive ..., repeating acceleration from the stop state and deceleration until the stop state each time the rotational direction is reversed. This makes it possible to cause a large inertial force to act on the liquid in the chamber 11. As a result, the dispersoid and the dispersion medium 82 are efficiently agitated. As described above, in the case where the rotational direction of the cartridge 100 is reversed, a large inertial force can be exerted when reversing the rotational direction, making it possible to efficiently prepare the emulsion 83.

FIG. 4B illustrates an example operation of acceleration and deceleration in the same direction. FIG. 4B indicates, for convenience, acceleration with solid arrows and deceleration with dashed arrows in the circumferential direction of the cartridge 100. In FIG. 4B, the cartridge 100 is rotated in the clockwise direction, but may be in the counterclockwise direction. The cartridge 100 is rotated in a direction about the rotational shaft 221 with the rotational shaft 221 at the center, so as to alternately repeat acceleration and deceleration. Regarding the deceleration, the cartridge 100 may be decelerated until its rotation is stopped, or acceleration and deceleration may be repeated between predetermined high speed and low speed. In FIG. 4B, acceleration and deceleration are alternately repeated, and the cartridge 100 may be rotated in a non-alternately repeating pattern such as acceleration, acceleration, deceleration, acceleration, acceleration, deceleration, ..., for example. When the cartridge 100 is accelerated and decelerated in the same direction, it is unnecessary to reversely rotate the rotation mechanism for rotating the cartridge 100, which makes it possible to easily prepare the emulsion 83.

### (Overview of Specimen Processing Apparatus)

Next, an overview of a specimen processing apparatus 200 according to a first embodiment is described with reference to FIG. 5A and FIG. 5B.

The specimen processing apparatus 200 of a first embodiment is an apparatus for performing the above-described specimen processing method. Specifically, the specimen processing apparatus 200 is an apparatus that processes a detection matter MD contained in a specimen using a cartridge 100 provided with a chamber 11 to store a liquid. The specimen processing apparatus 200 may perform mixing of the dispersoid containing the specimen 80 with the dispersion medium 82, agitation, heating or cooling, moving of a solid or liquid used for processing, and various other operations inside the cartridge 100.

As illustrated in FIG. 5A, the specimen processing apparatus 200 includes a setting part 210, a rotation mechanism 220, and a controller 230. The setting part 210, the rotation mechanism 220, and the controller 230 are stored in, for example, a housing 201.

The housing 201 is a box-shaped member having an internal space of a predetermined volume, a combination of a frame and an exterior plate, or the like. For example, the housing 201 of the specimen processing apparatus 200 has a shape of a small box which can be set on a tabletop.

The setting part 210 can set the cartridge 100 provided with the chamber 11 to store the specimen 80 and the dispersion medium 82. The setting part 210 includes, for example, an upper surface which constitutes the setting surface of the cartridge 100, and supports the lower surface of the cartridge 100. The setting part 210 supports the cartridge 100 rotatably about the rotational shaft 221. Regarding the setting part 210 in the example of FIG. 5A, the setting part 210 itself is attached to the rotational shaft 221, and can rotate integrally with the rotational shaft 221. The setting part 210 may be provided separately from the rotational shaft 221, and may rotatably support the cartridge 100 with a bearing or the like, for example.

The rotation mechanism 220 can rotate the cartridge 100 set on the setting part 210 about the rotational shaft 221. The rotation mechanism 220 may include the rotational shaft 221 and a drive unit 222 which rotates the rotational shaft 221. The rotational shaft 221 extends in, for example, the vertical direction. In the example of FIG. 5A, the rotation mechanism 220 supports the setting part 210 fixed to one end portion of the rotational shaft 221, and rotates the rotational shaft 221 and the setting part 210 integrally with the drive unit 222 connected to the other end side of the rotational shaft 221. The drive unit 222 is, for example, an electric motor. Thus, the rotation mechanism 220 rotates the cartridge 100 placed on the setting part 210 in a horizontal plane about the rotational shaft 221. The rotation mechanism 220 is capable of changing the rotational speed of the rotational shaft 221 in at least two stages. The at least two stages may be, for example, stages of stopping and rotating, where the stopping corresponds to the rotational speed of zero.

The controller 230 can control the rotation mechanism 220. Specifically, the controller 230 performs control of causing the rotation mechanism 220 to rotate the cartridge 100 including the chamber 11 storing the specimen 80 and the dispersion medium 82, thereby agitating the specimen 80, reagent 81, and the dispersion medium 82 in the chamber 11, and forming an emulsion 83 in which the dispersoid containing the specimen 80 is dispersed in the dispersion medium 82. Thus, the controller 230 forms in the chamber 11 the emulsion 83 (see FIG. 5B) in which the dispersoid containing the specimen 80 is dispersed in the dispersion medium 82. The controller 230 is a computer including, for example, a processor such as a CPU and a memory such as a memory. The controller 230 may include, for example, a programmable device such as a field-programmable gate array (FPGA) configured for the specimen processing apparatus 200.

With the above configuration, the specimen processing apparatus 200 can perform the specimen processing method illustrated in FIG. 2 to form the emulsion 83. Specifically, when the cartridge 100 storing the specimen 80 and the dispersion medium 82 in the chamber 11 is set on the setting part 210, the specimen processing apparatus 200 rotates the cartridge 100 about the rotational shaft 221 with the controller 230 controlling the rotation mechanism 220. As a result, the specimen processing apparatus 200 agitates the specimen 80 and the dispersion medium 82 in the chamber 11 to form the emulsion 83 in the chamber 11 of the cartridge 100. As described later, the specimen processing apparatus 200 may be structured to store in the chamber 11 the specimen 80, the reagent 81, and the dispersion medium 82 placed in the cartridge 100 at a position other than the chamber 11. In addition, the specimen processing apparatus 200 may also perform processing of forming the emulsion 83 in which the dispersoid containing the specimen 80 and the reagent 81 is dispersed in the dispersion medium 82, and causing a reaction between the detection matter MD and the reagent 81 in each of the finely compartmentalized individual droplets 84.

As described above, in the specimen processing apparatus 200 of a first embodiment, mere rotation of the cartridge 100 about the rotational shaft 221 without using liquid feeding by air pressure control makes it possible to form the emulsion 83 in the chamber 11. As a result, it is possible to more simply prepare the emulsion 83 when processing a specimen using a cartridge. Moreover, in the case of forming an emulsion using liquid feeding by air pressure control, it is necessary to provide a mechanism for applying air pressure to the part for storing the dispersion medium 82 and the part for storing the specimen, and a mechanism for accurately controlling the air pressure. In contrast, in the configuration illustrated in FIG. 5A and FIG. 5B, the emulsion 83 can be formed only by providing the rotation mechanism 220. Therefore, the apparatus configuration can be simplified to obtain a smaller and simpler specimen processing apparatus 200.

Note that, as illustrated in FIG. 4A and FIG. 4B, the controller 230 may form the emulsion 83 in the chamber 11 by controlling the rotation mechanism 220 such that the rotation mechanism 220 repeats an operation of changing a rotational speed of the cartridge 100 being rotated about the rotational shaft 221. With such configuration, when the operation of changing the rotational speed of the cartridge 100 is repeated, the inertial force associated with the speed change acts to effectively agitate the specimen 80, the reagent 81, and the dispersion medium 82 in the chamber 11, making it possible to finely shear the dispersoid with the dispersion medium 82 rapidly. As a result, the emulsion 83 can be efficiently prepared.

### (Program)

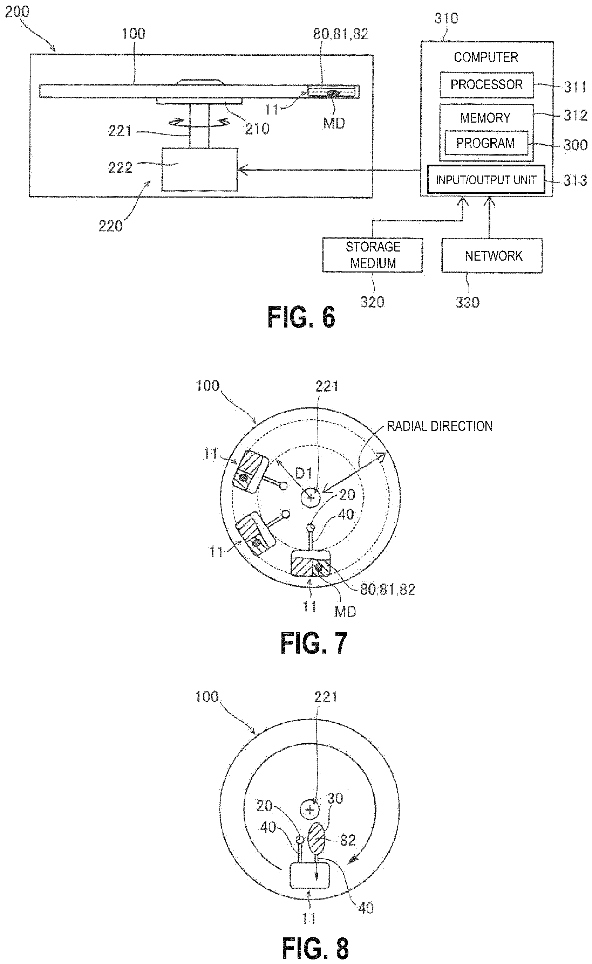

Next, a program 300 according to a first embodiment is described with reference to FIG. 6.

As illustrated in FIG. 6, the rotation mechanism 220 may be controlled by an external computer 310 without the controller 230 included in the specimen processing apparatus 200. The program 300 according to a first embodiment is a control program for causing the computer 310 to function as a controller of the specimen processing apparatus 200.

The computer 310 includes, for example, a processor 311 such as a CPU, a memory 312 including volatile memory and non-volatile memory, and an input/output unit 313 connected by wire or wirelessly to a network or an external device including the specimen processing apparatus 200. The program 300 is stored in the memory 312 and executed by the processor 311 to cause the computer 310 to function as a controller of the specimen processing apparatus 200.

Specifically, a program 300 causes a computer, connected to a specimen processing apparatus that processes a specimen 80 using a cartridge 100 including a chamber 11 to store a liquid, to function as a controller 230 that performs control of: causing a rotation mechanism 220 included in the specimen processing apparatus to rotate the cartridge 100, in which the chamber 11 stores the specimen 80 and a dispersion medium 82, about a rotational shaft 221; rotating the cartridge 100 to agitate the specimen 80 and the dispersion medium 82 in the chamber 11, to thereby form an emulsion 83 in which a dispersoid containing the specimen 80 is dispersed in the dispersion medium 82.

The program 300 may be provided by being stored on, for example, a computer-readable non-transitory storage medium 320. The program 300 stored on the storage medium 320 is read by a not-illustrated reading apparatus of the computer 310 and is thereby stored in the memory 312 of the computer 310. The storage medium 320 does not include a transitory propagation signal for transmitting the program 300, but includes, for example, a non-volatile semiconductor memory, an optical or magnetic storage medium, and the like. In addition, the program 300 may be provided from a network 330 connected to the computer 310 by, for example, a transitory propagation signal for transmitting the program 300.

As described above, the program 300 of a first embodiment causes the computer 310 to function as the controller 230 that performs control of rotating the cartridge 100 to agitate the specimen 80 and the dispersion medium 82 in the chamber 11, to thereby form an emulsion 83 in which a dispersoid containing the specimen 80 is dispersed in the dispersion medium 82. Thereby, mere rotation of the cartridge 100 about the rotational shaft 221 without using liquid feeding by air pressure control makes it possible to form the emulsion 83 in the chamber 11. As a result, it is possible to more simply prepare the emulsion 83 when processing a specimen using a cartridge.

### (Specimen Processing Cartridge)

Next, a specimen processing cartridge according to a first embodiment (hereinafter referred to as the cartridge 100) is described with reference to FIG. 1A and FIG. 1B.

The cartridge 100 includes a specimen introducing unit 20 that introduces a specimen 80. The specimen introducing unit 20 is, for example, an opening formed in the outer surface of the cartridge 100. The opening as the specimen introducing unit 20 may be closed in advance, and in that case, is unsealed by the operator when the cartridge 100 is used. The specimen introducing unit 20 at least receives the specimen 80. The specimen introducing unit 20 may receive a mixed liquid of the specimen 80 and the reagent 81 to store the detection matter MD.

The cartridge 100 includes the chamber 11 capable of storing a liquid therein. The chamber 11 may be a substantially closed space so that liquid does not leak inside the cartridge 100. The substantially closed space causes communication with the outside of the cartridge 100 through the specimen introducing unit 20. The chamber 11 may be structured such that the liquid in the chamber 11 does not flow back to the specimen introducing unit 20 under normal use conditions. The chamber 11 is capable of storing the introduced specimen 80, the reagent 81 to process the detection matter MD in the specimen 80, and the dispersion medium 82.

The cartridge 100 is structured to, by being rotated about a rotational shaft 221, agitate the specimen 80 and the dispersion medium 82 in the chamber 11, and form an emulsion 83 in which a dispersoid containing the specimen 80 is dispersed in the dispersion medium 82. The cartridge 100 may be structured to form the emulsion 83 in the chamber 11 by repetition of the operation of changing the rotational speed during rotation about the rotational shaft 221.

As described above, the cartridge 100 is, for example, a flat plate-shaped member having a space formed therein. The cartridge 100 does not have to be of a flat plate shape, and may have a block shape or a spherical shape. In order to agitate the dispersoid and the dispersion medium 82 in the chamber 11 by the inertial force in changing the rotational speed, it is preferable that the cartridge 100 be lightweight for easy acceleration and deceleration, and be not eccentric so as not to generate vibration with rotation. Specifically, a disk shape is preferable.

In addition, the shape of the chamber 11 formed in the cartridge 100 is not particularly limited, and is preferably a shape which extends in the circumferential direction about the rotational shaft 221 or in the tangential direction of a circle centered on the rotational shaft 221. When the dispersoid containing the specimen 80 and the reagent 81 is a water-based and the dispersion medium 82 is an oil-based liquid, for example, the dispersoid and the dispersion medium 82 are vertically separated in the chamber 11, as in FIG. 3A and FIG. 3B. For this reason, in the case of agitating the dispersoid and the dispersion medium 82 by the change in rotational speed, the larger the area of the interface of contact between the dispersoid and the dispersion medium 82, the easier it is to apply a shearing force between the dispersoid and the dispersion medium 82. Therefore, the chamber 11 preferably has a flat shape along the rotational direction. In addition, if the chamber 11 is in the radial direction with the rotational shaft 221 at the center, the inertial force associated with the change in the rotational speed is likely to vary between the inner peripheral side and the outer peripheral side in the radial direction. Therefore, the chamber 11 preferably extends in the circumferential direction about the rotational shaft 221 or in the tangential direction of a circle centered on the rotational shaft 221.

In the cartridge 100 of a first embodiment, rotation of the cartridge 100 about the rotational shaft 221 applies an inertial force associated with the rotation of the cartridge 100 to the chamber 11, which agitates the specimen 80 and the dispersion medium 82 in the chamber 11. With agitation, the dispersoid is finely sheared by the dispersion medium 82. As a result, the emulsion 83 is formed in which droplets 84 of the dispersoid containing a detection matter MD are dispersed in the dispersion medium 82. As described above, mere rotation of the cartridge 100 about the rotational shaft 221 without using liquid feeding by air pressure control makes it possible to form the emulsion 83 in the chamber 11. Therefore, the emulsion 83 can be more simply prepared when processing a specimen using a cartridge.

In the example illustrated in FIG. 7, the cartridge 100 includes multiple chambers 11. Specifically, the cartridge 100 is provided with multiple chambers 11 to form the emulsion 83 in which the dispersoid containing the specimen 80 is dispersed in the dispersion medium 82. This makes it possible to form the emulsion 83 by processing, collectively in one cartridge 100, multiple specimens 80 collected from different living bodies or the same specimens 80 in an amount exceeding the throughput of one chamber 11.

In the example of FIG. 7, the cartridge 100 includes multiple chambers 11 arranged in an arc form whose radial distances from the rotational shaft 221 are substantially equal. In FIG. 7, the multiple chambers 11 are provided at such positions that the rotational trajectories of the chambers 11 coincide with each other in a plan view. The circumferential movement speed of the chambers 11 during rotation of the cartridge 100 is proportional to the radial distance D1 from the rotational shaft 221 of each chamber 11. Therefore, when the distance of each chamber 11 from the rotational shaft 221 is different, the magnitudes of the inertial forces acting on the chambers 11 are different. Thus, the quality of the emulsions 83 prepared may vary from one chamber 11 to another. On the other hand, in FIG. 7 in which the multiple chamber 11 are arranged in an arc form with substantially equal radial distances from the rotational shaft 221, the magnitudes of the inertial forces acting on the respective chambers 11 during rotation are substantially equal. Therefore, the quality of the emulsions 83 prepared can be made more uniform. For example, rotation makes it possible to suppress variations in the size of droplets 84 and the number of droplets 84 contained in the emulsion 83 prepared in each chamber 11.

As illustrated in FIG. 8, in addition to the specimen introducing unit 20 and the chamber 11, the cartridge 100 can include a liquid storing unit 30 which stores a liquid used for processing. The liquid storing unit 30 is a space formed to have a predetermined volume in the cartridge 100 as in the chamber 11. The liquid storing unit 30 is connected to the chamber 11 via the passage 40 formed in the cartridge 100.

FIG. 8 illustrates, as an example of the liquid storing unit 30, a liquid storing unit 30 storing the dispersion medium 82 in advance. Specifically, the cartridge 100 includes the liquid storing unit 30 that is fluidly connected to the chamber 11 and stores the dispersion medium 82 in advance. Then, the cartridge 100 is structured to, by being rotated about the rotational shaft 221, transfer the dispersion medium 82 from the liquid storing unit 30 to the chamber 11.

Specifically, the liquid storing unit 30 is provided in the cartridge 100 at a position closer to the rotational shaft 221 than is the chamber 11. The passage 40 is provided, with respect to the liquid storing unit 30, at a position on the outer peripheral side in the radial direction with the rotational shaft 221 at the center, and is connected to the chamber 11. For this reason, when the cartridge 100 is rotated about the rotational shaft 221, a centrifugal force outwards in the radial direction acts on the dispersion medium 82 stored in the liquid storing unit 30. As a result, the dispersion medium 82 is transferred from the liquid storing unit 30 into the chamber 11 by the centrifugal force generated by the rotation. In the case of transferring the liquid from the liquid storing unit 30 into the chamber 11, it suffices to generate a centrifugal force of a magnitude necessary to move the liquid. Therefore, it is unnecessary to repeatedly change the rotational speed of the cartridge 100.

With the above configuration, since the dispersion medium 82 is stored in the cartridge 100 in advance, the operation of injecting the dispersion medium 82 into the cartridge 100 is unnecessary. Additionally, mere rotation of the cartridge 100 about the rotational shaft 221 without using liquid feeding by air pressure control makes it possible to transfer the dispersion medium 82 into the chamber 11. Thanks to the above, the emulsion 83 can be more simply prepared when processing a specimen using the cartridge 100.

### (Specific Configuration Example of Specimen Processing Method)

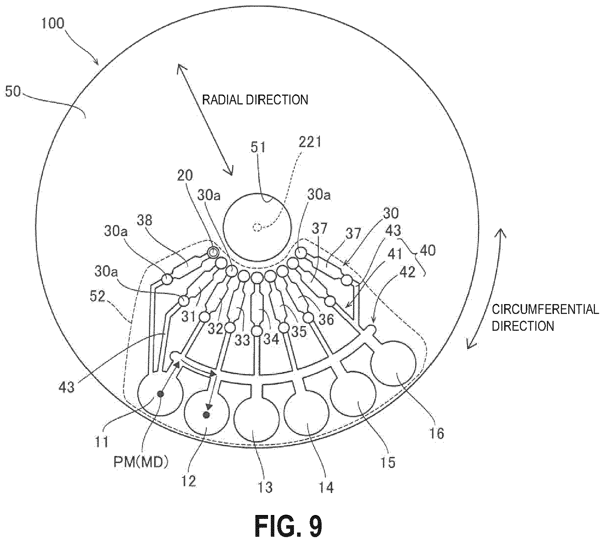

Next, a specific configuration example of the specimen processing method according to a first embodiment is described. Hereinafter, an example using the cartridge 100 illustrated in FIG. 9 is described.

### <Cartridge>

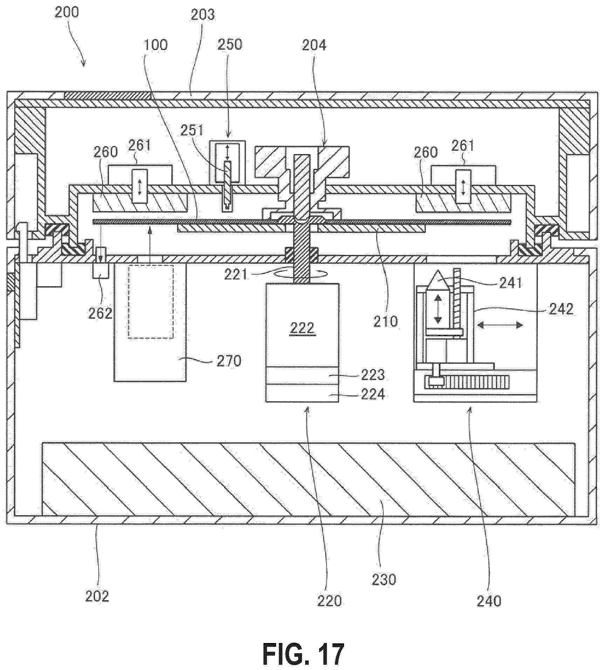

A specific configuration example of the cartridge 100 is described. In the example of FIG. 9, the cartridge 100 is a disk type cartridge being a plate-shaped and disk-shaped base plate 50. Each of the units in the cartridge 100 is formed by bonding together through hole portions formed in the base plate 50 and not-illustrated films covering the entire surface including the through hole portions on both surfaces of the base plate 50. The base plate 50 has such a thickness that facilitates temperature control of the cartridge 100 by the temperature adjusting unit 260 to be described later (see FIG. 17). For example, the thickness of the base plate 50 is several millimeters, specifically about 1.2 mm.

The base plate 50 is provided with a pore 51 and a specimen processing region 52. The specimen processing region 52 is provided with the multiple chambers 11 to 16, the specimen introducing unit 20, the multiple liquid storing units 30, and the multiple passages 40.

The pore 51 penetrates the base plate 50 at the center of the base plate 50. The cartridge 100 is set on the specimen processing apparatus 200 such that the center of the pore 51 coincides with the center of the rotational shaft 221. Hereinafter, the radial direction and the circumferential direction of a circle centered on the pore 51 are referred to as a "radial direction" and a "circumferential direction," respectively.

The specimen introducing unit 20 is an opening for introducing the specimen 80 into the cartridge 100. In the example of FIG. 9, the specimen introducing unit 20 is injected with a mixed liquid of the specimen 80 and the reagent 81 to store the detection matter MD. The mixed liquid is prepared in advance by the operator and injected into the cartridge 100, but the separately injected specimen 80 and reagent 81 may be mixed in the cartridge 100.

The specimen 80 is a solution containing nucleic acids as the detection matter MD. The nucleic acid is, for example, the DNA of a subject. This makes it possible to form the emulsion 83 in which fine droplets 84 of the dispersoid containing nucleic acids as the detection matter MD and the reagent 81 to store the detection matter MD are dispersed in the dispersion medium 82 (see FIG. 3A and FIG. 3B). As a result, individual molecules of the detection matter MD are compartmentalized into droplets 84, making it possible to reliably process individual molecules of the detection matter MD with the reagent 81.

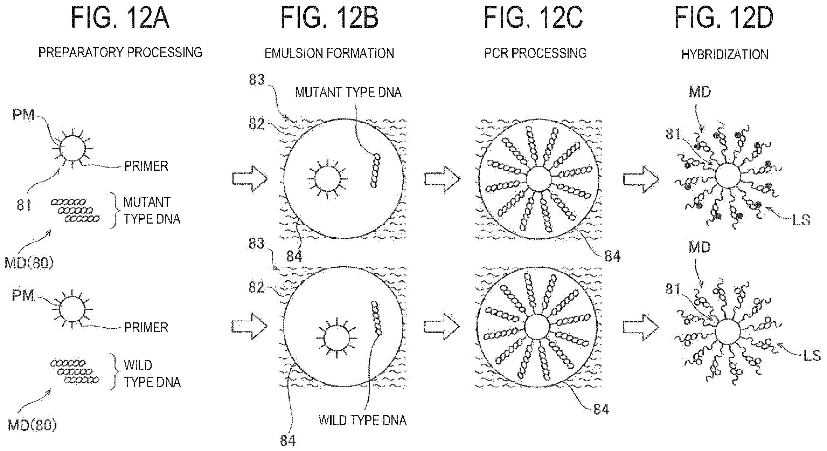

The reagent 81 includes a nucleic acid amplification reagent that amplifies the nucleic acid in the specimen 80. The nucleic acid amplification reagent contains substances necessary for PCR such as DNA polymerase. Moreover, the reagent 81 contains magnetic particles PM (see FIG. 12A to FIG. 12D) that bind to the nucleic acids in the specimen 80. The magnetic particles PM have a primer for nucleic acid amplification applied to their surfaces. The magnetic particles may be particles which contain a material having magnetism as a base material and are used for normal sample measurement. For example, it is possible to use magnetic particles using, as a base material, Fe₂O₃ and/or Fe₃O₄, cobalt, nickel, phyllite, magnetite, or the like. The dispersoid formed by mixing the specimen 80, the nucleic acid amplification reagent, and the magnetic particles PM is introduced from the specimen introducing unit 20 into the cartridge 100. In the dispersoid, the nucleic acids being the detection matter MD are bound to the magnetic particles PM.

The chambers 11 to 16 are chambers capable of storing a liquid. The chambers 11 to 16 are arranged in the circumferential direction near the outer periphery of the base plate 50. FIG. 9 illustrates an example of providing six chambers 11 to 16 in the cartridge 100.

The liquid storing units 30 are storage spaces for storing a liquid to be transferred to the chambers. Each of the liquid storing units 30 is connected to any one of the chambers via the passage 40. FIG. 9 illustrates an example of providing nine liquid storing units 30 in the cartridge 100. The liquid storing units 30 are arranged in the circumferential direction near the center of the base plate 50. Each liquid storing unit 30 is placed at a position closer to the rotational shaft 221 than is the connected chamber. For this reason, rotation of the cartridge 100 about the rotational shaft 221 makes it possible to transfer by centrifugal force the liquid stored in the liquid storing units 30 from the liquid storing units 30 to the chambers.

The liquid storing unit 30 includes a sealing body 30a that seals a connecting portion with the chamber 11. The liquid in the liquid storing units 30 is stored in advance in the liquid storing units 30 in the manufacture of the cartridge 100. The sealing body 30a is a plug which closes the liquid storing unit 30 and the external space. The sealing body 30a is structured to be openable when pressed from above by the unsealing mechanism 250 of the specimen processing apparatus 200 (see FIG. 17). Before the sealing body 30a is unsealed, the reagent in the liquid storing unit 30 does not flow into the passage 40, and when the sealing body 30a is unsealed, the reagent in the liquid storing unit 30 flows out.

Note that the liquid storing unit 30 includes a specimen storing unit 38 which stores the mixed liquid injected from the specimen introducing unit 20. Rotation of the cartridge 100 about the rotational shaft 221 transfers the mixed liquid of the specimen 80 and the reagent 81 from the specimen storing unit 38 to the chamber 11.

Each of the liquid storing units 30 storing the reagent in advance stores the reagent which enables measurement once. Specifically, the cartridge 100 includes a liquid storing unit 30 storing the reagent which enables measurement once on the detection matter. In the cartridge 100 configured as described above, a different reagent is stored in each cartridge 100 in a disposable manner.

In FIG. 9, the six chambers 11, 12, 13, 14, 15, and 16 of substantially the same shape are arranged adjacent to each other in the circumferential direction, and are connected via the passage 40 extending in the circumferential direction. Among the six chambers 11 to 16, molecules of the detection matter are sequentially transferred one by one via the passage 40 from one side (the side of the chamber 11) to the other side (the side of the sixth chamber 16).

The passage 40 includes six radially extending radial regions 41 and a circumferentially extending arc-shaped circumferential region 42. The circumferential region 42 is connected to the six radial regions 41. The six radial regions 41 are connected to the respective chambers 11 to 16. Each liquid storing unit 30 is connected to the chamber or passage via a substantially radially extending flow path 43.

The combination of the rotation of the cartridge 100 and the action of the magnetic force transfers the detection matter MD bound to the magnetic particles PM to the passage 40 and other chambers 11. Specifically, the magnetic particles PM are moved by the magnetic force in the radial direction between the inside of the chamber 11 and the arc-shaped circumferential region 42 of the passage 40. As the cartridge 100 is rotated, the magnetic particles PM move in the circumferential direction in the arc-shaped circumferential region 42. When the magnetic particles PM move to the position of the adjacent chamber, the magnetic particles PM are moved by the magnetic force in the radial direction between the arc-shaped circumferential region 42 of the passage 40 and the inside of the adjacent chamber. As described above, the combination of the radial movement by the action of magnetic force and the circumferential movement by rotation sequentially moves the magnetic particles PM bound to the detection matter MD to the chambers 11 to 16.

Note that the specimen processing region 52 in the example of FIG. 9 is formed only in a region of approximately one third of the base plate 50. However, the present invention is not limited to this, and two more specimen processing regions 52 may be formed in the remaining region corresponding to two thirds of the base plate 50, and three specimen processing regions 52 may be provided on the base plate 50. Note that one specimen processing region 52 may be formed over a region larger than the region of one third of the base plate 50.

In addition, the chamber 11 and the passage 40 are not limited in number and shape to those illustrated in FIG. 9. The configuration of each unit of the specimen processing region 52 is determined in accordance with the content of the specimen processing assay performed in the specimen processing region 52.

### <Flow of Specimen Processing>

Next, an example of a specific assay using the cartridge 100 of FIG. 9 is described.

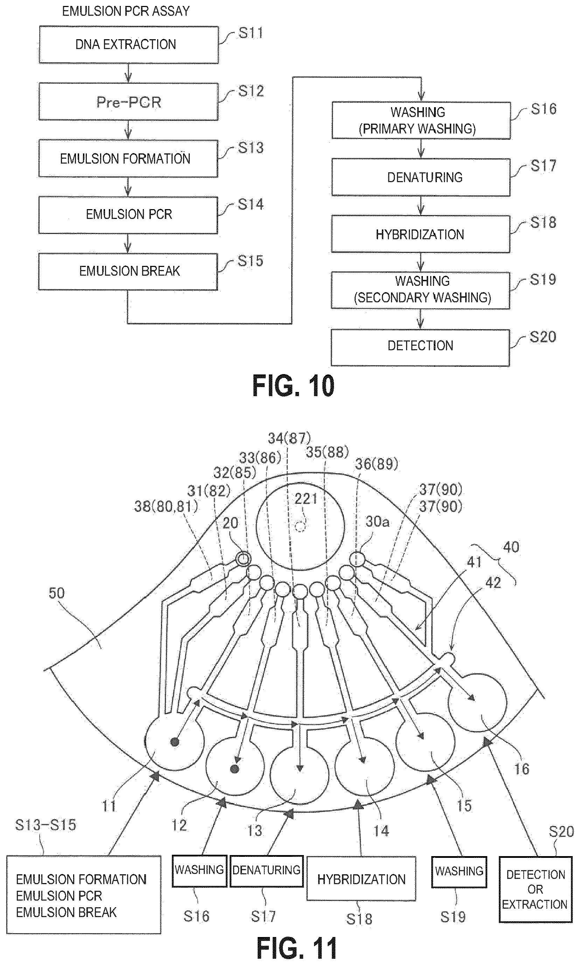

FIG. 10 illustrates a flow of an emulsion PCR assay. FIG. 12A to FIG. 12D are diagrams for explaining the progress of reaction in the emulsion PCR assay. The processing using the cartridge 100 illustrated in FIG. 9 includes steps S13 to S19, and may further include step S20.

In step S11, DNA is extracted by pretreatment from a specimen such as blood (see FIG. 12A). The pretreatment is performed using a dedicated nucleic acid extracting device.

In step S12, the extracted DNA is amplified by pre-PCR processing (see FIG. 12A). The pre-PCR processing is a process for preamplifying the DNA, contained in the extraction liquid after the pretreatment, to such an extent that enables subsequent emulsion preparation processing. The pre-PCR processing mixes the extracted DNA with a reagent for PCR amplification containing a polymerase and a primer, and amplifies the DNA in the dispersoid by temperature control with a thermal cycler. The thermal cycler performs thermal cycling in which the dispersoid is subjected multiple times to a cycle of changing to multiple different temperatures. Depending on the concentration of the nucleic acid extracted, the pre-PCR processing may be omitted.

Step S13 is an emulsion forming step of dispersing in the dispersion medium 82 the dispersoid containing nucleic acids (DNAs) being the detection matter MD, a nucleic acid amplification reagent, and magnetic particles PM as nucleic acid carriers. The reagent for nucleic acid amplification reaction contains substances necessary for PCR such as DNA polymerase. In step S13, the cartridge 100 is used to form the emulsion 83 which contains a DNA and a reagent containing magnetic particles, a polymerase, and the like (see FIG. 12B). Specifically, droplets 84 which contain therein a dispersoid containing a DNA and a nucleic acid amplification reagent containing magnetic particles PM, a polymerase, and the like are formed, and the dispersoid including a large number of droplets 84 is dispersed in the dispersion medium 82. As a result, the DNAs being the detection matter MD are subjected to limiting dilution (dilution so that one or zero target component is in each fine compartment) and dispersed in fine compartments. Specifically, the individual droplets 84 in the emulsion 83 form into fine compartments, and the droplets 84 are formed such that approximately one magnetic particle and approximately one target DNA molecule being the detection matter MD are contained in a droplet 84.

Step S14 is an emulsion PCR step of amplifying the nucleic acids (DNAs) in the droplets 84 formed by the emulsion forming step. In step S14, due to the temperature control with a thermal cycler, the DNAs bind to the primer on the magnetic particle PM and amplified in each droplet 84 of the emulsion 83 (emulsion PCR) (see FIG. 12C). This amplifies the target DNA molecule in the individual droplets 84. Specifically, in each droplet 84, amplification products of the nucleic acids are formed. In the droplet 84, the amplified nucleic acids bind to the magnetic particle PM being a carrier via the primer.

Step S15 is an emulsion breaking step of breaking the droplets 84 containing the magnetic particles PM carrying the amplification products of the nucleic acids (DNAs) by the emulsion PCR step. In other words, step S15 is a step of demulsifying the emulsion 83 after the emulsion PCR step. After amplification of DNA on the magnetic particles PM in step S14, the emulsion 83 is broken in step S15, and the magnetic particles PM containing the amplified DNAs are taken out of the droplets 84 (emulsion break). A reagent 85 to demulsify the emulsion (see FIG. 11) is used to destruct the emulsion. The reagent 85 to demulsify the emulsion contains an alcohol or a surfactant.

Step S16 is a washing step of washing the magnetic particles PM taken out of the droplets 84 by the demulsification in the emulsion breaking step. In step S16, the magnetic particles PM taken out of the droplets 84 are washed by a reagent 86 to wash the detection matter MD (see FIG. 11) (primary washing). The reagent 86 to wash the detection matter MD contains, for example, alcohol. The alcohol removes the oil films on the magnetic particles.

Step S17 is a denaturing step of denaturing the amplified double-stranded DNAs binding to the magnetic particles PM into single strands. In step S17, the washed magnetic particles PM are denatured by a reagent 87 to denature the nucleic acid (see FIG. 11). The reagent 87 to denature the nucleic acid denatures by pH change the amplified double-stranded DNAs into single strands. The reagent 87 to denature the nucleic acid includes an alkaline reagent such as an aqueous solution of sodium hydroxide.

Step S18 is a hybridizing step of reacting the DNA amplification products with a labeling substance. After the denaturation step, in step S18, the DNAs denatured to single strands on the magnetic particles PM are hybridized, by a reagent 88 containing a labeling substance LS that reacts with the amplification products of the nucleic acids, with the labeling substance LS (see FIG. 11) (hybridization) (see FIG. 12D). The labeling substance LS includes, for example, a substance which emits fluorescence. The labeling substance LS is designed to specifically bind to the DNA to be detected.

In step S19, the magnetic particles PM carrying the DNAs bound to the labeling substance LS are washed with a reagent 89 to wash the detection matter MD (see FIG. 11) (secondary washing). In the secondary washing step, phosphate buffered saline (PBS) is used as a washing liquid, for example. PBS removes unreacted labeling substance having not bound to DNA (including labeling substance nonspecifically adsorbed to the magnetic particles).

In step S20, the DNA being the detection matter MD is detected via the hybridized labeling substance LS. DNA is detected by, for example, a flow cytometer. In the flow cytometer, magnetic particles containing DNAs bound to the labeling substance flow through a flow cell, and the magnetic particles are irradiated with laser light. The fluorescence of the labeling substance emitted by the irradiation laser light is detected.

DNA may be detected by image processing. For example, magnetic particles containing DNAs bound to the labeling substance are dispersed on a flat slide, and an image of the dispersed magnetic particles is captured by a camera unit. The number of magnetic particles emitting fluorescence is counted based on the captured image. As described above, the specimen processing method of a first embodiment may include processing the detection matter MD with the reagent 88 containing the labeling substance LS to label the detection matter MD contained in the specimen 80, and detecting a signal based on the labeling substance LS. In addition, the specimen processing apparatus 200 of a first embodiment may be provided with a detection unit to detect a signal based on the labeling substance LS.

Next, details of processing using the cartridge 100 is described with reference to FIG. 11.

The cartridge 100 includes the chamber 11 in which the emulsion 83 is formed. As illustrated in FIG. 11, steps S13 to S15 are performed in the chamber 11.

The liquid storing unit 30 includes a first liquid storing unit 31 to store the dispersion medium 82. The dispersion medium 82 includes an oil that is immiscible with the specimen 80 and the reagent 81. Thus, in this example, the dispersoid is water-based, and the dispersion medium 82 is oil-based. The dispersion medium 82 is stored in advance in the first liquid storing unit 31 in the manufacture of the cartridge 100.

### <Emulsion Formation>

In step S13, first, the dispersoid containing nucleic acids (DNAs) being the detection matter MD, the nucleic acid amplification reagent, and magnetic particles PM as nucleic acid carriers are injected from the specimen introducing unit 20 to the specimen storing unit 38. In addition, the sealing body 30a of the first liquid storing unit 31 is unsealed. Then, the cartridge 100 is rotated about the rotational shaft 221. The centrifugal force associated with the rotation transfers the dispersion medium 82 from the first liquid storing unit 31 to the chamber 11, and transfers the dispersoid from the specimen storing unit 38. Thus, mere rotation of the cartridge 100 enables not only producing the emulsion 83 but also an operation of storing the dispersion medium 82 in the chamber 11. Therefore, since it is not necessary to apply air pressure to, for example, the first liquid storing unit, the specimen can be more easily processed.

The chamber 11 stores the introduced specimen 80, the reagent 81 to process the detection matter MD in the specimen 80, and the dispersion medium 82. Then, the cartridge 100 forms in the chamber 11 the emulsion 83 in which the dispersoid containing the specimen 80 and the reagent 81 is dispersed in the dispersion medium 82 by repetition of the operation of changing the rotational speed during rotation about the rotational shaft 221.

Here, rotation of the cartridge 100 forms in the chamber 11 the emulsion 83 containing droplets 84 of the dispersoid each storing one molecule or one particle of the detection matter MD. Thus, as illustrated in FIG. 12A to FIG. 12D, individual molecules of the detection matter MD contained in the specimen can be subjected to compartmentalization into the respective droplets 84 contained in the emulsion 83, and can be reliably processed. As a result, even the detection matter MD contained in an extremely small amount in the specimen can be processed with high accuracy. Therefore, it is possible to improve the detection accuracy also in the detection of the processed detection matter MD.

### <Emulsion PCR>

In step S14, after the emulsion 83 is formed, a temperature of the cartridge 100 is cyclically changed to multiple temperature ranges to amplify the nucleic acid contained in the dispersoid. Thus, it is possible to perform thermal cycling to efficiently amplify nucleic acids in the droplets 84 contained in the emulsion 83 (see FIG. 12A to FIG. 12D).

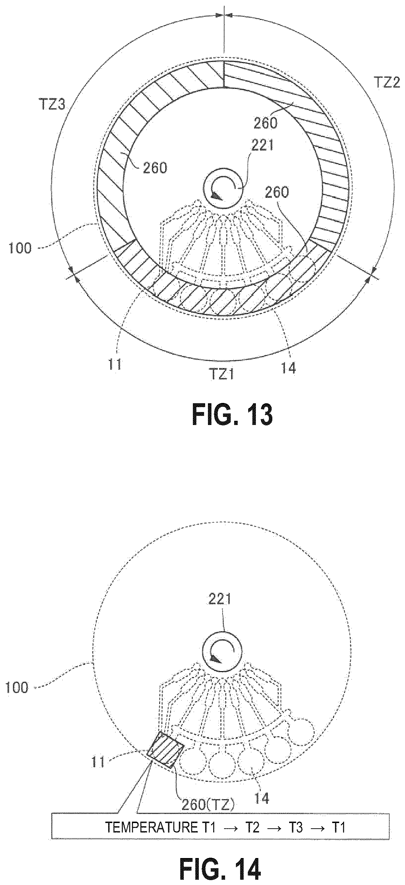

The thermal cycling is performed in the chamber 11 by the temperature adjusting unit 260 included in the specimen processing apparatus 200 (see FIG. 13). The temperature adjusting unit 260 is an apparatus that changes the temperature of the cartridge 100 at least by heating. The temperature adjusting unit 260 can be, for example, a heating apparatus such as a heater or an apparatus capable of heating and cooling such as a Peltier element. In step S14, the temperature of the cartridge 100 is changed by bringing the cartridge 100 into contact with a temperature adjusting unit 260. Thus, as compared with the case of heating the cartridge 100 in a non-contact manner, heat can be efficiently conducted by bringing the temperature adjusting unit 260 into contact with the cartridge 100. Therefore, the temperature of the cartridge 100 can be easily changed.

The temperature adjusting unit 260 adjusts temperature by contacting the cartridge 100 from one or both of the upper surface and the lower surface of the disk-shaped cartridge 100. In FIG. 13, in a plan view, the temperature adjusting unit 260 having a shape along the circumferential direction about the rotational shaft 221 is used, for example. In the example of FIG. 13, the temperature adjusting unit 260 is provided in a band-shaped area coinciding with the rotational trajectory of the chamber 11 in a plan view. Multiple temperature adjusting units 260 are provided in the circumferential direction, and form temperature zones TZ set to different temperatures. In FIG. 13, three temperature adjusting units 260 form three temperature zones TZ1 to TZ3 equally divided in the range of 120 degrees in the circumferential direction. The temperature zones TZ1 to TZ3 are set to three temperatures of low temperature, medium temperature, and high temperature in a range of, for example, 30°C or more and 90°C or less. The thermal cycling is performed by rotating the cartridge 100 and placing the chamber 11 in which the emulsion 83 is formed sequentially in the temperature zones TZ1 to TZ3. The number of temperature adjusting units 260 (that is, the number of temperature zones TZ) is not limited to this, and may be four or more. In the configuration of FIG. 13, since the set temperature of each temperature adjusting unit 260 can be kept constant, temperature control is possible easily and accurately.

In addition, in FIG. 14, the thermal cycling is performed by one temperature adjusting unit 260 in a plan view, for example. In the example of FIG. 14, the temperature adjusting unit 260 is provided at a position on the rotational trajectory of the chamber 11 in a plan view. The temperature adjusting unit 260 can store one chamber 11 in the formed temperature zone TZ. The temperature adjusting unit 260 forms a temperature zone TZ which can be changed to a desired temperature within a variable range with a Peltier element or the like. In FIG. 14, one temperature adjusting unit 260 forms a temperature zone TZ whose temperature changes in three stages. The temperature zone TZ is changed to three temperatures T1 to T3 of low temperature, medium temperature, and high temperature in a range of, for example, 30°C or more and 90°C or less. The thermal cycling is performed by rotating the cartridge 100, placing the chamber 11 in which the emulsion 83 is formed at the position on the temperature zone TZ, and repeatedly changing the temperature of the temperature adjusting unit 260. The number of temperature stages changed by the temperature adjusting unit 260 is not limited to this, and may be four or more. In the configuration of FIG. 14, since one temperature adjusting unit 260 can process one chamber 11, the apparatus configuration to perform thermal cycling can be simplified. Note that, in the case of providing multiple chambers 11, the thermal cycling can be performed simultaneously if multiple temperature adjusting units 260 are also provided.

With the above configuration PCR processing is performed in the emulsion 83. After the thermal cycling, the temperature adjusting unit 260 is released from the cartridge 100. The cartridge 100 is returned to room temperature.

### <Emulsion Break>

Back to FIG. 11, the liquid storing unit 30 includes a second liquid storing unit 32 that is connected to the chamber 11 and stores a reagent 85 to demulsify the emulsion when mixed. In step S15, after the nucleic acid contained in the dispersoid is amplified, the reagent 85 to demulsify the emulsion is transferred from the second liquid storing unit 32 to the chamber 11 in which the emulsion 83 is formed.

First, after amplification of the nucleic acid contained in the dispersoid, the sealing body 30a of the second liquid storing unit 32 is unsealed. Thus, the sealing body 30a makes it possible to prevent unintended feeding of the reagent 85 to demulsify the emulsion from the second liquid storing unit 32. Therefore, the emulsion 83 can be demulsified after reliably forming the emulsion 83 and amplifying nucleic acids.

Next, when the cartridge 100 is rotated about the rotational shaft 221, the reagent 85 to demulsify the emulsion is transferred from the second liquid storing unit 32 to the chamber 11 in which the emulsion 83 is formed. This makes it possible to take out nucleic acids amplified in the droplets 84 contained in the emulsion 83 with the reagent 85 to demulsify the emulsion. Here, unlike in the case of liquid feeding by air pressure or the like, mere rotation of the cartridge 100 about the rotational shaft 221 causes the reagent 85 to demulsify the emulsion to be fed to the chamber 11, making it possible to simply demulsify the emulsion 83.

After the reagent 85 to demulsify the emulsion is transferred into the chamber 11, the emulsion 83 is broken, and the magnetic particles PM containing the amplified nucleic acids are taken out of the droplets 84. The inside of the chamber 11 may be agitated by rotating the cartridge 100 and changing the rotational speed. This agitates the reagent 85 to demulsify the emulsion and the emulsion 83, making it possible to perform efficient demulsification.

### <Primary Washing>

The cartridge 100 includes a second chamber 12 that is connected to the chamber 11. The liquid storing unit 30 includes a third liquid storing unit 33 that is connected to the second chamber 12 and stores a reagent 86 to wash the detection matter MD. In step S16, after the nucleic acid at least contained in the dispersoid is amplified, the cartridge 100 is rotated about the rotational shaft 221 to transfer the reagent 86 to wash the detection matter MD from the third liquid storing unit 33 to the second chamber 12.

For example, the sealing body 30a of the third liquid storing unit 33 is unsealed at the same timing as that of unsealing the sealing body 30a of the second liquid storing unit 32 after the nucleic acid contained in the dispersoid is amplified. Thus, the sealing body 30a makes it possible to prevent unintended feeding of the reagent 86 to wash the detection matter MD from the third liquid storing unit 33. In addition, if the second liquid storing unit 32 and the third liquid storing unit 33 are unsealed at the same timing, the operation of rotating the cartridge 100 for liquid feeding may be performed only once.

As described above, the reagent 86 to wash the detection matter MD contains an alcohol. In step S14, in amplifying the nucleic acid contained in the dispersoid, the temperature of the cartridge 100 is changed in a range of 30°C or more and 90°C or less. For this reason, when the reagent 86 to wash the detection matter MD is affected by the temperature change, the alcohol may vaporize. Thus, when the reagent 86 to wash the detection matter MD is fed to the second chamber 12 after amplification of the nucleic acids, it is possible to suppress the influence of the temperature change on the reagent 86 to wash the detection matter MD. This configuration is particularly effective in that the vaporization of alcohol can be suppressed in the case where the reagent 86 to wash the detection matter MD contains an alcohol.

In step S16, when the reagent 85 to demulsify the emulsion is transferred to the chamber 11 and then a magnetic force is applied to the cartridge 100, the nucleic acid bound to the magnetic particles PM is transferred from the demulsified liquid generated by the demulsification in the chamber 11 to the second chamber 12. Since the magnetic particles PM carrying nucleic acids are taken out of the droplets 84 by demulsification, the magnetic particles PM are moved to the second chamber 12 by the magnetic force and the rotation of the cartridge 100, as described above.

In the second chamber 12, the magnetic particles PM carrying nucleic acids are washed with the reagent 86 to wash the detection matter MD.

In step S16, as described above, it is possible to simply transfer the detection matter MD to the second chamber 12 by merely applying an external magnetic force to the cartridge 100 without feeding by air pressure or the like. In addition, in the case of liquid feeding by air pressure or the like, the detection matter MD in the chamber 11 is transferred together with the liquid. On the other hand, in the magnetic transfer, only the detection matter MD bound to the magnetic particles PM can be transferred to the second chamber 12 while leaving the liquid. As a result, it is possible to efficiently wash the nucleic acids in the second chamber 12 by suppressing the transfer of unnecessary components to the second chamber 12.

### <Denaturing>

The cartridge 100 includes a third chamber 13 that is connected to the second chamber 12. The liquid storing unit 30 includes a fourth liquid storing unit 34 that is connected to the third chamber 13 and stores a reagent 87 to denature the nucleic acid.

In step S17, after the nucleic acid at least contained in the dispersoid is amplified, the cartridge 100 is rotated about the rotational shaft 221 to transfer the reagent 87 to denature the nucleic acid from the fourth liquid storing unit 34 to the third chamber 13. Thus, in step S17, the nucleic acids after being subjected to the processing of washing the nucleic acids in the third chamber 13 can be further subjected to processing of denaturing the nucleic acids in the cartridge 100. Additionally, also in the case of performing the processing of denaturing the nucleic acids, mere rotation of the cartridge 100 about the rotational shaft 221 makes it possible to feed liquid to the third chamber 13 instead of liquid feeding using air pressure or the like. Therefore, it is possible to simply perform the processing of denaturing the nucleic acids.

The sealing body 30a of the fourth liquid storing unit 34 is unsealed, for example, at the same timing as that of unsealing the sealing bodies 30a of the second liquid storing unit 32 and the third liquid storing unit 33 after the nucleic acid contained in the dispersoid is amplified. Thus, the sealing body 30a makes it possible to prevent unintended feeding of the reagent 87 to denature the nucleic acid from the fourth liquid storing unit 34. In addition, if the second liquid storing unit 32, the third liquid storing unit 33, and the fourth liquid storing unit 34 are unsealed at the same timing, the operation of rotating the cartridge 100 for liquid feeding may be performed only once.

In step S17, when the reagent 87 to denature the nucleic acid is transferred to the chamber 11 and then a magnetic force is applied to the cartridge 100, the nucleic acids bound to the wash processed magnetic particles PM are transferred from the second chamber 12 to the third chamber 13. The magnetic particles PM are moved to the third chamber 13 by the magnetic force and the rotation of the cartridge 100, as described above.

In the third chamber 13, the reagent 87 to denature the nucleic acid denatures amplified double-stranded DNA into single strands.

### <Hybridization>

The cartridge 100 includes a fourth chamber 14 that is connected to the third chamber 13. The liquid storing unit 30 includes a fifth liquid storing unit 35 that is connected to the fourth chamber 14 and stores a reagent 88 containing a labeling substance LS that reacts with an amplification product of the nucleic acid.

In step S18, after the nucleic acid contained in the dispersoid is amplified, the cartridge 100 is rotated about the rotational shaft 221 to transfer the reagent 88 containing the labeling substance LS from the fifth liquid storing unit 35 to the fourth chamber 14.

The sealing body 30a of the fifth liquid storing unit 35 is unsealed, for example, at the same timing as that of unsealing the sealing bodies 30a of the second liquid storing unit 32 to the fourth liquid storing unit 34 after the nucleic acid contained in the dispersoid is amplified. If the second liquid storing unit 32 to the fifth liquid storing unit 35 are unsealed at the same timing, the operation of rotating the cartridge 100 for liquid feeding may be performed only once.

In step S18, when the reagent 88 containing the labeling substance LS is transferred to the fourth chamber 14 and then a magnetic force is applied to the cartridge 100, the nucleic acids bound to the denaturing processed magnetic particles PM are transferred from the third chamber 13 to the fourth chamber 14. The magnetic particles PM are moved to the fourth chamber 14 by the magnetic force and the rotation of the cartridge 100, as described above.

In step S18, change of the temperature of the cartridge 100 causes a reaction between the nucleic acid and the labeling substance LS transferred to the fourth chamber 14. This makes it possible to perform, in the cartridge 100, not only the processing of denaturing the nucleic acids but also the processing of further labeling the nucleic acids being the detection matter MD for the purpose of detection. Additionally, also in the case of performing the processing of labeling the nucleic acids, mere rotation of the cartridge 100 about the rotational shaft 221 makes it possible to feed liquid to the fourth chamber 14 instead of liquid feeding using air pressure or the like. Therefore, it is possible to simply perform the processing of labeling the nucleic acids.

The hybridization processing can be performed with the temperature adjusting unit 260 (see FIG. 13 and FIG. 14) which performs the above PCR thermal cycling. Specifically, the temperature adjusting unit 260 causes a reaction between the nucleic acid and the labeling substance LS in the fourth chamber 14 by raising the temperature of the cartridge 100 by at least heating. Thus, both the processing of amplifying the nucleic acids and the processing of labeling the nucleic acids can be performed in the cartridge 100 simply by at least heating the cartridge 100. Therefore, not only the preparation of the emulsion 83 but also the processing of amplifying the nucleic acids and the processing of labeling the nucleic acids using the cartridge 100 can be more simply performed.

In the fourth chamber 14, the labeling substance LS in the reagent 88 containing the labeling substance LS labels the target DNA by binding to the DNA being the detection matter MD supported on the magnetic particles PM (see FIG. 12A to FIG. 12D).

### <Secondary Washing>

The cartridge 100 includes a fifth chamber 15 that is connected to the fourth chamber 14. The liquid storing unit 30 includes a sixth liquid storing unit 36 that is connected to the fifth chamber 15 and stores the reagent 89 to wash the detection matter MD. In step S19, after the nucleic acid at least contained in the dispersoid is amplified, the cartridge 100 is rotated about the rotational shaft 221 to transfer the reagent 89 to wash the detection matter MD from the sixth liquid storing unit 36 to the fifth chamber 15.

The sealing body 30a of the sixth liquid storing unit 36 is unsealed, for example, at the same timing as that of unsealing the sealing bodies 30a of the second liquid storing unit 32 to the fifth liquid storing unit 35. Thus, the operation of rotating the cartridge 100 for liquid feeding may be performed only once.

In step S19, when the reagent 89 to wash the detection matter MD is transferred to the fifth chamber 15 and then a magnetic force is applied to the cartridge 100, the magnetic particles PM bound to the labeled nucleic acids are transferred from the fourth chamber 14 to the fifth chamber 15. The magnetic particles PM are moved to the fifth chamber 15 by the magnetic force and the rotation of the cartridge 100, as described above.

In the fifth chamber 15, the magnetic particles PM bound to the labeled nucleic acids are washed with the reagent 89 to wash the detection matter MD.

### <Detection>

The cartridge 100 includes a sixth chamber 16 that is connected to the fifth chamber 15. The liquid storing unit 30 includes a seventh liquid storing unit 37 that is connected to the sixth chamber 16 and stores a reagent 90 to disperse the detection matter MD. The example of FIG. 9 and FIG. 11 illustrates an example of providing two seventh liquid storing units 37. As described above, one may provide multiple liquid storing units for storing the same reagent. This makes it possible to easily secure the required amount of liquid. The reagent 90 to disperse the detection matter MD is, for example, phosphate buffered saline (PBS).

In step S20, after the nucleic acid at least contained in the dispersoid is amplified, the cartridge 100 is rotated about the rotational shaft 221 to transfer the reagent 90 to disperse the detection matter MD is transferred from the seventh liquid storing unit 37 to the sixth chamber 16.

The sealing body 30a of the seventh liquid storing unit 37 is unsealed, for example, at the same timing as that of unsealing the sealing bodies 30a of the second liquid storing unit 32 to the sixth liquid storing unit 36. Thus, the operation of rotating the cartridge 100 for liquid feeding may be performed only once.

In the case of detection using a flow cytometer, the operator collects the sample by, for example, puncturing the sixth chamber 16 with a pipette (not illustrated) or unsealing a sealing body for sample collection (not illustrated). If the sixth chamber 16 is filled with a predetermined amount of PBS, it is possible to secure the amount of sample liquid necessary to detect the magnetic particles PM containing labeled nucleic acids. The labeling substance LS in the collected sample is detected with a flow cytometer.

In the case of detection with a camera unit, an image of the sixth chamber 16 of the cartridge 100 is captured, and magnetic particles are counted by image processing, for example. If the sixth chamber 16 is filled with a predetermined amount of PBS, the magnetic particles PM containing the labeled nucleic acids are in a state of being sufficiently dispersed in the sixth chamber 16.

With the above configuration, the specimen processing method of a first embodiment uses one cartridge 100 to perform the processing illustrated in steps S13 to S19 in FIG. 10 or the processing illustrated in steps S13 to S20.

### (Specific Configuration Example of Specimen Processing Apparatus)

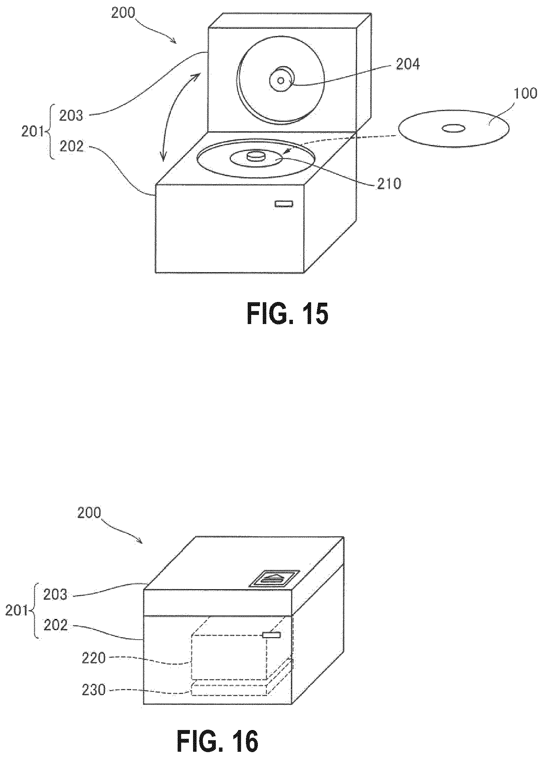

Next, a specific configuration example of the specimen processing apparatus 200 which performs the above-described specimen processing method using the cartridge 100 is described with reference to FIG. 15 to FIG. 19. Specifically, the specimen processing apparatus 200 is capable of performing, on the nucleic acids (DNAs) being the detection matter MD, the processes of emulsion formation (step S13), emulsion PCR (step S14), emulsion break (step S15), primary washing (step S16), denaturing (step S17), hybridization (step S18), and secondary washing (step S19). The specimen processing apparatus 200 may be able to execute the detection processing (step S20) as described later.

As illustrated in FIGS. 15 and 16, the housing 201 of the specimen processing apparatus 200 includes a main part 202 and a lid part 203. The lid part 203 is provided to cover substantially the entire surface of the upper surface portion of the main part 202. The upper surface portion of the main part 202 is provided with the setting part 210 on which the cartridge 100 is placed. The lid part 203 is provided to be openable and closable by pivoting up and down relative to the main part 202 to a state in which the setting part 210 illustrated in FIG. 15 is opened and to a state in which the setting part 210 illustrated in FIG. 16 is covered. The main part 202 includes the rotation mechanism 220 and the controller 230.

### <Internal Structure of Specimen Processing Apparatus>

As illustrated in FIG. 17, the setting part 210 is structured as a support member which supports the cartridge 100 from below. The setting part 210 is provided on the upper end portion of the rotational shaft 221 of the rotation mechanism 220. The setting part 210 is, for example, a turntable, and is fitted in the pore 51 of the cartridge 100 (see FIG. 9) to align the center of the cartridge 100 with the rotational shaft 221. The lid part 203 is provided with a clamper 204. With the lid part 203 closed, the clamper 204 rotatably supports the central portion of the upper surface of the cartridge 100 set on the setting part 210.

In the example of FIG. 17, the specimen processing apparatus 200 includes the rotation mechanism 220, a transfer mechanism 240, and the unsealing mechanism 250.

The rotation mechanism 220 includes the rotational shaft 221 and the drive unit 222 including an electric motor. The rotation mechanism 220 drives the drive unit 222 to rotate the cartridge 100, set on the setting part 210, about the rotational shaft 221. The rotation mechanism 220 includes an encoder 223 to detect the angle of rotation of the drive unit 222, and an origin sensor 224 to detect the position of the origin of the angle of rotation. When the drive unit 222 is driven based on the angle detected by the encoder 223 with reference to the position detected by the origin sensor 224, it is possible to move the cartridge 100 to any rotational position.

The rotation mechanism 220 is structured to perform at least part of the specimen processing by rotating the cartridge 100 about the rotational shaft 221. Specifically, the rotation mechanism 220 performs, by rotation, processing such as emulsion formation, specimen transfer, transfer of reagents to the chambers 11 to 16 (see FIG. 11), agitation of reagents and specimens, and transfer of magnetic particles PM in the circumferential direction to the chambers 11 to 16 inside the cartridge 100.

The transfer mechanism 240 includes a magnet 241, and has a function of moving the magnetic particles PM inside the cartridge 100 in the radial direction with the magnet moving mechanism 242. The transfer mechanism 240 is placed below the setting part 210. The magnet moving mechanism 242 is a combination of linear motion mechanisms, and is structured to move the magnet 241 in the radial direction. In addition, the magnet moving mechanism 242 is structured to move the magnet 241 in a direction towards or away from the cartridge 100. When the magnet 241 is brought close, the magnetic particles PM in the cartridge 100 are gathered, and when the magnet 241 is separated, the gathering of the magnetic particles PM is released. The transfer mechanism 240 can move the magnetic particles PM in the cartridge 100 into and out of the chambers (see FIG. 11) by moving the magnet 241 in the radial direction in the state of magnetic gathering. Thus, the transfer mechanism 240 transfers the magnetic particles PM to the second chamber 12 connected to the chamber 11. Similarly, the transfer mechanism 240 transfers the magnetic particles PM to other chambers.

The unsealing mechanism 250 includes a pin member 251 which can proceed and recede toward the cartridge 100 from above the cartridge 100 placed on the setting part 210. The unsealing mechanism 250 causes the pin member 251 to proceed and recede with a drive source such as a solenoid or a motor. When the sealing body 30a (see FIG. 9) is placed at a position immediately below the pin member 251 by the rotation mechanism 220, the unsealing mechanism 250 causes the pin member 251 to protrude for contact with the cartridge 100, and to unseal the sealing body 30a in the cartridge 100 by pressing. After opening, the unsealing mechanism 250 moves the pin member 251 away from the cartridge 100 to the retracted position where there is no contact.

In addition, the specimen processing apparatus 200 includes a temperature adjusting unit 260 that changes a temperature of the cartridge 100 to cause a reaction between the detection matter MD and the reagent 81 contained in the dispersoid in the emulsion 83. This makes it possible to reliably cause a reaction between the detection matter MD and the reagent 81 by temperature change in the state in which the detection matter MD is stored for compartmentalization in the droplets 84 of the dispersoid (see FIG. 12A to FIG. 12D) together with the reagent 81 to process the detection matter MD. In addition, as compared with the case of causing the reaction between the detection matter MD and the reagent 81 to naturally proceed under the temperature of the setting environment of the cartridge 100, it is possible to efficiently cause a reaction between the detection matter MD and the reagent 81 using the temperature change even in the droplets 84 contained in the emulsion 83.

The temperature adjusting unit 260 is provided at a position immediately above the cartridge 100 placed on the setting part 210. The temperature adjusting unit 260 is placed on the inner surface of the lid part 203 so as to face the cartridge 100. As he planar shape of the temperature adjusting unit 260, it is possible to employ the configuration illustrated in FIG. 13 or FIG. 14. The temperature adjusting unit 260 is a heater or a Peltier element.

One or more temperature adjusting units 260 cyclically change the temperature of the cartridge 100 to multiple temperature ranges to amplify the nucleic acid contained in the dispersoid. Thus, it is possible to perform so-called thermal cycling to efficiently amplify nucleic acids in the droplets 84 contained in the emulsion 83.

The temperature adjusting unit 260 is structured to be movable between a contact position and a floating position relative to the cartridge 100 placed on the setting part 210. In the example of FIG. 17, the temperature adjusting unit 260 is caused by a moving mechanism 261 to move to a position in contact with the surface of the cartridge 100 and a position away from the surface of the cartridge 100. The moving mechanism 261 includes a drive source such as a solenoid or a motor, for example. The temperature adjusting unit 260 changes the temperature of the cartridge 100 while in contact with the cartridge 100. Thus, as compared with the case of heating the cartridge 100 in a non-contact manner, heat can be efficiently conducted by bringing the temperature adjusting unit 260 into contact with the cartridge 100. Therefore, the temperature of the cartridge 100 can be easily changed.

Note that the configuration may be such that the temperature adjusting unit 260 is fixedly provided and that the setting part 210 moves the cartridge 100 towards or away from the temperature adjusting unit 260. Note that the specimen processing apparatus 200 includes a temperature sensor 262. The temperature sensor 262 detects the temperature of the cartridge 100 by, for example, infrared light. The temperature adjusting unit 260 is controlled based on the detection results of the temperature sensor 262.

The specimen processing apparatus 200 may be provided with a detection unit 270 to detect a signal based on the labeling substance LS. The detection unit 270 may be, for example, a flow cytometer, a camera unit (image capturing unit), or the like as described above. FIG. 17 illustrates an example in which the detection unit 270 is an image capturing unit. The detection unit 270 is placed at a position facing the cartridge 100 placed on the setting part 210 via an opening formed in the upper surface of the main part 202. Thus, the detection unit 270 captures an image of the inside of the sixth chamber 16 (see FIG. 11). Image processing of the captured image makes it possible to identify the individual magnetic particles PM in the sixth chamber 16 and to obtain a fluorescence color attributed to the labeling substance LS bound to the magnetic particles PM.

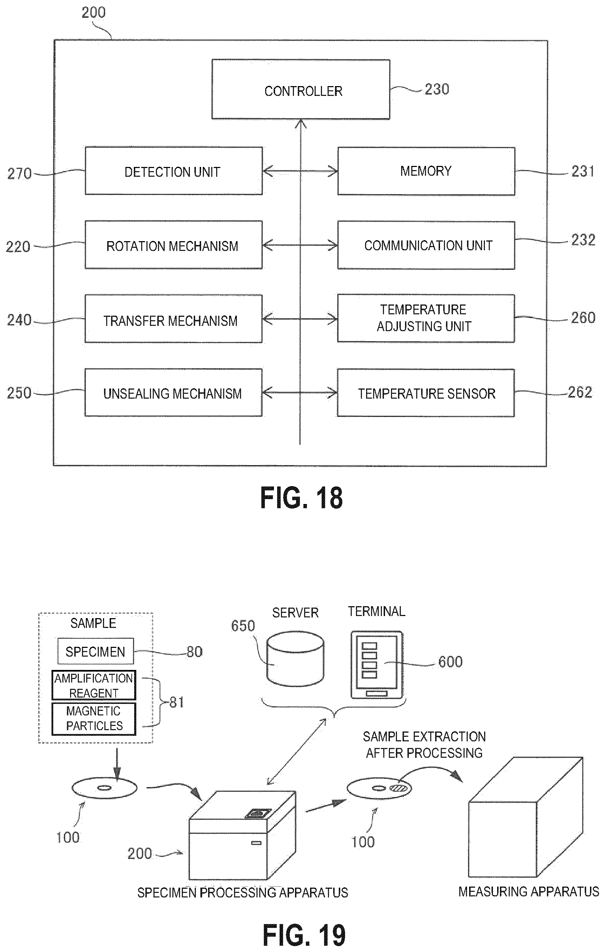

The specimen processing apparatus 200 does not have to include the detection unit 270. In that case, as illustrated in FIG. 19, the processed cartridge 100 is taken out of the sample specimen processing apparatus 200, and detection processing is performed using another measuring apparatus.

FIG. 18 illustrates a control configuration of the specimen processing apparatus 200.

The specimen processing apparatus 200 includes a controller 230. The controller 230 includes, for example, a processor and a memory. The processor is, for example, a CPU, an MPU, an FPGA, or the like. The memory is, for example, a ROM and a RAM. The controller 230 receives signals from the units of the specimen processing apparatus 200 and controls the units of the specimen processing apparatus 200.

The specimen processing apparatus 200 includes a memory 231. The memory 231 stores e.g. a program for causing the processor to function as the controller 230 of the specimen processing apparatus 200. The memory 231 is, for example, a flash memory, a hard disk, or the like.

The specimen processing apparatus 200 includes a communication unit 232. The communication unit 232 is capable of transmitting information to an external device and receiving information from an external device. The communication unit 232 includes, for example, a communication module, an interface for external connection, and the like. By wired or wireless communication, the communication unit 232 is capable of communication with a terminal 600 (see FIG. 19) capable of communicating with the specimen processing apparatus 200, and communication with a server 650 (see FIG. 19) via a network. The specimen processing apparatus 200 is structured to be able to display various types of operations and various types of information by using the external terminal 600. The terminal 600 includes, for example, a tablet type terminal, a portable information terminal such as a smartphone, an information terminal such as a personal computer (PC), and the like.

The controller 230 controls the units of the specimen processing apparatus 200 to execute the specimen processing method of a first embodiment.

### <Operation of Specimen Processing Apparatus>

Next, with reference to FIG. 9 to FIG. 17, the processing operation of the specimen processing apparatus 200 is described. The processing operation of the specimen processing apparatus 200 is controlled by the controller 230. Note that, as a preparatory operation, the operator injects into the cartridge 100 the dispersoid containing the specimen 80 and the reagent 81 after step S11 and step S12 of FIG. 10 are performed. Then, the cartridge 100 is set on the setting part 210 of the specimen processing apparatus 200 (see FIG. 15), and the lid part 203 is closed (see FIG. 16). In this state, the processing operation is started.

In step S13, the controller 230 controls the rotation mechanism 220 such that the dispersoid stored in the specimen storing unit 38 included in the cartridge 100 is transferred by rotation to the chamber 11. In addition, the controller 230 controls the unsealing mechanism 250 such that the sealing body 30a of the first liquid storing unit 31 is unsealed. After unsealing, the controller 230 controls the rotation mechanism 220 such that the dispersion medium 82 stored in the first liquid storing unit 31 included in the cartridge 100 is transferred by rotation to the chamber 11. The dispersoid and dispersion medium 82 are transferred into the chamber 11 by the centrifugal force.

Thus, mere rotation of the cartridge 100 by the rotation mechanism 220 enables not only producing the emulsion 83 but also an operation of storing the dispersion medium 82 in the chamber 11. Therefore, since it is not necessary to apply air pressure to, for example, the first liquid storing unit 31, the specimen can be more easily processed. In addition, it is unnecessary to provide e.g. a mechanism for applying air pressure in order to feed the dispersion medium 82 from the liquid storing unit 30 to the chamber 11. Therefore, the apparatus configuration can be further simplified.

The controller 230 forms in the chamber 11 the emulsion 83 in which the dispersoid containing the specimen 80 and the reagent 81 is dispersed in the dispersion medium 82 by controlling the rotation mechanism 220 such that the rotation mechanism 220 repeats an operation of changing a rotational speed of the cartridge 100.

For example, the controller 230 controls, when forming the emulsion 83, the rotation mechanism 220 such that the rotation mechanism 220 repeats an operation of reversing the rotational direction of the cartridge 100 in a cycle of 165 milliseconds or more and 330 milliseconds or less. Thus, only by repeating the operation of reversing the rotational direction in a cycle of 165 milliseconds or more and 330 milliseconds or less based on the results of the experiments to be described later, the emulsion 83 suitable for processing and detecting the detection matter MD can be prepared.

The controller 230 causes the cartridge 100 to rotate to form in the chamber 11 the emulsion 83 containing droplets 84 of the dispersoid each storing one molecule or one particle of the detection matter MD. Thus, individual molecules of the detection matter MD contained in the specimen can be subjected to compartmentalization into the respective droplets 84 contained in the emulsion 83, and can be reliably processed. As a result, even the detection matter MD contained in an extremely small amount in the specimen can be processed with high accuracy. Therefore, it is possible to improve the detection accuracy also in the detection of the processed detection matter MD.

In step S14, the controller 230 controls the temperature adjusting unit 260 such that the temperature of the cartridge 100 is changed in a range of 30°C or more and 90°C or less. Thereby, emulsion PCR processing is performed. For example, in the configuration example of FIG. 13, the controller 230 performs control such that the three temperature adjusting units 260 form the respective different temperature zones TZ1 to TZ3. Then, the rotation mechanism 220 is controlled such that the chamber 11 sequentially moves to the temperature zones TZ1 to TZ3. In each temperature zone TZ, the controller 230 controls the moving mechanism 261 such that the temperature adjusting unit 260 is brought into contact with the cartridge 100, and controls the inside of the chamber 11 at a desired temperature.

The controller 230 controls the unsealing mechanism 250 such that the sealing body 30a of the second liquid storing unit 32 is unsealed after the emulsion 83 is formed in the chamber 11 and the temperature of the cartridge 100 is changed to cause a reaction between the detection matter MD and the reagent 81. Thus, the sealing body 30a makes it possible to prevent unintended feeding of the reagent 85 to demulsify the emulsion from the second liquid storing unit 32. Therefore, if the unsealing mechanism 250 which unseals the sealing body 30a is provided, the emulsion 83 can be demulsified after reliably forming the emulsion 83 and amplifying nucleic acids. Similarly, the controller 230 controls the unsealing mechanism 250 such that the sealing bodies 30a of the third liquid storing unit 33 to the seventh liquid storing unit 37 are unsealed.

In step S15, the controller 230 controls the rotation mechanism 220 such that a reagent 85 to demulsify the emulsion is transferred by rotation from the second liquid storing unit 32 included in the cartridge 100 to the chamber 11 in which the emulsion 83 is formed. The reagent 85 to demulsify the emulsion demulsifies the emulsion 83 in the chamber 11. Thus, the specimen processing apparatus 200 enables not only the processing of forming the emulsion 83 but also the processing of demulsifying the emulsion 83 in the cartridge 100. Also in this case, it is unnecessary to provide e.g. a mechanism for applying air pressure in order to feed the reagent 85 to demulsify the emulsion to the chamber 11. Therefore, the apparatus configuration can be further simplified.

Here, if the sealing bodies 30a of the third liquid storing unit 33 to the seventh liquid storing unit 37 are also unsealed, each of the reagents stored in the liquid storing units 30 can also be fed to the corresponding chamber in one feeding operation.

As described above, the controller 230 controls the unsealing mechanism 250 such that the sealing body 30a of the third liquid storing unit 33 storing the reagent 86 to wash the detection matter MD is unsealed at least after the temperature adjusting unit 260 causes a reaction between the detection matter MD and the reagent 81. Thus, the reagent 86 to wash the detection matter MD is fed to the second chamber 12 after amplification due to the temperature change caused by use of the temperature adjusting unit 260. Therefore, it is possible to suppress the influence of the temperature change on the reagent 86 to wash the detection matter MD.

In step S16, after the emulsion 83 is demulsified, the controller 230 controls the transfer mechanism 240 such that the detection matter MD is transferred from the chamber 11 to the second chamber 12. Specifically, the controller 230 moves the magnetic particles PM in the radial direction by the magnetic force of the transfer mechanism 240, and moves the cartridge 100 in the circumferential direction relative to the magnetic particles PM by the rotation of the rotation mechanism 220. The combination of the magnetic force and the rotation of the cartridge 100 moves the magnetic particles PM bound to the detection matter MD to the second chamber 12 via the passage 40. The controller 230 washes the magnetic particles PM with the reagent 86 to wash the detection matter MD. Thus, nucleic acids can be washed in the second chamber 12 after amplifying the nucleic acids being the detection matter MD in the chamber 11 and taking out the nucleic acids from the droplets 84 by demulsification.

Note that the controller 230 unseals the sealing body 30a of the liquid storing unit 30 storing the reagent 87 to denature the nucleic acid after the temperature adjusting unit 260 causes a reaction between the detection matter MD and the reagent 81 (after step S14). Thus, the reagent 87 to denature the nucleic acid is fed to the third chamber 13 after amplification due to the temperature change caused by use of the temperature adjusting unit 260. Therefore, it is possible to suppress the influence of the temperature change on the reagent 87 to denature the nucleic acid.

In step S17, the controller 230 controls the transfer mechanism 240 such that the detection matter MD stored in the second chamber 12 is transferred to the third chamber 13. In the third chamber 13, the reagent 87 to denature the nucleic acid is transferred by rotation from the fourth liquid storing unit 34. As a result, in the third chamber 13, the nucleic acids (double-stranded DNAs) bound to the magnetic particles PM are denatured into single strands. Thus, the nucleic acids after being subjected to the processing of washing the nucleic acids in the second chamber 12 can be further subjected to processing of denaturing the nucleic acids in the cartridge 100. Additionally, also in the case of performing the processing of denaturing the nucleic acids, mere rotation of the cartridge 100 about the rotational shaft 221 makes it possible to feed liquid to the third chamber 13 instead of liquid feeding using air pressure or the like. Therefore, it is possible to perform the processing of denaturing the nucleic acids with a simple apparatus configuration.

Note that the controller 230 unseals the sealing body 30a of the liquid storing unit 30 storing the reagent 87 to denature the nucleic acid after the temperature adjusting unit 260 causes a reaction between the detection matter MD and the reagent 81 (after step S14). Thus, the reagent 87 to denature the nucleic acid is fed to the third chamber 13 after amplification due to the temperature change caused by use of the temperature adjusting unit 260. Therefore, it is possible to suppress the influence of the temperature change on the reagent 87 to denature the nucleic acid.

In step S18, the controller 230 controls the transfer mechanism 240 such that the detection matter MD stored in the third chamber 13 is transferred to the fourth chamber 14. In the fourth chamber 14, the reagent 88 containing the labeling substance LS that reacts with the amplification products of the nucleic acids is transferred by rotation from the fifth liquid storing unit 35. This makes it possible to perform, in the cartridge 100, not only the processing of denaturing the nucleic acids but also the processing of further labeling the nucleic acids being the detection matter MD for the purpose of detection. Additionally, also in the case of performing the processing of labeling the nucleic acids, mere rotation of the cartridge 100 by the rotation mechanism 220 makes it possible to feed liquid to the fourth chamber 14 instead of liquid feeding using air pressure or the like. Therefore, it is possible to perform the processing of labeling the nucleic acids with a simple apparatus configuration.

The controller 230 controls the temperature adjusting unit 260, thereby raising the temperature of the cartridge 100 to cause a reaction between the nucleic acid and the labeling substance LS in the fourth chamber 14. As a result, the controller 230 performs labeling processing on the amplification products of the nucleic acids in the fourth chamber 14. Thus, both the processing of amplifying the nucleic acids and the processing of labeling the nucleic acids can be performed in the cartridge 100 by the common temperature adjusting unit 260. Therefore, not only the preparation of the emulsion 83 but also the processing of amplifying the nucleic acids and the processing of labeling the nucleic acids using the cartridge 100 can be more simply performed with a simple apparatus configuration.

In step S19, the controller 230 controls the transfer mechanism 240 such that the detection matter MD stored in the fourth chamber 14 is transferred to the fifth chamber 15. In the fifth chamber 15, the reagent 89 to wash the detection matter MD is transferred by rotation from the sixth liquid storing unit 36. As a result, the controller 230 washes the labeling processed magnetic particles PM in the fifth chamber 15.

In step S20, the controller 230 controls the transfer mechanism 240 such that the detection matter MD stored in the fifth chamber 15 is transferred t to the sixth chamber 16. In the sixth chamber 16, the reagent 90 to disperse the detection matter MD (PBS) is transferred by rotation from the seventh liquid storing unit 37. As a result, the controller 230 disperses in the reagent the labeling processed magnetic particles PM in the sixth chamber 16.

The controller 230 controls the rotation mechanism 220 to place the sixth chamber 16 within the image capturing field of the detection unit 270. The controller 230 causes the detection unit 270 to acquire an image of the inside of the sixth chamber 16. The controller 230 counts the number of individual magnetic particles PM based on the acquired image, and acquires the fluorescence color and the fluorescence intensity of the magnetic particles PM. The controller 230 creates a scattergram between normal DNA and mutant DNA from, for example, the acquired fluorescence color and fluorescence intensity of the magnetic particles PM.

If the specimen processing apparatus 200 does not include the detection unit 270, the controller 230 terminates the processing without executing step S20. In this case, as illustrated in FIG. 19, the operator takes out the processed cartridge 100 from the specimen processing apparatus 200 and performs detection using another measuring apparatus.

### [Examples]

Next, description is provided for experiments (examples) conducted by the inventors of the present application on emulsion preparation according to a first embodiment. The inventors of the present application conduct experiments under various conditions of the cycle of changing the rotational speed of the cartridge 100, the amount of liquid (dispersoid and dispersion medium 82) stored in the chamber 11, the rotational direction, and the rotational speed. Then, the obtained experimental results and the comparative example are compared. Each of the experiments is described below.

As the specimen, a nucleic acid solution prepared by mixing 1% of mutant type DNA in wild type DNA is used in common in examples and a comparative example.

### (Comparative Example)

In the comparative example, the inventors of the present application measure using a flow cytometer a specimen subjected to the processing of steps S13 to S19 in FIG. 10 by a manual method. An emulsion of a dispersion medium and a dispersoid containing a specimen (nucleic acid solution) and a nucleic acid amplification reagent is prepared, and PCR is performed. A labeling substance which specifically binds to the wild type and the mutant type is hybridized to the DNAs taken out by the emulsion break processing, and then detected with a flow cytometer.

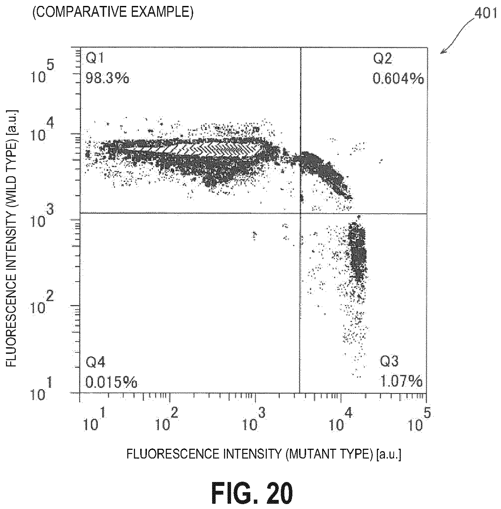

FIG. 20 illustrates a scattergram 401 according to the comparative example. In the scattergram, the vertical axis indicates fluorescence intensity for wild type DNA, and the horizontal axis indicates fluorescence intensity for mutant type DNA. The plots included in the area Q1, segmented by two straight lines parallel to the vertical axis and the horizontal axis, indicate wild type DNA, and the plots included in the area Q3 indicate mutant type DNA. The wild type DNA is 98.3% and the mutant type DNA is 1.07% with respect to the total number of plots included in the areas Q1 to Q4.

### (Example 1: Change cycle of Rotational Speed)

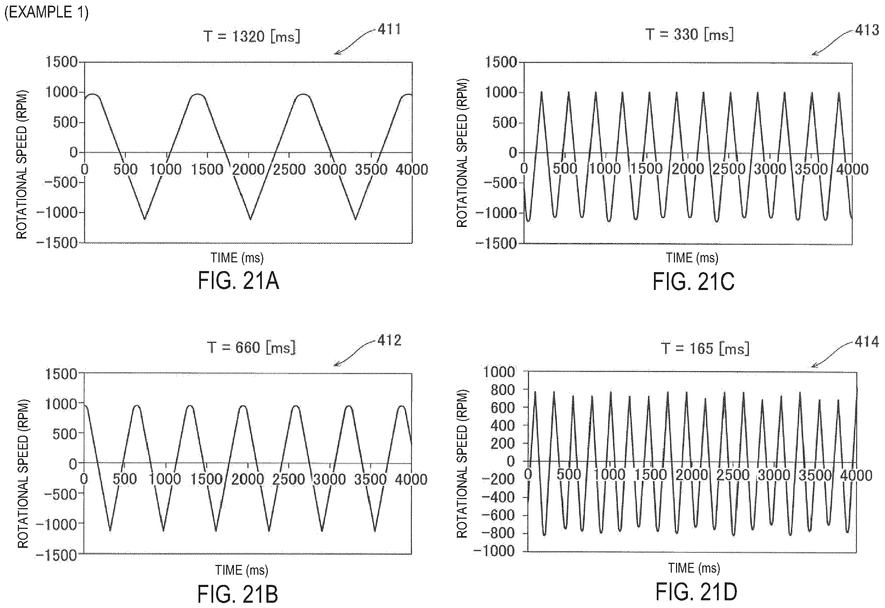

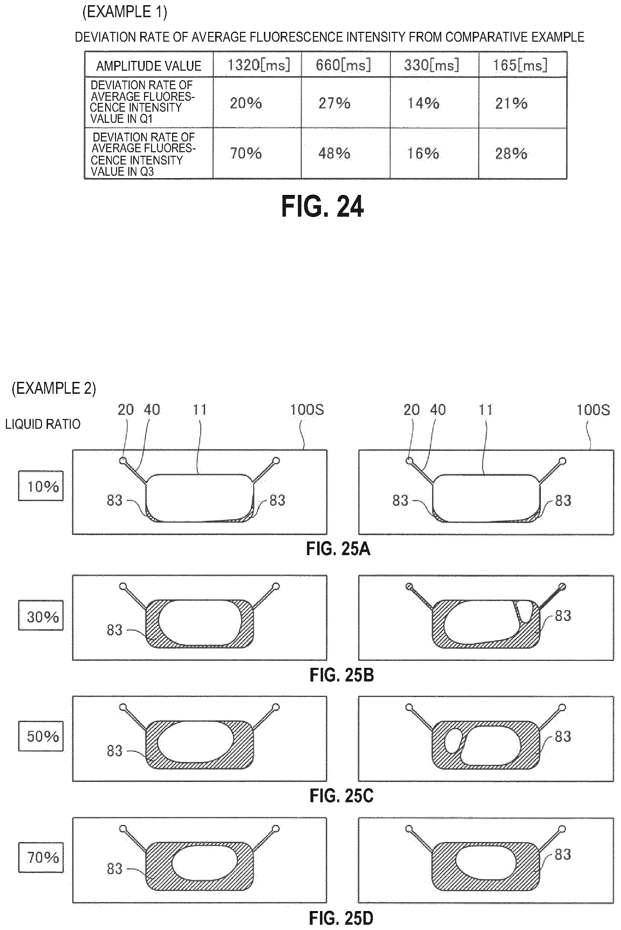

A test cartridge 100S illustrated in FIG. 25A to FIG. 25D is prepared, and emulsions are prepared under four conditions with different reversing cycles under the condition of repeating the operation of reversing the rotational direction of the cartridge 100S. The change cycle T of the rotational speed is set to four conditions of T = 1320 milliseconds, 660 milliseconds, 330 milliseconds, and 165 milliseconds. FIG. 21A to FIG. 21D illustrate change graphs 411 to 414 of rotational speeds for the different cycles T.

The volume of the chamber 11 used is 284.62 µL. In the chamber 11, a dispersion medium and a mixed liquid of a specimen (nucleic acid solution) and a nucleic acid amplification reagent are injected in an amount of 90 µL. The ratio of the total volume of the dispersoid and the dispersion medium 82 to the chamber 11 is about 30%. The operation of changing the rotational speed of the cartridge 100S at each change cycle T is repeated for one minute.

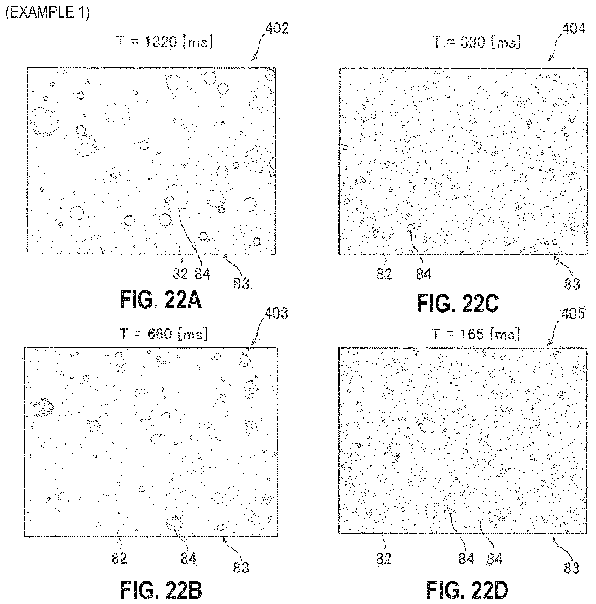

FIG. 22A to FIG. 22D illustrate microscope images 402 to 405 of the emulsions 83 prepared in various cycles T of Example 1. It is found from the microscopic images that, for all cycles T, the emulsions 83 in which the droplets 84 of the dispersoid are dispersed in the dispersion medium 82 are formed. In addition, as can be seen from the images, it is found that the smaller the cycle T, the smaller the droplet size (diameter in the images).

For each cycle T, 25 microscope images 402 to 405 are acquired, and the droplets 84 included in the 25 microscope images are counted by image processing. The total number of droplets for each cycle T is as follows.

| | |
|---|---|
| T = 1320 milliseconds | Number of droplets: 410 |
| T = 660 milliseconds | Number of droplets: 766 |
| T = 330 milliseconds | Number of droplets: 2440 |
| T = 165 milliseconds | Number of droplets: 2726 |

In Example 1, it is found from the above results that, first, the emulsion 83 can be formed even when the cycle T is changed. Then, it is found that changing the cycle T makes it possible to control the size and the number of droplets 84 contained in the emulsion 83. Here, for the purpose of forming the emulsion 83 containing the droplets 84 of dispersoid each storing one molecule or one particle of the target DNA, the number of droplets 84 sufficient for the majority of the droplets 84 to contain 0 or 1 DNA is statistically determined based on the number of DNAs contained in the specimen 80. Specifically, if one can prepare an emulsion 83 containing droplets 84 in a number statistically determined based on the number of DNAs contained in the specimen 80, it is possible to perform digital processing of the target DNA limitingly diluted into each droplet 84 (that is, PCR processing on each single DNA contained in the specimen). Example 1 suggests that, by appropriately setting the change cycle T of the rotational speed of the cartridge 100S, it is possible to perform digital processing of the target DNA according to the concentration of DNA contained in the specimen.

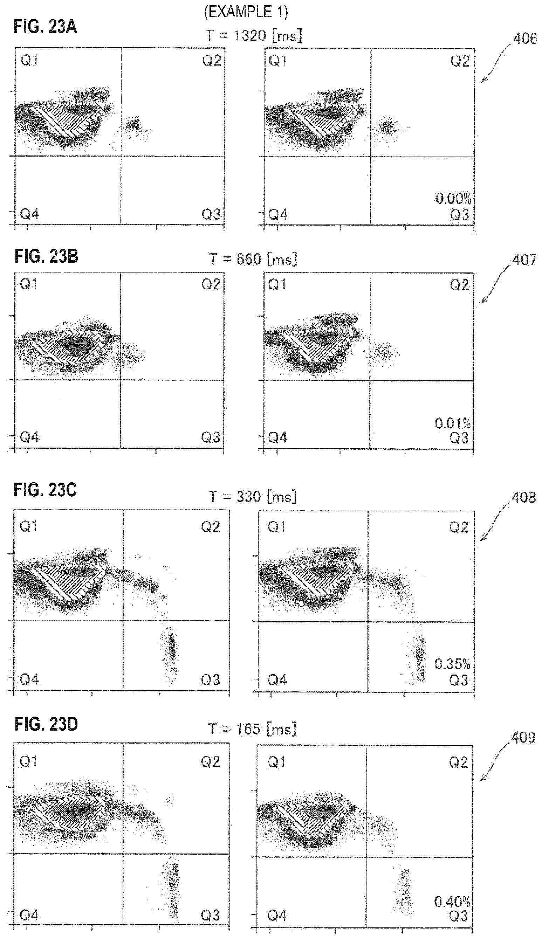

Next, the emulsions 83 prepared at various cycles T are subjected to PCR processing, and a labeling substance which specifically binds to the wild type and the mutant type is hybridized to the DNAs taken out by the emulsion break processing, and then detected with a flow cytometer. Then, scattergrams similar to that of the comparative example (see FIG. 20) are created. FIG. 23A to FIG. 23D illustrate scattergrams 406 to 409 processed under the conditions of various cycles T. The experiment is conducted several times, and the two scattergrams are illustrated for each of 406 to 409.

Assuming that the number of mutant DNAs detected in the comparative example is Mr and the number of mutant DNAs detected in Example 1 is Mt, the deviation rate from the comparative example is determined as (Mr - Mt)/Mr. The deviation rates are as follows. The deviation rate of the number of mutant type DNAs detected is focused on. At the DNA concentration of the specimen used in the experiment, it is found that the condition of cycle T = 330 milliseconds (deviation rate: 16%) is most preferable, and the condition of cycle T = 165 milliseconds (deviation rate: 28%) is second most preferable. As a result, it is found that it is possible to prepare an emulsion more suitable for DNA detection by appropriately setting the cycle T for changing the rotational speed of the cartridge 100S based on the DNA concentration of the specimen. Particularly in a cycle of 165 milliseconds or more and 330 milliseconds or less, the number of droplets 84 formed increases, and it is possible to increase the DNA concentration of the specimen accordingly. The results are preferable because more DNAs can be efficiently processed and detected in one process.

### (Example 2)

Emulsions are prepared under four conditions with different ratios Rv of the total volume of the dispersoid and the dispersion medium 82 to the chamber 11 of the cartridge 100S. The volume of the chamber 11 is 284.62 µL. The total volume ratios Rv are set to four conditions of 10%, 30%, 50%, and 70%. Under any of the conditions, the volume ratio of the dispersion medium and the dispersoid containing the specimen (nucleic acid solution) and the nucleic acid amplification reagent is maintained at dispersoid:dispersion medium = 2:7, and the liquid amount is made different. For emulsion preparation, the change cycle T of the rotational speed is set to 330 milliseconds under the condition of repeating the operation of reversing the rotational direction of the cartridge 100S. The operation of changing the rotational speed of the cartridge 100S at cycle T is repeated for one minute.

FIG. 25A to FIG. 25D illustrate schematic diagrams of the situation inside the chamber 11 after emulsion preparation. The experiment is conducted several times, and two schematic diagrams under each condition are illustrated. The unhatched portion in the chamber 11 is a region occupied by air. It is found that the emulsion 83 indicated with hatching is formed at any total volume ratio Rv.

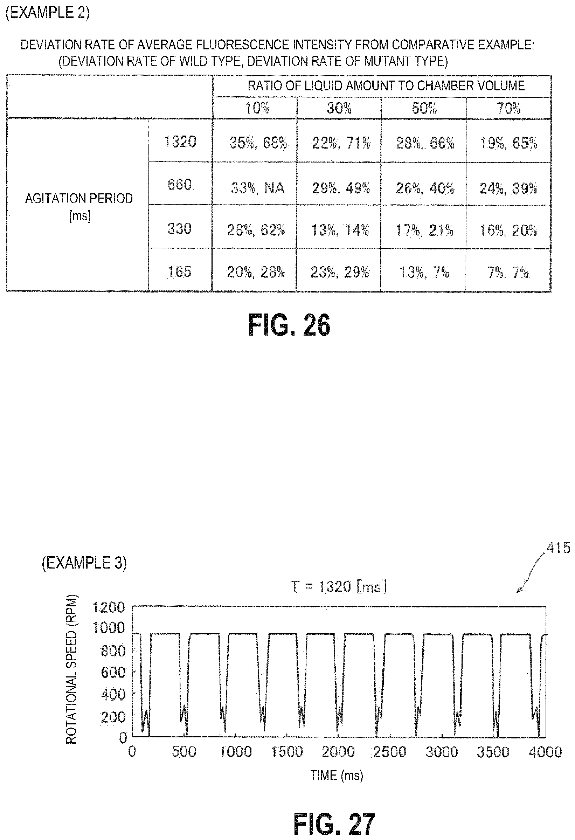

Next, scattergrams of the detected DNA are prepared by performing the same processing as that in Example 1 above in the case where the total volume ratio Rv is set to four conditions of 10%, 30%, 50% and 70% and where, at each ratio Rv, the emulsion 83 is prepared under four conditions of T = 1320 milliseconds, 660 milliseconds, 330 milliseconds, and 165 milliseconds as the change cycle T of the rotational speed. Then, in each of the obtained scattergrams, the deviation rate of the number of detected wild type DNAs as compared with the comparative example and the deviation rate of the number of detected mutant DNAs as compared with the comparative example are calculated. FIG. 26 illustrates the calculation results of the deviation rate.

It is found from the results of FIG. 26 that an appropriate combination of the ratio Rv and the cycle T makes it possible to obtain a detection accuracy equivalent to that of the comparative example. Specifically, with the total volume ratio Rv = 30% and the change cycle T of the rotational speed = 330 milliseconds, the deviation rate is about 13% in all cases, and the results obtained are equivalent to those of the manual method. In addition, with the total volume ratio Rv = 50% or 70% and the change cycle T of the rotational speed = 165 milliseconds, the deviation rate is about 7%, and the results obtained are equivalent to those of the manual method. Note that the variation in results due to the operations in the manual method is about 10% to 20%, and the results under the above preferable conditions may be considered equivalent to the results by the manual method.

As a result of Example 1 and Example 2 above, in the specimen processing method of a first embodiment, preferably, the operation of changing the rotational speed of the cartridge 100S includes an operation of reversing a rotational direction of the cartridge 100S in a cycle of 165 milliseconds or more and 330 milliseconds or less. As a result, only by repeating the operation of reversing the rotational direction in a cycle of 165 milliseconds or more and 330 milliseconds or less, the emulsion 83 suitable for processing and detecting the detection matter MD can be prepared.

In addition, in the specimen processing method of a first embodiment, a ratio of a total volume of the dispersoid and the dispersion medium 82 to the chamber 11 is 30% or more and 70% or less. This makes it possible to prepare the emulsion 83 suitable for processing and detecting the detection matter MD.

In addition, in the specimen processing method of a first embodiment, more preferably, the operation of changing the rotational speed of the cartridge 100S includes an operation of reversing a rotational direction of the cartridge 100S in a cycle of 330 milliseconds, and a ratio of a total volume of the dispersoid and the dispersion medium 82 to the chamber 11 is 30%. This makes it possible to prepare the emulsion 83 even more suitable for processing and detecting the detection matter MD.

Similarly, in the specimen processing method of a first embodiment, preferably, the operation of changing the rotational speed of the cartridge 100S includes an operation of reversing a rotational direction of the cartridge 100S in a cycle of 165 milliseconds, and a ratio of a total volume of the dispersoid and the dispersion medium 82 to the chamber 11 is 50% or more and 70% or less. This makes it possible to prepare the emulsion 83 even more suitable for processing and detecting the detection matter MD.

### (Example 3)

Example 3 verifies whether or not it is possible to prepare the emulsion 83 and detect DNA in the case of repeating the operation of acceleration and deceleration in the same direction as the operation of changing the rotational speed of the cartridge 100S.

Specifically, as illustrated in FIG. 4B, an emulsion is formed under the condition of repeating the operation of accelerating and decelerating the cartridge 100S in the same direction. The change cycle T of the rotational speed is set to T = 380 milliseconds. FIG. 27 illustrates a change graph 415 of the rotational speed in Example 3. The other experimental conditions are the same as those in Example 1 above.

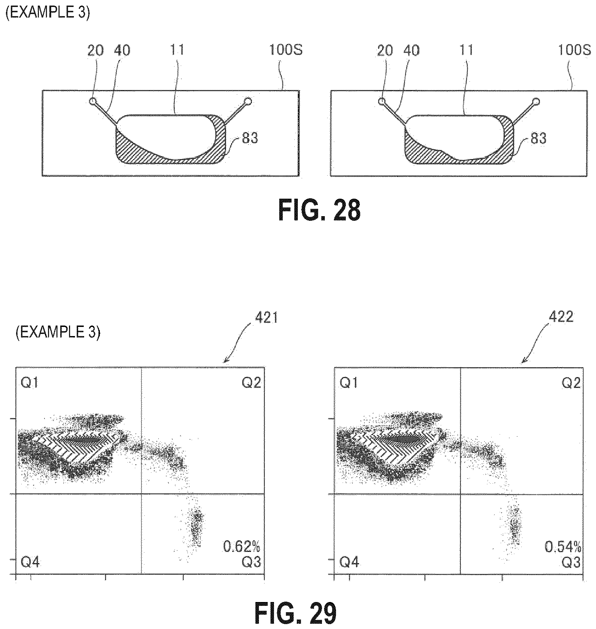

FIG. 28 provides schematic diagrams of the situation inside the chamber 11 after emulsion preparation. The experiment is conducted several times, and two schematic diagrams under each condition are illustrated. It is found from FIG. 28 that the emulsion 83 indicated with hatching is formed. It is found in Example 3 that it is possible to form the emulsion 83 even in the case of repeating the operation of accelerating and decelerating the cartridge 100S in the same direction.

Next, FIG. 29 provides scattergrams 421 and 422 processed under the conditions of Example 3. In the area Q3 indicating the mutant type DNA in each scattergram, the number of mutant DNAs detected is 0.62% (first time) and 0.54% (second time) with respect to the total number of plots included in the areas Q1 to Q4. Thus, it is found that nucleic acids can be detected using the emulsion 83 prepared under the condition of repeating the operation of accelerating and decelerating the cartridge 100S in the same direction.

### (Example 4: Rotational Speed)

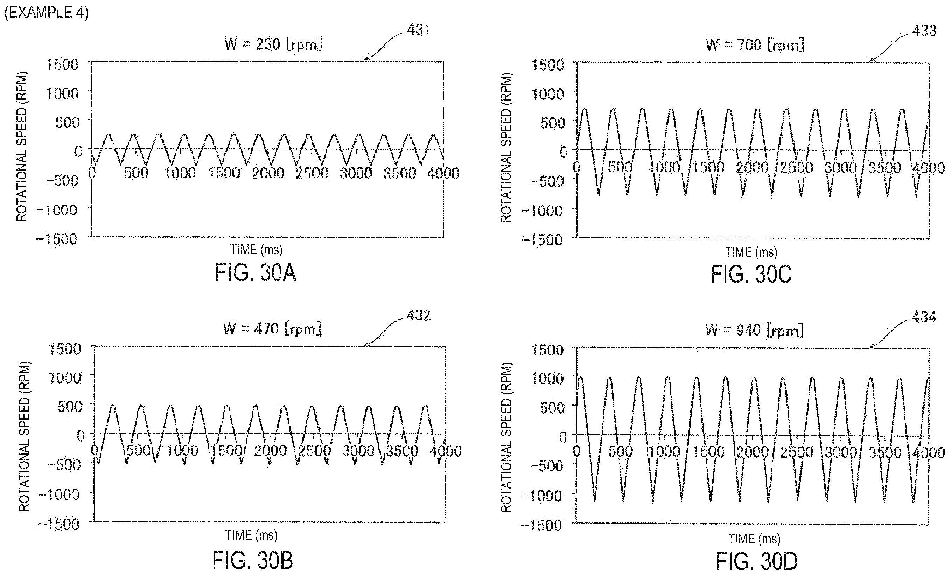

Emulsions are prepared under four conditions with different maximum values of the rotational speed under the condition of repeating the operation of reversing the rotational direction of the cartridge 100S. The rotational speed W is set to four conditions of W = 230 rpm, 470 rpm, 700 rpm, and 940 rpm. The change cycle T of the rotational speed is set to T = 330 milliseconds. FIG. 30A to FIG. 30D illustrate change graphs 431 to 434 of rotational speeds under various speed conditions. The ratio Rv of the total volume of the dispersoid and the dispersion medium 82 to the chamber 11 is 30%, and the operation of changing the rotational speed of the cartridge 100S under each speed condition is repeated for one minute. It is found that the emulsion is formed at any speed W.

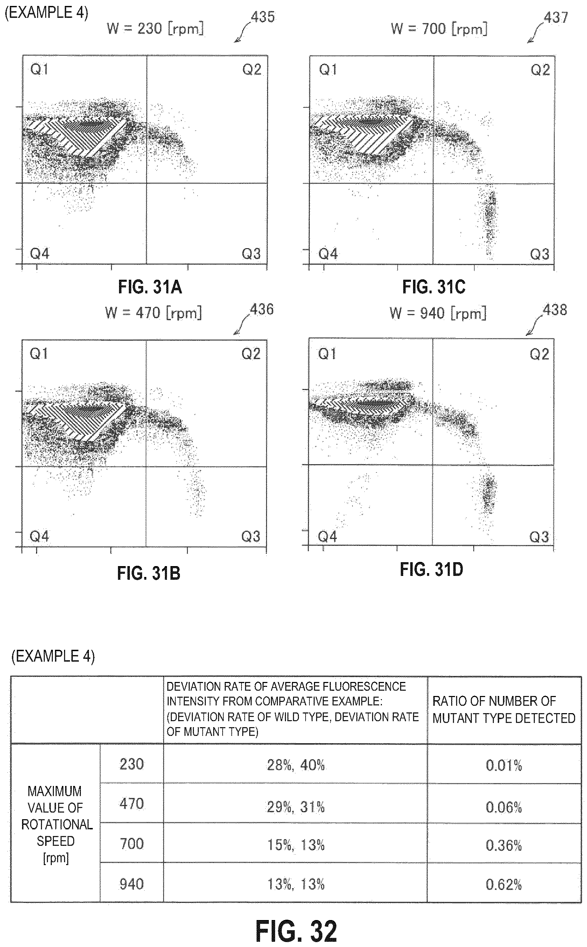

Next, the emulsions 83 prepared at various speeds W are subjected to PCR processing, and a labeling substance which specifically binds to the wild type and the mutant type is hybridized to the DNAs taken out by the emulsion break processing, and then detected with a flow cytometer. Then, as illustrated in FIG. 31A to FIG. 31D, scattergrams 435 to 438 are created. In addition, FIG. 32 illustrates ratios of the number of mutant type DNAs detected in the scattergrams 435 to 438 and deviation rates as compared with the comparative example.

It is found from the results of FIG. 32 that, at the DNA concentration of the specimen used in the experiment, the condition of speed W = 940 rpm is most preferable, and the condition of speed W = 700 rpm is second most preferable. It is found that it is possible to prepare an emulsion more suitable for DNA detection by appropriately setting the rotational speed W of the cartridge 100S based on the DNA concentration of the specimen.

### (Modified Example)

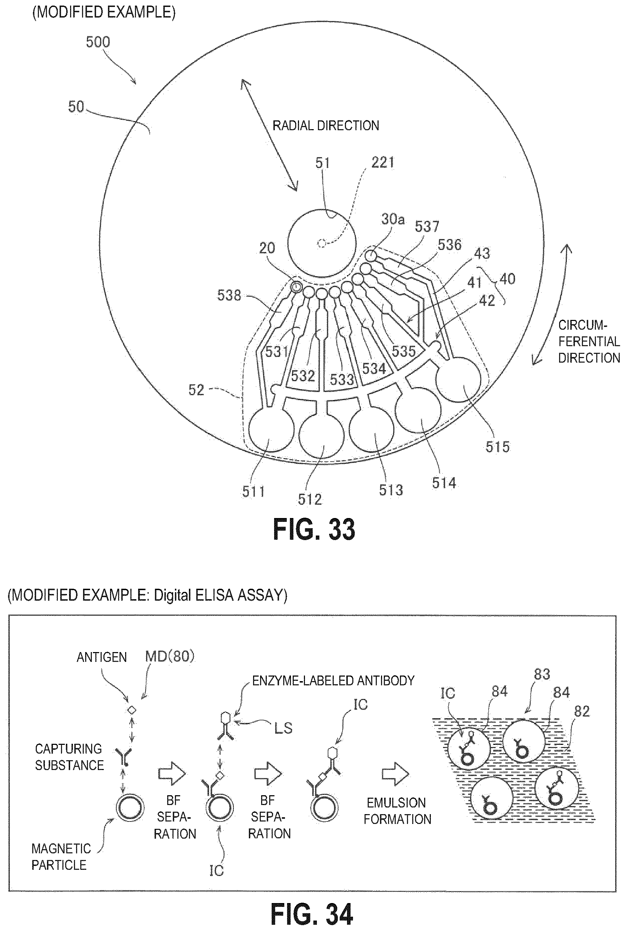

The configuration example described above presents an example in which the detection matter MD is a nucleic acid and the reagent 81 is a nucleic acid amplification reagent. Meanwhile, in the modified example of the specimen processing method illustrated in FIG. 33, the detection matter MD is a protein, and the reagent 81 contains a substrate that reacts with a labeling substance specifically bound to the protein in the specimen 80. The protein is specifically an antigen or an antibody. In this modified example, description is provided for a specimen processing method including using an antigen-antibody reaction to make the detection matter in the sample detectable, and performing an immunoassay which measures the detection matter based on the detection results.

### (Immunoassay <Digital ELISA>)

Hereinafter, a configuration example of a cartridge 500 used for digital enzyme-linked immunosorbent assay (ELISA) is presented.

FIG. 34 illustrates an overview of digital ELISA. ELISA is a method including forming an immune complex by causing magnetic particles to carry an antigen (which may be an antibody) being the target component and a labeling substance, and detecting the target component based on the label in the immune complex. Digital ELISA is a method including dispersing, in fine compartments, a sample which has been subjected to limiting dilution (dilution so that one or zero target component is in each fine compartment), directly counting the number of fine compartments for which signals based on the label are positive, and thereby absolutely measuring the concentration of the target component in the sample. In the case of FIG. 34, individual droplets 84 in the emulsion are fine compartments. The cartridge 500 of FIG. 33 is used to perform the assay illustrated in the example of FIG. 34.

### <Primary Reaction>

The cartridge 500 includes five chambers and eight liquid storing units. The specimen 80 containing an antigen being the detection matter MD is injected from the specimen introducing unit 20, and is stored in the specimen storing unit 538. In liquid storing unit 531 connected to the chamber 511, an R1 reagent containing a capturing substance to be bound to the detection matter MD is stored. The capturing substance includes, for example, a primary antibody to be bound to the detection matter being an antigen. The chamber 511 stores an R2 reagent containing magnetic particles PM.

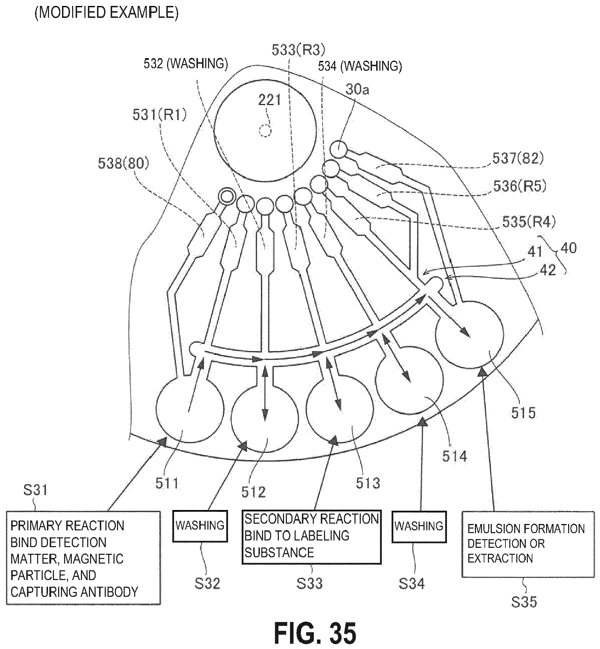

As illustrated in FIG. 35, in step S31, rotation of the cartridge 100 transfers the specimen 80 and the R1 reagent containing the primary antibody to the chamber 511. The specimen 80, the magnetic particles PM, and R1 reagent containing the primary antibody is mixed in the chamber 511. The temperature adjusting unit 260 controls the cartridge 100 at a predetermined reaction temperature to generate an immune complex IC containing the antigen, the primary antibody, and the magnetic particles (see FIG. 34). The temperature is controlled at about 40°C to about 50°C, more preferably about 42°C.

### <Washing (Primary BF Separation)>

The liquid storing unit 532 connected to the chamber 512 stores a reagent to wash the immune complex. In step S32, rotation of the cartridge 100 transfers the reagent to wash the immune complex to the chamber 512. The immune complex generated in the chamber 511 is transferred to the adjacent chamber 512 by the combination of the magnetic force exerted by the transfer mechanism 240 and the rotation of the cartridge 100 by the rotation mechanism 220. The immune complex is mixed with the reagent to wash the immune complex in the chamber 512 and washed (primary BF separation).

### <Secondary Reaction>

In the liquid storing unit 533 connected to the chamber 513, an R3 reagent containing the labeling substance LS is stored. The labeling substance LS is an enzyme-labeled antibody that includes a label and a capturing substance that specifically binds to the detection matter, and binds to an antigen being the detection matter MD, for example. In step S33, rotation of the cartridge 100 transfers the R3 reagent containing the labeling substance LS from the liquid storing unit 533 to the chamber 513. The immune complex washed in the chamber 512 is transferred to the adjacent chamber 513 by the combination of the magnetic force and the rotation of the cartridge 100. The immune complex IC (see FIG. 34) is mixed with the R3 reagent containing the labeling substance LS in the chamber 513, and binds to the labeling substance LS.

### <Washing (Secondary BF Separation)>

In the liquid storing unit 534 connected to the chamber 514, a reagent to wash the immune complex IC is stored. In step S34, rotation of the cartridge 100 transfers the reagent to wash the immune complex IC to the chamber 514. The immune complex IC bound to the labeling substance LS in the chamber 513 is transferred to the adjacent chamber 514 by the combination of the magnetic force and the rotation of the cartridge 100. The immune complex IC is mixed with the reagent to wash the immune complex IC in the chamber 514 and washed (secondary BF separation).

### <Emulsion Formation>

The chamber 515 is connected with three liquid storing units 535, 536, and 537. The liquid storing unit 535 stores an R4 reagent to disperse the immune complex, the liquid storing unit 536 stores an R5 reagent containing a substrate that reacts with the labeling substance LS, and the liquid storing unit 537 stores the dispersion medium 82. In step S35, rotation of the cartridge 100 transfers the R4 reagent, the R5 reagent, and the dispersion medium 82 to the chamber 515. The immune complex IC washed in the chamber 514 is transferred to the adjacent chamber 515 by the combination of the magnetic force and the rotation of the cartridge 100. The immune complex IC is mixed with the R4 reagent and the R5 reagent in the chamber 515. The R4 reagent is, for example, a buffer. The R5 reagent is a luminescent reagent containing a luminescent substrate which produces light upon reaction with a labeled antibody bound to the complex. Specifically, the R5 reagent is the reagent 81 containing a substrate that reacts with the labeling substance LS specifically bound to the protein in the specimen 80. The chamber 515 stores a dispersoid containing the immune complex IC, the luminescent substrate, and the buffer solution.

Then, in the chamber 515, the cartridge 100 is rotated about the rotational shaft 221 to agitate the specimen 80, the reagent 81, and the dispersion medium 82 in the chamber 11, and thereby the emulsion 83 is formed in which a dispersoid containing the specimen 80 and the reagent 81 is dispersed in the dispersion medium 82. Specifically, the cartridge 100 is rotated about the rotational shaft 221 to repeat the operation of changing the rotational speed of the cartridge 100. This exerts an inertial force associated with the speed change, agitating the immune complex IC in the chamber 515, the substrate that reacts with the labeling substance LS specifically bound to the protein in the specimen 80, and the dispersion medium 82 to form the emulsion 83 (see FIG. 34).

Rotation of the cartridge 100 forms in the emulsion 83 droplets 84 of the dispersoid containing one or zero particle of the detection matter MD. The temperature adjusting unit 260 controls the cartridge 100 at a predetermined reaction temperature. As a result, the substrate and the immune complex react in each droplet, a fluorescent substance is generated as a reaction product, and fluorescence is produced.

When the specimen processing apparatus 200 includes the detection unit 270, the detection unit 270 may be a camera unit which captures an image of the droplets 84 in the chamber 515 to detect fluorescence. The detection unit 270 makes it possible to detect one molecular unit of the detection matter MD contained in each droplet. In addition, in the case of detection in a specimen container without forming the emulsion 83, reaction products of the enzyme reaction is diluted in the sample. On the other hand, when the emulsion 83 is formed, the reaction products of the enzyme reaction are trapped in the droplets 84 and are not diluted. Therefore, the fluorescence of the individual droplets 84 can be detected with high sensitivity.

As described above, in the specimen processing method and the specimen processing apparatus according to the modified example, the detection matter MD is a protein. Therefore, it is possible to form an emulsion 83 in which the fine droplets 84 of the dispersoid containing a protein as the detection matter MD and the reagent 81 to store the detection matter MD are dispersed in the dispersion medium 82. As a result, the individual molecules of the detection matter MD are compartmentalized into the droplets 84, making it possible to reliably process the individual molecules of the detection matter MD with the reagent 81.

In addition, in the specimen processing method and the specimen processing apparatus according to the modified example, the reagent 81 contains a substrate that reacts with a labeling substance specifically bound to the protein in the specimen 80. Therefore, the detection based on the labeling substance for the protein as the detection matter MD can be reliably performed on individual molecules of the detection matter MD in the droplets 84 contained in the emulsion 83.

Note that, in the modified example, the magnetic particles may be coated with a binding substance for binding to the detection matter, or may be bound to the detection matter via a capturing substance for binding the magnetic particles and the detection matter together. The capturing substance is not particularly limited as long as it specifically binds to the detection matter. For example, the capturing substance binds to the detection matter upon an antigen-antibody reaction. More specifically, when the capturing substance is an antibody and the detection matter is an antibody, the capturing substance may be an antigen of that antibody. In addition, when the detection matter is a nucleic acid, the capturing substance may be a nucleic acid complementary to the detection matter. Examples of the label contained in the labeling substance include enzymes, fluorescent substances, and radioactive isotopes. Examples of enzymes include alkaline phosphatase (ALP), peroxidase, glucose oxidase, tyrosinase, acid phosphatase, and luciferase. In the case of electrochemiluminescence as chemiluminescence, the label is not particularly limited as long as it is a substance which emits light by electrochemical stimulation, and examples thereof include ruthenium complexes. Examples of fluorescent substances available include fluorescein isothiocyanate (FITC) and green fluorescent protein (GFP). Examples of radioactive isotopes available include 125l, 14C, and 32P.

In addition, when the label is an enzyme, it suffices to appropriately select, as a luminescent substrate for the enzyme, a known luminescent substrate depending on the enzyme to be used. In the case of using alkaline phosphatase as an enzyme, examples of the luminescent substrate available include chemiluminescent substrates such as CDP-Star (registered trademark), (4-chloro-3-(methoxyspiro[1,2-dioxetane-3,2'-(5'-chloro)trixilo[3.3.1.13,7]decane]-4-yl)disodium phenyl phosphate), and CSPD (registered trademark) (3-(4-methoxyspiro[1,2-dioxetane-3,2-(5'-chloro)tricyclo[3.3.1.13,7]decane]-4-yl)disodium phenyl phosphate); luminescent substrates such as p-nitrophenyl phosphate, 5-bromo-4-chloro-3-indolyl phosphate (BCIP), 4-nitro blue tetrazolium chloride (NBT), and iodonitro tetrazolium (INT); fluorescent substrates such as 4-methylumbelliferyl phosphate (4MUP); and chromogenic substrates such as 5-bromo-4-chloro-3-indolyl phosphate (BCIP), 5-bromo-6-chloro-indolyl disodium phosphate, and p-nitrophenyl phosphate.

### [Second Embodiment]

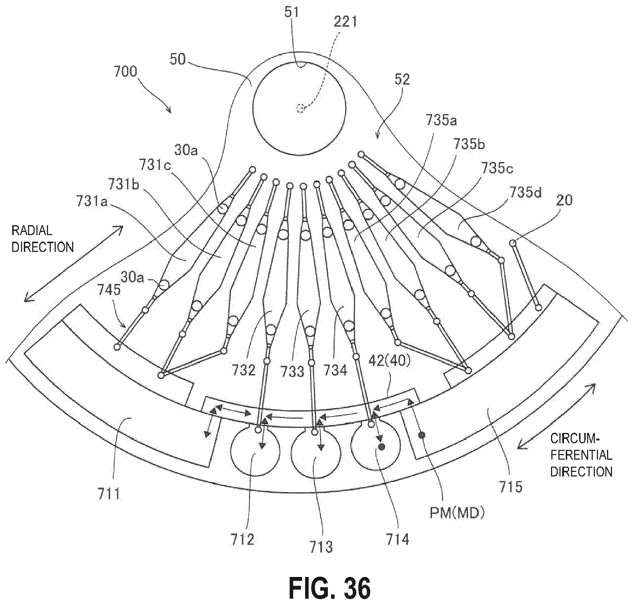

FIG. 36 illustrates a cartridge 700 according to a second embodiment. The cartridge 700 includes five chambers 711 to 715 and ten liquid storing units.

The chambers 711 to 715 are arranged in the circumferential direction. The chamber 711 is connected with three liquid storing units, namely liquid storing units 731a, 731b, and 731c. The chamber 712 is connected with one liquid storing unit 732. The chamber 713 is connected with one liquid storing unit 733. The chamber 714 is connected with one liquid storing unit 734. The chamber 715 is connected with four liquid storing units, namely liquid storing units 735a, 735b, 735c, and 735d. In addition, the chamber 715 is connected with a specimen introducing unit 20. The five chambers 711 to 715 are connected via the circumferential region 42 of the passage 40.

Each of the ten liquid storing units 731a to 731c, 732 to 734, and 735a to 735d communicates on the radially inner side with the outside of the cartridge 700, and is connected on the radially outer side to a flow path 745 communicating with the corresponding chamber. The connecting part with the outside and the connecting part with the corresponding flow path 745 of each liquid storing unit are each provided with a sealing body 30a, which can be opened by the unsealing mechanism 250. The other configurations of the cartridge 700 are the same as those of the cartridge 100, and thus the description is omitted.

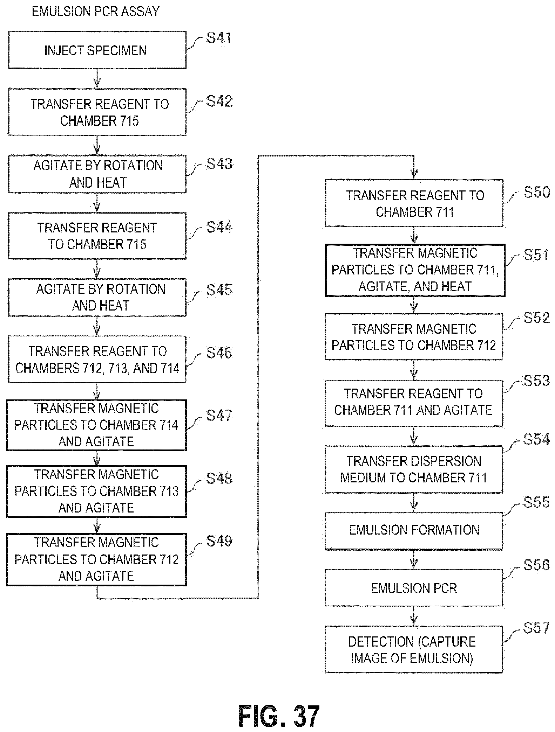

FIG. 37 illustrates the flow of processing using the cartridge 700. The processing using the cartridge 700 includes steps S41 to S56, and may further include step S57.

In step S41, the specimen 80 containing an antigen being the detection matter MD is injected from the specimen introducing unit 20. The specimen 80 is, for example, a blood specimen such as whole blood, plasma, or serum. Then, rotation of the cartridge 700 transfers the specimen 80 to the chamber 715.

In step S42, the reagent is transferred to the chamber 715. First, the liquid storing units 735a to 735d are storing an enzyme reagent, a solubilization reagent, a nucleic acid extraction reagent, and a magnetic particle reagent, respectively.

The enzyme reagent contains a proteolytic enzyme which degrades the protein contained in the specimen 80. The proteolytic enzyme causes efficient extraction of nucleic acids from cells in the specimen 80. The proteolytic enzyme is, for example, Proteinase K. The solubilization reagent contains a chaotropic compound such as a guanidine compound, and acts to lyse cells and bind nucleic acids to carriers. The nucleic acid extraction reagent mainly contains an alcohol component, and acts such that a nucleic acid-extracted nucleic acid solution is made into a hydrophobic environment, and thereby the nucleic acids are preferentially adsorbed to the magnetic particles PM. The magnetic particle reagent is a liquid containing magnetic particles PM coated with carriers which adsorb nucleic acids, and the carriers are, for example, silica. The nucleic acids are preferentially adsorbed to the carriers of the magnetic particles PM in an environment where a solubilization reagent and a nucleic acid extraction reagent are present.

Step S42 unseals the sealing bodies 30a of the two liquid storing units storing, among those reagents, the enzyme reagent and the solubilization reagent. Rotation of the cartridge 700 transfers the enzyme reagent and the solubilization reagent from the liquid storing units to the chamber 715.

In step S43, the dispersoid containing the specimen 80 and the reagent is agitated and heated to a predetermined temperature. The operation of changing the rotational speed of the cartridge 700 is repeated to agitate the liquid in the chamber 715. Then, the temperature adjusting unit 260 adjusts the temperature of the dispersoid in the chamber 715 at, for example, 75°C for a predetermined reaction time.

In step S44, the reagent is transferred to the chamber 715. The step first unseals the sealing bodies 30a of the two liquid storing units storing, among the reagents stored in the liquid storing units 735a to 735d, the nucleic acid extraction reagent and the magnetic particle reagent. Rotation of the cartridge 700 transfers the nucleic acid extraction reagent and the magnetic particle reagent from the liquid storing units to the chamber 715.

In step S45, agitation and heating are performed in the same manner as step S43. The temperature during heating is, for example, 75°C. The reaction in the dispersoid adsorbs the detection matter MD in the specimen 80 to the magnetic particles PM.

In step S46, the reagent 86 to wash the detection matter MD is transferred into the chambers 712, 713, and 714. Each of the liquid storing units 732, 733, and 734 stores the reagent 86 to wash the detection matter MD. The reagent 86 to wash the detection matter MD contains, for example, an alcohol such as ethanol. By making the nucleic acid solution into a hydrophobic environment, the alcohol suppresses the detachment of nucleic acids from the carriers of the magnetic particles PM, and removes unnecessary components adhering to the magnetic particles PM other than the detection matter MD. The reagents contained in the liquid storing units 732, 733, and 734 may have the same or different alcohol content ratios. For example, the alcohol content ratio in the reagent stored has such a relationship that the liquid storing units 732 > 733 > 734. Then, for the purpose of improving the washing effects, the content ratio of washing component such as surfactant in the reagent stored has such a relationship that the liquid storing units 732 < 733 < 734.

First, the sealing body 30a of each of the liquid storing units 732 to 734 is unsealed. Rotation of the cartridge 700 transfers the reagent 86 to wash the detection matter MD from the liquid storing units 732 to 734 to the corresponding chambers 712 to 714.

In step S47, the magnetic particles PM are transferred from the chamber 715 to the chamber 714. When the transfer mechanism 240 applies a magnetic force to the cartridge 700, the magnetic particles PM bound to the detection matter MD are gathered from the dispersoid in the chamber 715. The magnetic particles PM carrying the detection matter MD are moved to the chamber 714 by the magnetic force and the rotation of the cartridge 700. The magnetic particles PM moved into the chamber 714 are agitated in the wash the detection matter MD inside the chamber 714 by repeating the operations of moving the magnet 241 close and away or by repeating the operation of changing the rotational speed of the cartridge 700. Thereby, the magnetic particles PM are washed.

In step S48, the magnetic particles PM are transferred from the chamber 714 to the chamber 713. Then, in the same manner as step S47, the magnetic particles PM are agitated in the reagent 86 to wash the detection matter MD inside the chamber 713. Thereby, the magnetic particles PM are washed.

In step S49, the magnetic particles PM are transferred from the chamber 713 to the chamber 712. Then, in the same manner as step S47, the magnetic particles PM are agitated in the reagent 86 to wash the detection matter MD inside the chamber 712. Thereby, the magnetic particles PM are washed.

In step S50, the reagent is transferred to the chamber 711. First, the liquid storing units 731a to 731c are storing an elution reagent, the reagent 81, and the dispersion medium 82. The elution reagent makes the nucleic acid solution into a hydrophilic environment, whereby the nucleic acids adsorbed to the carriers of the magnetic particles PM are detached from the carriers and eluted in the solution. For example, the elution reagent contains a buffer solution such as Tris/EDTA (TE) buffer. The reagent 81 contains a nucleic acid amplification reagent that amplifies the nucleic acid in the specimen 80. In the example of FIG. 36, the nucleic acid amplification reagent contains substances necessary for PCR such as DNA polymerase, and further contains the labeling substance LS. The dispersion medium 82 includes an oil that is immiscible with the specimen 80 and the reagent 81. Step S50 unseals the sealing body 30a of the liquid storing unit storing, among those reagents, the elution reagent. Rotation of the cartridge 700 transfers the elution reagent from the liquid storing unit to the chamber 711.

In step S51, the magnetic particles PM are transferred from the chamber 712 to the chamber 711. Then, the operation of changing the rotational speed of the cartridge 700 is repeated to agitate the magnetic particles PM in the elution reagent inside the chamber 711. After that, the chamber 711 is heated, whereby the nucleic acids adsorbed to the carriers of the magnetic particles PM are detached from the magnetic particles PM and eluted in the elution reagent.

In step S52, the magnetic particles PM are transferred from the chamber 711 to the chamber 712. In step S52, since the nucleic acids are eluted in the solution inside the chamber 711, the magnetic particles PM without nucleic acids adsorbed thereto are transferred to the chamber 712. The nucleic acids being the detection matter MD are left in the solution inside the chamber 711.

In step S53, the reagent is transferred to the chamber 711. The step unseals the sealing body 30a of the liquid storing unit storing, among the reagents stored in the liquid storing units 731a to 731c, the nucleic acid amplification reagent being the reagent 81. Rotation of the cartridge 700 transfers the nucleic acid amplification reagent from the liquid storing unit to the chamber 711. Then, the operation of changing the rotational speed of the cartridge 700 is repeated to agitate the nucleic acid amplification reagent and the nucleic acid solution in the chamber 711.

In step S54, the reagent is transferred to the chamber 711. The step unseals the sealing body 30a of the liquid storing unit storing, among the reagents stored in the liquid storing units 731a to 731c, the oil being the dispersion medium 82. Rotation of the cartridge 700 transfers the dispersion medium 82 from the liquid storing unit to the chamber 711.

In step S55, an emulsion forming step is performed in the chamber 711. The chamber 711 stores the detection matter MD in the specimen 80, the nucleic acid amplification reagent being the reagent 81, the labeling substance LS, and the oil being the dispersion medium 82. Then, rotation of the cartridge 100 about the rotational shaft 221 agitates the specimen 80, the reagent 81, and the dispersion medium 82 in the chamber 11, and forms an emulsion 83 in which the dispersoid containing the specimen 80 and the reagent 81 is dispersed in the dispersion medium 82. For example, the cartridge 700 forms in the chamber 711 the emulsion 83 in which the dispersoid containing the specimen 80 and the reagent 81 is dispersed in the dispersion medium 82 by repetition of the operation of changing the rotational speed during rotation about the rotational shaft 221. The emulsion forming step repeats the operation of changing the rotational speed faster than the agitating operation in the chambers.

Step S56 performs an emulsion PCR step of amplifying the nucleic acids (DNAs) in the droplets 84 formed by the emulsion forming step. After the emulsion 83 is formed, the temperature adjusting unit 260 performs thermal cycling in which the temperature of the cartridge 700 is cyclically changed to multiple temperature ranges, thereby amplifying nucleic acids contained in the dispersoid. Thus, nucleic acids are efficiently amplified in the droplets 84 contained in the emulsion 83 (see FIG. 12A to FIG. 12D). In addition, the nucleic acid amplification products in the droplets 84 react with the labeling substance LS contained in the nucleic acid amplification reagent to emit fluorescence.

Step S57 performs a step of detecting the detection matter MD. For example, a detection unit 270 being an image capturing unit captures an image of the chamber 711 of the cartridge 700. The controller 230 analyzes the captured image to detect the detection matter MD. Specifically, based on the captured image of the emulsion 83 in the chamber 711 captured by the detection unit 270, the controller 230 outputs the presence or absence of nucleic acids being the detection matter MD. Note that, in the example of FIG. 36 and FIG. 37, the emulsion breaking step is not performed. Thus, with the droplet 84 formed to contain one molecule or one particle of nucleic acid as a unit of detection, the presence or absence of nucleic acids being the detection matter MD is counted from the captured image. The captured image may be transmitted from the specimen processing apparatus 200 to the server 650 (see FIG. 19) and subjected to image analysis in the server 650. In that case, based on the captured image of the emulsion 83 in the chamber 711, the server 650 outputs to the external terminal 600 (see FIG. 19) the presence or absence of nucleic acids being the detection matter MD directly or via the specimen processing apparatus 200.

### [Third Embodiment]

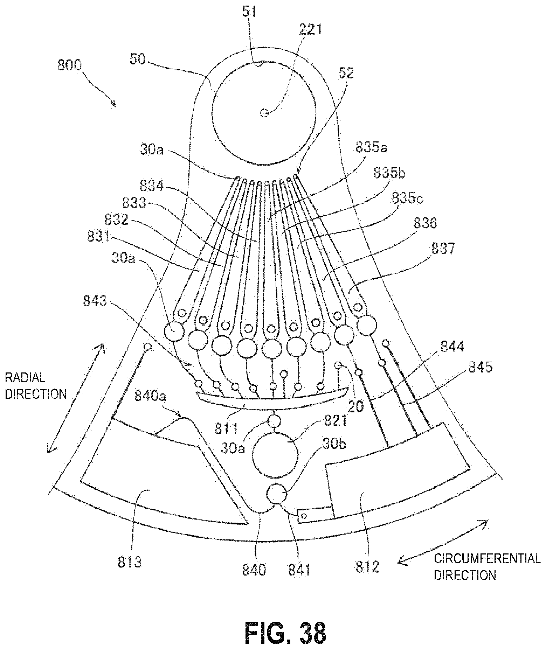

FIG. 38 illustrates a cartridge 800 according to a third embodiment. The cartridge 800 includes three chambers 811 to 813, nine liquid storing units, and a filter unit 821 for nucleic acid extraction.

The chamber 811 extends in the circumferential direction in an arc form at a position radially inward of the chambers 812 and 813. The chambers 812 and 813 are arranged in the circumferential direction in the vicinity of the radial outer edge of the cartridge 800. The chamber 811 is connected with seven liquid storing units, namely liquid storing units 831, 832, 833, 834, 835a, 835b, and 835c. In addition, the chamber 811 is connected with the specimen introducing unit 20. The chamber 812 is connected with two liquid storing units 836 and 837.

The chamber 811 and the chambers 812 and 813 are connected via the filter unit 821. Specifically, the chamber 811 communicates with the filter unit 821 via the sealing body 30a. The filter unit 821 communicates with each of the chambers 812 and 813 via the sealing body 30b on the radially outer side. The filter unit 821 communicates with the chamber 812 via a flow path 841 extending from the sealing body 30b, and communicates with the chamber 813 via a flow path 840 extending from the sealing body 30b. The sealing body 30b is provided to cause communication between the filter unit 821 and the flow path 840 and to seal the flow path 841. When the sealing body 30b is unsealed, the filter unit 821 is caused to communicate with the chamber 812 via the flow path 841. Note that each of the chambers 811 to 813 communicates with the outside of the cartridge 800 on the radially inner side.

The seven liquid storing units 831 to 834 and 835a to 835c communicate with the outside of the cartridge 800 on the radially inner side, and are connected to flow paths 843 communicating with the chamber 811 on the radially outer side. Each of the flow paths 843, the flow path 844 connecting the liquid storing unit 836 and the chamber 812, and the flow path 845 connecting the liquid storing unit 837 and the chamber 812 is provided with a sealing body 30a, and can be unsealed by the unsealing mechanism 250.

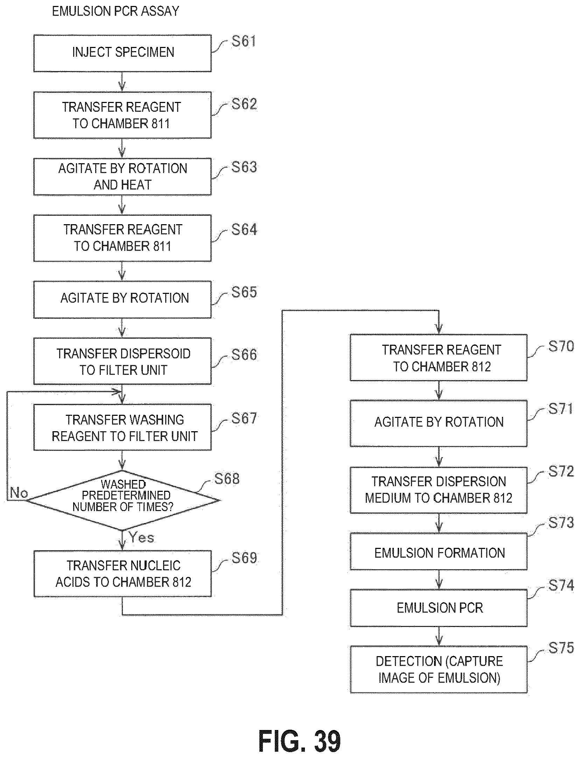

FIG. 39 illustrates the flow of processing using the cartridge 800. The processing using the cartridge 800 includes steps S61 to S74, and may further include step S75.

In step S61, the specimen 80 containing an antigen being the detection matter MD is injected from the specimen introducing unit 20. The specimen 80 is the same as that in a second embodiment described above. Then, rotation of the cartridge 800 transfers the specimen 80 to the chamber 811.

In step S62, the reagent is transferred to the chamber 811. First, the liquid storing units 831 to 834 are storing an enzyme reagent, a solubilization reagent, a nucleic acid extraction reagent, and an elution reagent, respectively. The step unseals the sealing bodies 30a of the two liquid storing units storing, among those, the enzyme reagent and the solubilization reagent. Rotation of the cartridge 800 transfers the enzyme reagent and the solubilization reagent from the liquid storing units to the chamber 811.

In step S63, the dispersoid containing the specimen 80 and the reagent is agitated and heated to a predetermined temperature. The operation of changing the rotational speed of the cartridge 800 is repeated to agitate the liquid in the chamber 811. Then, the temperature adjusting unit 260 adjusts the temperature of the dispersoid in the chamber 811 at, for example, 75°C for a predetermined reaction time.

In step S64, the reagent is transferred to the chamber 811. The step first unseals the sealing body 30a of the liquid storing unit storing, among the reagents stored in the liquid storing units 831 to 834, the nucleic acid extraction reagent. Rotation of the cartridge 800 transfers the nucleic acid extraction reagent from the liquid storing unit to the chamber 811.

In step S65, agitation is performed as in step S63.

In step S66, the dispersoid in the chamber 811 is transferred to the filter unit 821. The step first unseals the sealing body 30a between the chamber 811 and the filter unit 821. The dispersoid in the chamber 811 is a nucleic acid solution containing nucleic acids being the detection matter MD. Rotation of the cartridge 800 transfers the nucleic acid solution from the chamber 811 to the filter unit 821.

The filter unit 821 includes a filter having a carrier which adsorbs nucleic acids, and is a silica membrane filter, for example. The filter unit 821 adsorbs and captures nucleic acids in an environment where a solubilization reagent and a nucleic acid extraction reagent are present, and passes liquids and components other than nucleic acids. The nucleic acids in the nucleic acid solution are adsorbed and fixed by the filter unit 821.

In step S67, the washing processing is performed with the reagent 86 to wash the detection matter MD. Specifically, each of the liquid storing units 835a to 835c stores the reagent 86 to wash the detection matter MD. The reagent 86 to wash the detection matter MD is the same as that of a second embodiment described above. For example, the step unseals the sealing body 30a of the first liquid storing unit 835a among the liquid storing units 835a to 835c. Rotation of the cartridge 800 transfers the reagent 86 to wash the detection matter MD from the liquid storing unit via the chamber 811 to the filter unit 821.

With the reagent 86 to wash the detection matter MD transferred to the filter unit 821, the cartridge 800 is further rotated. Thereby, the reagent 86 to wash the detection matter MD is transferred from the filter unit 821 to the chamber 813 via the sealing body 30b and the flow path 840. As a result, unnecessary components present in the filter unit 821 are discharged together with the reagent. The unnecessary components are components other than the detection matter MD which are unnecessary for detection of the detection matter MD. Note that the reagent does not flow into the chamber 812 because the flow path 841 is sealed by the sealing body 30b.

In step S68, it is determined whether or not the washing processing has been performed a predetermined number of times. In the example of FIG. 38, since the reagent 86 to wash the detection matter MD is stored in three liquid storing units, namely the liquid storing units 835a to 835c, the predetermined number of times is three. If the washing processing has not been performed a predetermined number of times, the processing returns to step S67. The sealing body 30a of the next liquid storing unit 835b is unsealed, and rotation of the cartridge 800 transfers the reagent 86 to wash the detection matter MD via the filter unit 821 to the chamber 813. The same applies to the liquid storing unit 835c. When the washing processing has been performed a predetermined number of times, the processing proceeds to step S69.

In step S69, nucleic acids being the detection matter MD captured by the filter unit 821 are transferred to the chamber 812. Specifically, the step first unseals the sealing body 30a of the liquid storing unit storing the elution reagent among the liquid storing units 831 to 834. Moreover, the sealing body 30b sealing the flow path 841 connected to the chamber 812 is unsealed. Rotation of the cartridge 800 transfers the elution reagent from the liquid storing unit via the chamber 811 to the filter unit 821. As a result, the nucleic acids adsorbed to the filter unit 821 are detached from the filter unit 821 and dissolved in the elution reagent. Further continuing rotation of the cartridge 800 transfers the nucleic acid solution in the filter unit 821 to the chamber 812 via the sealing body 30a and the flow path 841.

Note that the flow path 840 connected to the chamber 813 extends to a flow path portion 840a located radially inward of the chamber 812, and then is connected to the chamber 813. Therefore, in the case of transferring the liquid by centrifugal force, the liquid does not flow through the flow path portion 840a into the chamber 813 with the sealing body 30b unsealed unless the chamber 812 is filled with the liquid. Thus, the nucleic acid solution flowing out of the filter unit 821 is transferred to the chamber 812 in substantially a total amount without flowing into the chamber 813.

In step S70, the reagent 81 is transferred to the chamber 812. First, the liquid storing units 836 and 837 are storing the reagent 81 and the dispersion medium 82, respectively. The reagent 81 and the dispersion medium 82 are the same as those in a second embodiment described above. The step unseals the sealing body 30a of the liquid storing unit 836 storing, among those, the reagent 81. Rotation of the cartridge 800 transfers the reagent 81 from the liquid storing unit 836 via the flow path 844 to the chamber 812.

In step S71, the operation of changing the rotational speed of the cartridge 800 is repeated to agitate the nucleic acid solution and the reagent 81 in the chamber 812.

In step S72, the dispersion medium 82 is transferred to the chamber 812. The step unseals the sealing body 30a of the liquid storing unit 837 storing the oil being the dispersion medium 82. Rotation of the cartridge 800 transfers the dispersion medium 82 from the liquid storing unit 837 via the flow path 845 to the chamber 812.

In step S73, an emulsion forming step is performed in the chamber 812. The chamber 812 stores the detection matter MD in the specimen 80, the nucleic acid amplification reagent being the reagent 81, the labeling substance LS, and the oil being the dispersion medium 82. Then, rotation of the cartridge 100 about the rotational shaft 221 agitates the specimen 80, the reagent 81, and the dispersion medium 82 in the chamber 11, and forms an emulsion 83 in which the dispersoid containing the specimen 80 and the reagent 81 is dispersed in the dispersion medium 82. The cartridge 800 forms in the chamber 812 the emulsion 83 in which the dispersoid containing the specimen 80 and the reagent 81 is dispersed in the dispersion medium 82 by repetition of the operation of changing the rotational speed during rotation about the rotational shaft 221. Note that, in the example of FIG. 38 and FIG. 39, the detection matter MD is not supported by the magnetic particles PM. Therefore, the magnetic particle reagent containing the magnetic particles PM is not stored in the cartridge 800. In the example of FIG. 38, since the filter unit 821 is provided instead of the magnetic particles PM as described above, the detection matter MD is transferred to each chamber only by the centrifugal force associated with the rotation of the cartridge 800. In the case where the magnetic particles PM are not used, it is unnecessary to provide the transfer mechanism 240.

Step S74 performs an emulsion PCR step of amplifying the nucleic acids (DNAs) in the droplets 84 formed by the emulsion forming step. After the emulsion 83 is formed, the temperature adjusting unit 260 performs thermal cycling in which the temperature of the cartridge 800 is cyclically changed to multiple temperature ranges, thereby amplifying nucleic acids contained in the dispersoid. Thus, nucleic acids are efficiently amplified in the droplets 84 contained in the emulsion 83 (see FIG. 12A to FIG. 12D). In addition, the nucleic acid amplification products in the droplets 84 react with the labeling substance LS contained in the nucleic acid amplification reagent to emit fluorescence.

Note that, in the example of FIG. 38, the emulsion breaking step is not performed as in a second embodiment. Step S75 performs a step of detecting the detection matter MD. For example, a detection unit 270 being an image capturing unit captures an image of the chamber 812 of the cartridge 800. The controller 230 analyzes the captured image to detect the detection matter MD. Specifically, based on the captured image of the emulsion 83 in the chamber 812 captured by the detection unit 270, the controller 230 outputs the presence or absence of nucleic acids being the detection matter MD. The image analysis may be performed at server 650.

### [Modified Example of Nucleic Acid Detection Apparatus]

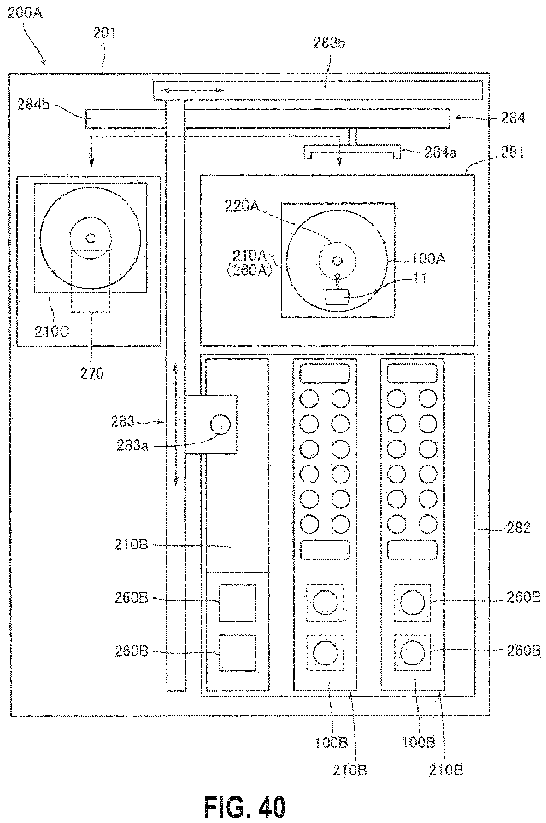

FIG. 40 illustrates a modified example of a specimen processing apparatus 200A according to a first embodiment. The specimen processing apparatus 200A according to the modified example is structured to perform processing using multiple cartridges.

In the example of FIG. 40, the specimen processing apparatus 200A includes a specimen processing unit 281 that processes a specimen using a first cartridge 100A that performs the step of preparing the emulsion 83 and the nucleic acid amplification step, and a specimen extraction unit 282 that processes a specimen using a second cartridge 100B that performs the nucleic acid extraction step.

In addition, the specimen processing apparatus 200A includes a specimen dispensing unit 283 to transfer a specimen between the cartridge 100B and the cartridge 100A, a detection unit 270, and a cartridge transfer mechanism 284 to transfer the cartridge 100A.

The specimen extraction unit 282 includes three setting parts 210B on which the rectangular cartridges 100B are set, and temperature adjusting units 260B provided on each setting part 210B. The specimen 80 containing nucleic acids is introduced into the cartridges 100B set on the specimen extraction unit 282. The specimen 80 may be introduced by the user or may be introduced by the specimen dispensing unit 283.

The specimen processing apparatus 200A performs the step of extracting nucleic acids from at least the specimen 80 using the cartridges 100B placed in the specimen extraction unit 282. In the cartridges 100B, for example, an enzyme reagent, a solubilization reagent, a nucleic acid extraction reagent, and an elution reagent may be sealed in advance as reagents to extract nucleic acids.

The specimen processing apparatus 200A may perform the step of washing the extracted nucleic acids with the reagent 86 to wash nucleic acids using the cartridges 100B placed in the specimen extraction unit 282. In that case, in the cartridges 100B, the reagent 86 to wash nucleic acids may be sealed in advance.

The specimen dispensing unit 283 transfers the specimen 80 and the reagents in the cartridges 100B by transferring the liquid between the wells formed in the cartridges 100B. The specimen dispensing unit 283 may apply pressure to the openings formed in the cartridges 100B to transfer the liquid by pressure in the cartridges 200B.

The specimen dispensing unit 283 includes, for example, an aspirator 283a which aspirates and discharges a liquid, and an aspirator moving mechanism 283b. The aspirator 283a is structured to be movable to the setting positions of the cartridges 100B in the specimen extraction unit 282 and to the setting position of the cartridge 100A in the specimen processing unit 281 by the aspirator moving mechanism 283b. The aspirator 283a is structured, for example, such that a disposable pipette tip can be attached and detached, and replaces the pipette tip when aspirating and dispensing a liquid containing nucleic acids. The specimen dispensing unit 283 uses the aspirator 283a to aspirate the solution containing nucleic acids extracted using the cartridges 100B from the cartridges 100B, and injects the solution into the specimen introducing unit 20 of the cartridge 100A.

The specimen processing unit 281 is provided with a setting part 210A on which the disk-shaped cartridge 100A is set, and a temperature adjusting unit 260A. In the example of FIG. 40, the specimen processing unit 281 includes a rotation mechanism 220A.

The sample specimen processing apparatus 200A performs at least the step of preparing the emulsion 83 and the nucleic acid amplification step using the cartridge 100A set on the specimen processing unit 281. For example, in the cartridge 100A, the reagent 81 to amplify nucleic acids, the labeling substance LS, and an oil being the dispersion medium 82 are sealed in advance.

The specimen processing apparatus 200A may perform the step of washing nucleic acids extracted in the cartridges 100B with the reagent 86 to wash nucleic acids using the cartridge 100A set on the specimen processing unit 281. In that case, the reagent 86 to wash nucleic acids may be sealed in the cartridge 100A in advance.

Specifically, the configuration of a first embodiment illustrated in FIG. 40 may be employed for the cartridge 100A. In that case, the specimen processing apparatus 200A includes the transfer mechanism 240 which exerts a magnetic force on the cartridge 100A. The cartridge 100A may employ a configuration omitting the structure relating to the extraction of nucleic acids from the configuration of a second embodiment described above and illustrated in FIG. 36 or the configuration of a third embodiment described above and illustrated in FIG. 38.

In the specimen processing unit 281, the emulsion 83 is formed in the chamber 11 of the cartridge 100A by the rotation mechanism 220A, and nucleic acids in the droplets 84 are amplified (that is, emulsion PCR) in the chamber 11 by the temperature adjusting unit 260A. In the specimen processing unit 281, the amplification products of nucleic acids react with the labeling substance LS.

The cartridge transfer mechanism 284 includes, for example, a gripping unit 284a which grips the cartridge 100A, and a gripping unit moving mechanism 284b. The gripping unit 284a holds the cartridge 100A by means such as pinching, adsorption, or engagement. The gripping unit 284a is structured to be movable to the setting position of the cartridge 100A in the specimen processing unit 281 and to a setting part 210C of the cartridge 100A in the detection unit 270 by the gripping unit moving mechanism 284b. After the processing in the specimen processing unit 281, the cartridge transfer mechanism 284 transports the cartridge 100A storing the nucleic acids bound to the labeling substance LS from the specimen processing unit 281 to the setting part 210C of the detection unit 270.

The detection unit 270 detects signals based on the nucleic acids amplified in the cartridge 100A. The detection unit 270 includes, for example, an image capturing unit. The detection unit 270 may be placed in the specimen processing unit 281 as in the configuration illustrated in FIG. 17. In that case, the detection unit 270 captures an image of the chamber 11 from below or above the cartridge 100A placed in the setting part 210A of the specimen processing unit 281. In this case, the cartridge transfer mechanism 284 may be omitted.

With the above configuration, the specimen processing apparatus 200A according to the modified example detects nucleic acids by transferring a specimen between cartridges using multiple cartridges.

It should be understood that the embodiments disclosed herein are illustrative and non-restrictive in all respects. The scope of the present invention is indicated not by the description of the embodiments above but by the claims, and further includes all modifications within the meanings and scope equivalent to those of the claims.

## Claims

1. A specimen processing method that uses a cartridge comprising a chamber configured to store a liquid, the method comprising:
storing a specimen and a dispersion medium in the chamber of the cartridge; and
rotating the cartridge about a rotational shaft to agitate the specimen and the dispersion medium in the chamber, to thereby form an emulsion in which a dispersoid containing the specimen is dispersed in the dispersion medium.

2. The specimen processing method according to claim 1, wherein the cartridge comprises a plurality of chambers arranged in an arc form whose radial distances from the rotational shaft are substantially equal.

3. The specimen processing method according to claim 1 or 2, wherein
the rotating the cartridge about the rotational shaft to form the emulsion comprises changing a rotational speed of the cartridge repeatedly.

4. The specimen processing method according to claim 3, wherein
the changing the rotational speed of the cartridge comprises:
reversing a rotational direction of the cartridge; or
accelerating and decelerating the rotational speed of the cartridge in one direction.

5. The specimen processing method according to claim 4, wherein
the changing the rotational speed of the cartridge comprises reversing the rotational direction of the cartridge in a cycle of 165 milliseconds or more and 330 milliseconds or less.

6. The specimen processing method according to any one of claims 3 to 5, wherein
a ratio of a total volume of the dispersoid and the dispersion medium to the chamber is 30% or more and 70% or less.

7. The specimen processing method according to any one of claims 3 to 6, wherein
the changing the rotational speed of the cartridge comprises reversing a rotational direction of the cartridge in a cycle of 330 milliseconds, and
a ratio of a total volume of the dispersoid and the dispersion medium to the chamber is 30%.

8. The specimen processing method according to any one of claims 3 to 7, wherein
the changing the rotational speed of the cartridge comprises reversing a rotational direction of the cartridge in a cycle of 165 milliseconds, and
a ratio of a total volume of the dispersoid and the dispersion medium to the chamber is 50% or more and 70% or less.

9. The specimen processing method according to any one of claims 1 to 8, wherein
the specimen comprises a nucleic acid or a protein to be detected as a detection matter.

10. The specimen processing method according to anyone of claims 1 to 9, further comprising:
processing a detection matter in the specimen with a reagent comprising a labeling substance to label the detection matter; and
detecting a signal based on the labeling substance.

11. The specimen processing method according to any one of claims 1 to 10, wherein
the storing the specimen and the dispersion medium in the chamber of the cartridge comprises storing, in the chamber, the specimen, the dispersion medium, and a reagent to process a detection matter in the specimen, and
the rotating the cartridge to form the emulsion in which the dispersoid containing the specimen is dispersed in the dispersion medium comprises rotating the cartridge to form the emulsion in which the dispersoid containing the specimen and the reagent is dispersed in the dispersion medium.

12. The specimen processing method according to claim 11, wherein
the reagent comprises:
a nucleic acid amplification reagent configured to amplify a nucleic acid in the specimen; or
a substrate that reacts with a labeling substance specifically bound to a protein in the specimen.

13. A specimen processing apparatus comprising:
a setting part in which a cartridge comprising a chamber configured to store a specimen and a dispersion medium is set;
a rotation mechanism that rotates the cartridge set in the setting part about a rotational shaft; and
a controller that causes the rotation mechanism to rotate the cartridge comprising the chamber storing the specimen and the dispersion medium so as:
to agitate the specimen and the dispersion medium in the chamber; and
to form an emulsion in which a dispersoid containing the specimen is dispersed in the dispersion medium.

14. A non-transitory computer-readable storage medium storing a program, which when read and executed, causes a computer, connected to a specimen processing apparatus that processes a specimen using a cartridge comprising a chamber to store a liquid, to perform operations comprising:
causing a rotation mechanism of the specimen processing apparatus to rotate the cartridge that stores the specimen and a dispersion medium about a rotational shaft so as to agitate the specimen and the dispersion medium in the chamber, to thereby form an emulsion in which a dispersoid containing the specimen is dispersed in the dispersion medium.

15. A specimen processing cartridge comprising:
a specimen introducing unit that introduces a specimen; and
a chamber that stores the introduced specimen and a dispersion medium, wherein
the specimen processing cartridge is configured to, by being rotated about a rotational shaft, agitate the specimen and the dispersion medium in the chamber, and to form an emulsion in which a dispersoid containing the specimen is dispersed in the dispersion medium.
